(19) Europäisches Patentamt
European Patent Office
Office européen des brevets

(11) Veröffentlichungsnummer: **0 154 115**
**B1**

(12) # EUROPÄISCHE PATENTSCHRIFT

(45) Veröffentlichungstag der Patentschrift: **26.10.88**

(21) Anmeldenummer: **85100295.6**

(22) Anmeldetag: **14.01.85**

(51) Int. Cl.⁴: **A 01 N 43/56,** A 01 N 53/00, A 01 N 57/22, A 01 N 47/30, C 07 D 231/14, C 07 D 231/38, C 07 C 121/82

(54) **Herbizide Mittel auf Basis von Pyrazolderivaten.**

(30) Priorität: **24.01.84 DE 3402308**

(43) Veröffentlichungstag der Anmeldung: **11.09.85 Patentblatt 85/37**

(45) Bekanntmachung des Hinweises auf die Patenterteilung: **26.10.88 Patentblatt 88/43**

(84) Benannte Vertragsstaaten: **AT BE CH DE FR GB IT LI NL SE**

(73) Patentinhaber: **BAYER AG, Konzernverwaltung RP Patentabteilung, D-5090 Leverkusen 1 Bayerwerk (DE)**

(72) Erfinder: **Schallner, Otto, Dr., Noldeweg 22, D-4019 Monheim (DE)**
Erfinder: **Gehring, Reinhold, Dr., Dasnöckel 49, D-5600 Wuppertal 11 (DE)**
Erfinder: **Klauke, Erich, Dr., Eichendorffweg 8, D-5068 Odenthal (DE)**
Erfinder: **Stetter, Jörg, Dr., Gellertweg 4, D-5600 Wuppertal 1 (DE)**
Erfinder: **Wroblowsky, Heinz-Jürgen, Dr., Gladbacher Strasse 36, D-4018 Langenfeld (DE)**
Erfinder: **Schmidt, Robert R., Dr., Im Waldwinkel 110, D-5060 Bergisch-Gladbach 2 (DE)**
Erfinder: **Santel, Hans-Joachim, Dr., Gerstenkamp 19, D-5000 Köln 80 (DE)**

(56) Entgegenhaltungen:
**EP - A - 0 034 845**
**EP - A - 0 053 699**
**DD - A - 202 612**
**DE - A - 3 226 513**

**THE JOURNAL OF ORGANIC CHEMISTRY, Band 36, September-Dezember 1971, Y. AHMAD, P.A.S. SMITH, "Pyrazolotriazines from condensation of nitro with amino groups", pp. 2972-2974**
**JOURNAL OF HETEROCYCLIC CHEMISTRY, Band 7, no. 1, Februar 1970, M.D. COBURN "Picrylamino-substituted Heterocycles. IV. Pyrazoles (1,2)", pp. 345-349**

(56) Entgegenhaltungen: (Fortsetzung)
**CHEMICAL ABSTRACTS, Band 62, 24. Mai - 21. Juni 1965, Columbus, Ohio, USA, A. TAKAMIZAWA et al. "Synthesis of pyrazol derivatives VIII. Supplement of the reaction of propionitriles and related compounds with aryl-hydrazines", Zusammenfassung Nr. 13 137c**

**Die Akte enthält technische Angaben, die nach dem Eingang der Anmeldung eingereicht wurden und die nicht in dieser Patentschrift enthalten sind.**

EP 0 154 115 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Verwendung von teilweise bekannten substituierten 5-Amino-1-phenyl-pyrazolen als Herbizide.

Es ist bereits bekannt, dass bestimmte in 4-Stellung durch eine Cyano-Gruppe substituierte 5-Amino-1-phenyl-pyrazole, wie beispielsweise das 4-Cyano-5-propionylamino-1-(2,4,6-trichlorphenyl)-pyrazol

herbizide Eigenschaften besitzen (vgl. z.B. DE-A-3 226 513).

Deren herbizide Wirksamkeit gegenüber einigen Problemunkräutern ebenso wie ihre Verträglichkeit gegenüber wichtigen Nutzpflanzen ist jedoch nicht immer in allen Anwendungsbereichen völlig zufriedenstellend.

Weiterhin sind in 4-Stellung unsubstituierte oder in 4-Stellung durch Methyl oder Phenyl substituierte 5-Amino-1-phenylpyrazole, wie beispielsweise das 5-Amino-1-(2,4-dinitrophenyl)-4-methylpyrazol, das 5-Amino-1-(2,4-dinitrophenyl)-4-phenyl-pyrazol, das 5-Amino-4-(4-chlorphenyl)-1-(2,4-dinitrophenyl)-pyrazol, das 5-Amino-1-(2,4-dinitrophenyl)-4-(4-methoxyphenyl)-pyrazol, das 1-(2-Nitrophenyl)-4-methyl-5-amino-pyrazol oder das 5-Acetamido-1-(2,4,6-trinitrophenyl)-pyrazol bekannt (vgl. J. Org. Chemistry 36, 2972–2974 [1971]; J. Heterocycl. Chem. 7, 345–349 [1970], C.A. 62, 13137c). Über deren Wirksamkeit als Herbizide ist jedoch nichts bekannt.

Es wurde gefunden, dass die teilweise bekannten substituierten 5-Amino-1-phenyl-pyrazole der allgemeinen Formel (I),

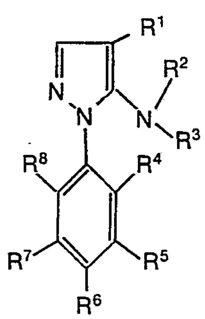

(I)

in welcher

$R^1$ für Wasserstoff, Nitroso, Nitro, Halogen, jeweils geradkettiges oder verzweigtes Alkyl oder Halogenalkyl mit jeweils bis zu 6 Kohlenstoffatomen und im Fall des Halogenalkyl bis zu 9 gleichen oder verschiedenen Halogenatomen, ferner für gegebenenfalls einfach oder mehrfach, gleich oder verschieden durch Halogen, Nitro, Alkyl, Alkoxy oder Halogenalkyl mit jeweils bis zu 4 Kohlenstoffatomen substituiertes Phenyl oder für einen der Reste

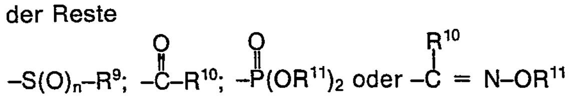

steht,

$R^2$ für Wasserstoff oder einen Rest

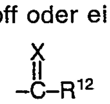

steht,

$R^3$ unabhängig von $R^2$ für die gleichen Reste wie $R^2$ steht und zusätzlich für geradkettiges oder verzweigtes Alkyl mit bis zu 4 Kohlenstoffatomen steht,

$R^4$ und $R^6$ unabhängig voneinander für Cyano, Nitro, Halogen oder für jeweils geradkettiges oder verzweigtes Alkyl, Alkoxy oder Alkoxycarbonyl mit jeweils bis zu 4 Kohlenstoffatomen ausserdem für jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder für einen Rest $-S(O)_n-R^{13}$ stehen, und

$R^5$, $R^7$ und $R^8$ unabhängig voneinander und von $R^4$ und $R^6$ für die gleichen Reste wie $R^4$ und $R^6$ stehen und zusätzlich für Wasserstoff stehen, wobei

$R^9$ für Wasserstoff, Hydroxy, Fluor, Chlor, Brom, Amino, für jeweils geradkettiges oder verzweigtes Alkylamino, Dialkylamino, Alkyl oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen sowie für gegebenenfalls einfach oder mehrfach, gleich oder verschieden substituiertes Phenyl steht, wobei als Substituenten in Frage kommen: Halogen, jeweils geradkettiges oder verzweigtes Alkyl, Alkoxy oder Halogenalkyl mit jeweils bis zu 4 Kohlenstoffatomen und im Fall des Halogenalkyl mit bis zu 9 gleichen oder verschiedenen Halogenatomen,

$R^{10}$ für Wasserstoff, für geradkettiges oder verzweigtes Alkyl mit bis zu 4 Kohlenstoffatomen oder für gegebenenfalls einfach oder mehrfach, gleich oder verschieden substituiertes Phenyl steht, wobei als Phenylsubstituenten die bei $R^9$ genannten in Frage kommen,

$R^{11}$ für Wasserstoff, für Halogenalkyl mit bis zu 4 Kohlenstoffatomen und bis zu 6 gleichen oder verschiedenen Halogenatomen, für jeweils geradkettiges oder verzweigtes Alkyl, Alkenyl oder Alkinyl mit jeweils bis zu 8 Kohlenstoffatomen, sowie für geradkettiges oder verzweigtes Phenylalkyl mit bis zu 4 Kohlenstoffatomen im Alkylteil steht,

$R^{12}$ für Wasserstoff, für jeweils geradkettiges oder verzweigtes Alkyl mit 1–12 C-Atomen, sowie für Alkenyl, Alkinyl, Alkoxyalkyl, Alkylthioalkyl, Alkoxy, Alkylthio, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen, ausserdem für gegebenenfalls einfach oder mehrfach, gleich oder verschieden durch Halogen, niederes Alkyl oder niederes Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten die bei $R^9$ genannten in Frage kommen;

$R^{13}$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall

des Halogenalkyl mit bis zu 9 gleichen oder verschiedenen Halogenatomen steht,

X für Sauerstoff oder Schwefel steht und

n für eine Zahl 0, 1 oder 2 steht,

herbizide Eigenschaften, insbesondere auch selektivherbizide Eigenschaften besitzen.

Überraschenderweise zeigen die erfindungsgemäss zu verwendenden substituierten 5-Amino-1-phenyl-pyrazole der Formel (I) neben einer verbesserten herbiziden Wirkung gegen bestimmte Schadpflanzen auch eine deutlich verbesserte Verträglichkeit gegenüber wichtigen Kulturpflanzen als die aus dem Stand der Technik bekannten 4-Cyano-5-amino-1-phenyl-pyrazole, wie beispielsweise das 4-Cyano-5-propionylamino-1-(2,4,6-trichlorphenyl)-pyrazol, welches eine chemisch und wirkungsmässig naheliegende Verbindung ist.

Die erfindungsgemäss zu verwendenden substituierten 5-Amino-1-phenyl-pyrazole stellen somit eine Bereicherung der Technik dar.

Die erfindungsgemäss zu verwendenden substituierten 5-Amino-1-phenyl-pyrazole sind durch die Formel (I) allgemein definiert. Bevorzugt sind diejenigen Verbindungen der Formel (I) bei welchen

$R^1$ für Wasserstoff, Nitroso, Nitro, Fluor, Chlor, Brom, Methyl, Ethyl, n- und i-Propyl, n-, i-, s- und t-Butyl, für Trifluormethyl, für gegebenenfalls ein- bis dreifach gleich oder verschieden durch Fluor, Chlor, Brom, Nitro, Methyl, Ethyl, Methoxy oder Trifluormethyl substituiertes Phenyl oder für einen der Reste

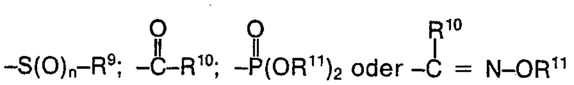

steht,

$R^2$ für Wasserstoff oder einen Rest

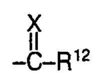

steht,

$R^3$ unabhängig von $R^2$ für die gleichen Reste wie $R^2$ steht und zusätzlich für Methyl, Ethyl, n- und i-Propyl, n-, i-, s- und t-Butyl steht,

$R^4$ und $R^6$ unabhängig voneinander für Cyano, Nitro, Fluor, Chlor, Brom, Iod, Methyl, Ethyl, n- und i-Propyl, n-, i-, s- und t-Butyl, für Methoxy, Ethoxy, Methoxycarbonyl, Ethoxycarbonyl, für Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, für Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder für einen Rest $-S(O)_n-R^{12}$ stehen und

$R^5$, $R^7$ und $R^8$ unabhängig voneinander und von $R^4$ und $R^6$ für die gleichen Reste wie $R^4$ und $R^6$ stehen und zusätzlich für Wasserstoff stehen, wobei

$R^9$ für Wasserstoff, Hydroxy, Fluor, Chlor, Brom, Amino, Methylamino, Dimethylamino, Ethylamino, Diethylamino, Diisopropylamino, Di-n-butylamino, Methyl, Ethyl, Dichlorfluormethyl, Trifluormethyl sowie gegebenenfalls ein- bis dreifach gleich oder verschieden durch Methyl, Methoxy, Trifluormethyl oder Chlor substituiertes Phenyl steht,

$R^{10}$ für Wasserstoff, Methyl, Ethyl, n- und i-Propyl sowie für gegebenenfalls ein- bis dreifach, gleich oder verschieden durch Methyl, Methoxy, Trifluormethyl oder Chlor substituiertes Phenyl steht,

$R^{11}$ für Wasserstoff, Methyl, Ethyl, n- und i-Propyl, n-, i-, s- und t-Butyl, für Alkyl, Butenyl, Propargyl, Benzyl, Chlorethyl oder Bromethyl steht,

$R^{12}$ für Wasserstoff, Methyl, Ethyl, n- und i-Propyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, Heptafluor-n-propyl, für jeweils gegebenenfalls ein- bis vierfach, gleich oder verschieden durch Fluor, Chlor, Brom, Methyl oder Trifluormethyl substituiertes Cyclopropyl, Cyclopentyl oder Cyclohexyl, sowie für jeweils gegebenenfalls ein- bis dreifach, gleich oder verschieden durch Methyl, Methoxy, Chlor oder Trifluormethyl substituiertes Phenyl, Phenoxy, Phenylthio oder Phenylamino, ferner für Undecyl, Vinyl oder Chlorpropyl steht,

$R^{13}$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht,

X für Sauerstoff oder Schwefel steht und

n für eine Zahl 0, 1 oder 2 steht.

Im einzelnen seien ausser den bei den Herstellungsbeispielen genannten Verbindungen die folgenden 5-Amino-1-phenyl-pyrazole der allgemeinen Formel (I) genannt:

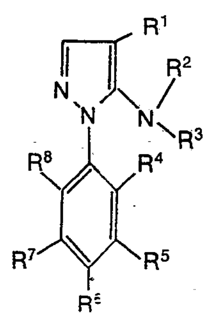

(I)

Tabelle 1

| $R^1$ | $R^2$ | $R^3$ | $R^4$ | $R^5$ | $R^6$ | $R^7$ | $R^8$ |
|---|---|---|---|---|---|---|---|
| H | $CH_3CO$ | H | Cl | H | Cl | H | H |
| H | $CH_3CO$ | $CH_3CO$ | Cl | H | Cl | H | H |
| H | $CH_3CO$ | H | Cl | H | Cl | H | Cl |
| H | $CH_3CO$ | $CH_3$ | Cl | H | Cl | H | Cl |
| H | $CH_3CO$ | H | $NO_2$ | H | $NO_2$ | H | H |
| H | H | H | Cl | H | $CF_3$ | H | Cl |
| H | $CH_3CO$ | H | Cl | H | $OCF_3$ | H | H |
| H | $C_2H_5CO$ | H | $CF_3$ | H | $SCF_3$ | H | H |
| H | $C_2H_5CO$ | H | Cl | Cl | Cl | H | H |
| H | H | H | Cl | Cl | Cl | H | H |
| H | $CH_3CO$ | $CH_3CO$ | Cl | Cl | Cl | H | H |
| H | $CH_3CO$ | $C_2H_5CO$ | Cl | Cl | Cl | H | H |
| H | $C_2H_5CO$ | H | Cl | H | $CF_3$ | H | Cl |
| H | $CH_3CO$ | H | Cl | H | $CF_3$ | H | Cl |
| H | 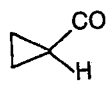 | H | Cl | H | $CF_3$ | H | Cl |
| H | $CH_3OCO$ | H | Cl | H | $CF_3$ | H | Cl |
| H | $n-C_3H_7CO$ | H | Cl | H | $CF_3$ | H | Cl |
| H | $CH_3CO$ | $CH_3CO$ | Cl | H | $CF_3$ | H | Cl |
| H | $C_2H_5CO$ | H | Cl | H | $OCF_3$ | H | H |
| H | 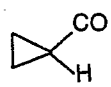 | H | Cl | H | $OCF_3$ | H | H |
| H | $n-C_4H_9-CO$ | H | Cl | H | $OCF_3$ | H | H |
| H | $C_2H_5CO$ | H | Cl | H | $SCF_3$ | H | Cl |
| H | $n-C_3H_7CO$ | H | Cl | H | $SCF_3$ | H | Cl |
| H | 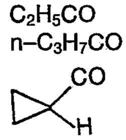 | H | Cl | H | $SCF_3$ | H | Cl |
| H | $CH_3CO$ | H | Cl | H | $SCF_3$ | H | Cl |
| H | $CH_3OCO$ | H | Cl | H | $SCF_3$ | H | Cl |
| H | $CH_3OCO$ | H | Cl | H | $OCF_3$ | H | H |
| H | $C_2H_5CO$ | H | Cl | Cl | Cl | H | Cl |
| H | $CH_3CO$ | H | Cl | Cl | Cl | H | Cl |
| H | $CH_3CO$ | H | Cl | Cl | Cl | H | H |
| H | 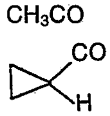 | H | Cl | Cl | Cl | H | Cl |
| H | $CH_3OCO$ | H | Cl | Cl | Cl | H | Cl |
| H | $CH_3CO$ | H | Cl | Cl | $CF_3$ | H | Cl |
| H | $C_2H_5CO$ | H | Cl | Cl | $CF_3$ | H | Cl |
| H | $n-C_3H_7CO$ | H | Cl | Cl | $CF_3$ | H | Cl |
| H | 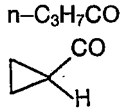 | H | Cl | Cl | $CF_3$ | H | Cl |
| H | $CH_3OCO$ | H | Cl | Cl | $CF_3$ | H | Cl |
| H | $-CO-NHCH_3$ | H | Cl | Cl | Cl | H | H |
| H | $-CO-NHCH_3$ | H | Cl | H | $CF_3$ | H | Cl |
| H | $CH_3CO$ | H | Cl | Cl | $SCF_3$ | H | H |
| H | $CH_3CO$ | H | F | F | $OCF_3$ | H | F |
| H | $C_2H_5CO$ | $CH_3CO$ | F | F | $OCF_3$ | F | F |
| Cl | H | H | Cl | Cl | Cl | H | H |
| Cl | H | H | Cl | H | Cl | H | H |
| Cl | H | H | Cl | H | $CF_3$ | H | Cl |
| Cl | H | H | Cl | H | $SCF_3$ | H | Cl |
| Cl | $CH_3CO$ | H | Cl | Cl | Cl | H | H |
| Cl | $CH_3CO$ | H | Cl | Cl | $OCF_3$ | H | H |
| Cl | $CH_3CO$ | $CH_3$ | Cl | Cl | $SCF_3$ | H | H |

Fortsetzung Tabelle 1

| $R^1$ | $R^2$ | $R^3$ | $R^4$ | $R^5$ | $R^6$ | $R^7$ | $R^8$ |
|---|---|---|---|---|---|---|---|
| Cl | $C_2H_5CO$ | H | F | F | $OCF_3$ | F | F |
| Cl | $C_2H_5CO$ | H | Br | H | $SCF_3$ | H | H |
| Cl | $C_2H_5CO$ | H | Cl | H | $SCF_3$ | H | Cl |
| Cl | $C_2H_5CO$ | $CH_3CO$ | Cl | Cl | $SCF_3$ | H | H |
| Cl | $CH_3CO$ | $CH_3CO$ | Cl | Cl | Cl | H | H |
| Cl | $CH_3CO$ | $C_2H_5CO$ | Cl | Cl | Cl | H | H |
| Cl | $-CO-NHCH_3$ | H | Cl | Cl | $OCF_3$ | H | H |
| Cl | $-CO-OC_2H_5$ | H | Br | H | $OCF_3$ | H | Br |
| Cl | $-CO-C_6H_5$ | H | $NO_2$ | H | $NO_2$ | H | $NO_2$ |
| Cl | $-CO-CF_3$ | H | $NO_2$ | H | $NO_2$ | H | H |
| Br | $CH_3CO$ | H | Cl | Cl | Cl | H | H |
| Br | $CH_3OCH_2CO$ | H | Cl | H | Cl | H | Cl |
| Br | $CH_3SCH_2-CO$ | H | $NO_2$ | H | $NO_2$ | H | H |
| Br | $CH_3CO$ | $CH_3CO$ | Cl | Cl | $CF_3$ | H | H |
| Br | $C_2H_5CO$ | $CH_3CO$ | Cl | H | $CF_3$ | H | Cl |
| Br | $CH_3CO$ | H | Cl | H | $OCF_3$ | H | Cl |
| Br | 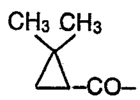 | H | F | H | $OCF_3$ | H | F |
| Br | 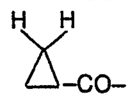 | H | F | F | $OCF_3$ | H | F |
| Br | 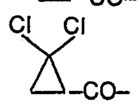 | H | F | F | $SCF_3$ | H | F |
| Br | H | H | F | H | $SCF_3$ | H | F |
| Br | $C_2H_5CO$ | H | Cl | H | Cl | H | Cl |
| Br | 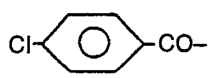 | H | Cl | H | Cl | H | H |
| Br | 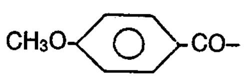 | H | Cl | Cl | Cl | H | H |
| Br | $CH_3CO$ | $CH_3$ | Cl | H | $SO_2CF_3$ | H | Cl |
| Br | $F_3CCO$ | H | Cl | H | $SOCF_3$ | H | Cl |
| Br | $ClCH_2CO$ | H | $CF_3$ | H | $CF_3$ | H | $CF_3$ |
| Br | $Cl_2CHCO$ | H | $CF_3$ | H | $SO_2CF_3$ | H | $CF_3$ |
| I | H | H | Cl | Cl | Cl | H | H |
| I | H | H | Cl | H | Cl | H | Cl |
| I | H | H | Cl | H | $CF_3$ | H | Cl |
| I | $CH_3CO$ | H | Cl | Cl | Cl | H | H |
| I | $CH_3CO$ | H | Cl | H | $CF_3$ | H | Cl |
| I | $CH_3CO$ | $CH_3CO$ | Cl | H | $OCF_3$ | H | Cl |
| I | $CH_3CO$ | $CH_3CO$ | $CF_3$ | H | $SCF_3$ | H | $CF_3$ |
| I | $C_2H_5CO$ | H | $CF_3$ | H | $SCF_3$ | H | H |
| I | $C_2H_5CO$ | $CH_3CO$ | Br | H | $CF_3$ | H | Br |
| I | $C_2H_5CO$ | $CH_3$ | Cl | Cl | Cl | H | H |
| I | $C_6H_5CO$ | H | F | F | $OCF_3$ | F | F |
| I | 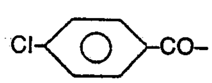 | H | Cl | H | $OCF_3$ | H | Cl |
| I | $F_3C-CO$ | H | F | H | $OCF_3$ | H | F |
| I | $ClCH_2CO$ | H | Cl | H | $SCF_3$ | H | Cl |
| I | $Cl_2CH-CO$ | H | Cl | H | $SO_2CF_3$ | H | Cl |
| I | $CH_3-CH-CO$<br>\|<br>Cl | H | F | F | $SO_2CF_3$ | F | F |
| I | 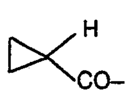 | H | Cl | Cl | Cl | H | H |

Fortsetzung Tabelle 1

| $R^1$ | $R^2$ | $R^3$ | $R^4$ | $R^5$ | $R^6$ | $R^7$ | $R^8$ |
|---|---|---|---|---|---|---|---|
| $CF_3$ | H | H | Cl | H | Cl | H | Cl |
| $CF_3$ | H | H | $NO_2$ | H | $NO_2$ | H | $NO_2$ |
| $CF_3$ | $CH_3CO$ | H | Cl | H | $CF_3$ | H | Cl |
| $CF_3$ | $CH_3CO$ | H | Cl | H | $OCF_3$ | H | Cl |
| $CF_3$ | $CH_3CO$ | $CH_3$ | Cl | Cl | $CF_3$ | H | H |
| $CF_3$ | $CH_3CO$ | $CH_3CO$ | Cl | Cl | Cl | H | H |
| $CF_3$ | $C_2H_5CO$ | H | Cl | H | $SCF_3$ | H | Cl |
| $CF_3$ | $C_2H_5CO$ | H | F | F | $SCF_3$ | H | F |
| $CF_3$ | $C_2H_5CO$ | $CH_3CO$ | F | F | $OCF_3$ | F | F |
| $CF_3$ | $C_6H_5CO$ | H | Cl | Cl | $SCF_3$ | Cl | Cl |
| $CF_3$ | $CH_3NHCO$ | H | Cl | H | $SO_2CH_3$ | H | Cl |
| $CF_3$ | 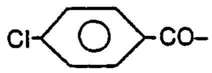 | H | Cl | H | $SO_2CF_3$ | H | Cl |
| $CF_3$ | 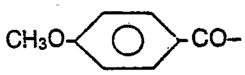 | H | Cl | Cl | Cl | H | H |
| $CF_3$ | 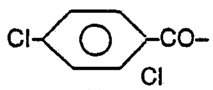 | H | Cl | H | Cl | H | Cl |
| $CF_3$ | 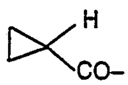 | H | Cl | Cl | Cl | H | H |
| $CF_3$ | $C_6H_5OCO$ | H | $CF_3$ | H | $CF_3$ | H | H |
| $CF_3$ | $C_2H_5OCO$ | H | Cl | H | $CF_3$ | H | Cl |
| $NO_2$ | H | H | Cl | Cl | Cl | H | H |
| $NO_2$ | H | H | Cl | F | Cl | H | Cl |
| $NO_2$ | H | H | Cl | H | $CF_3$ | H | Cl |
| $NO_2$ | H | H | Cl | H | $OCF_3$ | H | Cl |
| $NO_2$ | H | H | Cl | H | $SCF_3$ | H | Cl |
| $NO_2$ | $CH_3CO$ | H | Cl | Cl | Cl | H | H |
| $NO_2$ | $CH_2CO$ | H | Cl | H | $CF_3$ | H | Cl |
| $NO_2$ | $CH_3CO$ | H | $CF_3$ | H | $CF_3$ | H | $CF_3$ |
| $NO_2$ | $CH_3CO$ | $CH_3CO$ | $CF_3$ | H | $CF_3$ | H | H |
| $NO_2$ | $CH_3CO$ | $CH_3CO$ | Cl | Cl | Cl | H | H |
| $NO_2$ | $CH_3CO$ | $HH_3$ | Cl | H | Cl | H | Cl |
| $NO_2$ | $CH_3CO$ | $CH_3$ | $CF_3$ | H | $CF_3$ | H | H |
| $NO_2$ | $C_2H_5CO$ | H | Cl | H | Cl | H | Cl |
| $NO_2$ | $C_2H_5CO$ | $CH_3CO$ | Cl | Cl | Cl | H | H |
| $NO_2$ | $C_2H_5CO$ | H | F | F | F | F | F |
| $NO_2$ | $C_2H_5CO$ | H | F | F | $CF_3$ | F | F |
| $NO_2$ | $C_2H_5CO$ | H | Cl | Cl | Cl | H | H |
| $NO_2$ | $C_6H_5CO$ | H | Cl | H | Cl | H | H |
| $NO_2$ | $CH_3NHCo$ | H | Cl | Cl | Cl | H | H |
| $NO_2$ | $CH_3NHCO$ | $CH_3$ | Cl | H | Cl | H | Cl |
| $NO_2$ | $C_6H_5CO$ | H | Cl | Cl | Cl | Cl | Cl |
| $NO_2$ | 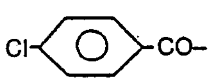 | H | F | F | F | F | F |
| $NO_2$ | 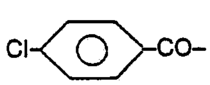 | H | F | H | $CF_3$ | H | F |
| $NO_2$ | 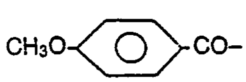 | H | Cl | H | $CF_3$ | H | Cl |
| $NO_2$ | $C_2H_5O–CO$ | H | Cl | Cl | $CF_3$ | H | H |
| $NO_2$ | $C_2H_5O–CO$ | H | Cl | H | $OCF_3$ | H | Cl |
| $NO_2$ | $C_6H_5O–CO$ | H | Cl | H | $OCF_3$ | H | H |
| $NO_2$ | $C_6H_5O–CO$ | H | Cl | Cl | Cl | H | H |
| $NO_2$ | 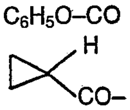 | H | Cl | H | $SO_2CH_3$ | H | Cl |

Fortsetzung Tabelle 1

| $R^1$ | $R^2$ | $R^3$ | $R^4$ | $R^5$ | $R^6$ | $R^7$ | $R^8$ |
|---|---|---|---|---|---|---|---|
| $NO_2$ | 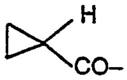 | H | Cl | H | $SO_2CF_3$ | H | Cl |
| $NO_2$ | $CH_3OCH_2CO$ | H | Cl | Cl | Cl | H | H |
| $NO_2$ | $ClCH_2CO$– | H | Cl | H | Cl | H | H |
| $NO_2$ | $Cl_2CHCO$ | H | Cl | H | Cl | H | Cl |
| $NO_2$ | $Cl_2CHCO$ | H | Cl | H | $CF_3$ | H | Cl |
| NO | H | H | Cl | Cl | Cl | H | H |
| NO | H | H | Cl | H | Cl | H | Cl |
| NO | H | H | Cl | H | $CF_3$ | H | Cl |
| NO | $CH_3CO$ | H | Cl | Cl | Cl | H | H |
| NO | $CH_3CO$ | H | Cl | H | Cl | H | Cl |
| NO | $CH_3CO$ | H | Cl | H | $CF_3$ | H | Cl |
| NO | $CH_3CO$ | $CH_3CO$ | Cl | H | $OCF_3$ | H | Cl |
| NO | $CH_3CO$ | $CH_3$ | Cl | Cl | $CF_3$ | Cl | Cl |
| NO | $CH_3CO$ | $C_2H_5CO$ | F | H | $OCF_3$ | H | F |
| NO | $C_2H_5CO$ | H | F | F | $OCF_3$ | F | F |
| NO | $C_2H_5CO$ | H | F | F | F | F | F |
| NO | $ClCH_2CO$ | H | Cl | H | $SO_2CH_3$ | H | Cl |
| NO | $Cl_2CHCO$ | H | Cl | H | $SO_2CF_3$ | H | Cl |
| NO | $CF_3CO$ | H | Cl | Cl | $SO_2CF_3$ | H | H |
| NO | $CH_3NHCO$ | H | Cl | H | Cl | H | H |
| NO | $C_2H_5OCO$ | H | Cl | Cl | Cl | H | H |
| NO | $CF_3CO$ | H | Cl | Cl | Cl | H | H |
| NO | $C_6H_5CO$ | H | Cl | Cl | Cl | H | H |
| H–CO– | H | H | Cl | Cl | Cl | H | H |
| H–CO– | H | H | Cl | H | Cl | H | Cl |
| H–CO– | H | H | Cl | H | $CF_3$ | H | Cl |
| H–CO– | $CH_3CO$ | H | Cl | H | $CF_3$ | H | Cl |
| H–CO– | $CH_3CO$ | H | Cl | H | $OCF_3$ | H | Cl |
| H–CO– | $CH_3CO$ | H | Cl | H | $SCF_3$ | H | Cl |
| H–CO– | $CH_3CO$ | $CH_3$ | Cl | Cl | Cl | H | Cl |
| H–CO– | $CH_3CO$ | $CH_3$ | Cl | H | $CF_3$ | H | Cl |
| H–CO– | $C_2H_5CO$ | H | Cl | Cl | Cl | H | H |
| H–CO– | $C_2H_5CO$ | H | Cl | H | Cl | H | Cl |
| H–CO– | $C_2H_5CO$ | $CH_3CO$ | Cl | Cl | Cl | H | Cl |
| H–CO– | $CH_3CO$ | $CH_3CO$ | Cl | H | $CF_3$ | H | Cl |
| H–CO– | $C_2H_5O$–CO | H | Cl | H | $CF_3$ | H | H |
| H–CO– | $C_2H_5OCO$ | H | F | H | $CF_3$ | H | F |
| H–CO– | 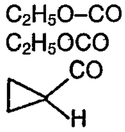 | H | Cl | Cl | Cl | H | H |
| H–CO– | $C_6H_5CO$ | H | Cl | H | Cl | H | Cl |
| H–CO– | $C_6H_5OCO$ | H | F | F | F | F | F |
| $CH_3$–CO | H | H | $NO_2$ | H | $NO_2$ | H | H |
| $CH_3$–CO | H | H | $NO_2$ | H | $CF_3$ | H | H |
| $CH_3$–CO | $CH_3CO$ | H | Cl | Cl | Cl | H | H |
| $CH_3$–CO | $CH_3CO$ | H | Cl | H | Cl | H | Cl |
| $CH_3$–CO | $CH_3CO$ | $CH_3CO$ | Cl | H | Cl | H | H |
| $CH_3$–CO | $C_2H_5CO$ | H | Cl | H | $CF_3$ | H | H |
| $CH_3$–CO | $C_2H_5CO$ | H | Cl | H | $CF_3$ | H | Cl |
| $CH_3$–CO | $C_2H_5CO$ | H | Cl | Cl | Cl | H | H |
| $CH_3$–CO | $CF_3CO$ | H | Cl | H | Cl | H | Cl |
| $CH_3$–CO– | $CF_3CO$ | H | Cl | H | $CF_3$ | H | H |
| $CH_3$–CO– | $ClCH_2CO$ | H | Cl | H | $OCF_3$ | H | Cl |
| $CH_3$–CO– | $CH_3OCH_2CO$ | H | Cl | H | $OCF_3$ | H | Cl |
| $CH_3$–CO– | $CH_3SCH_2CO$ | H | Cl | H | $SCF_3$ | H | Cl |
| $CH_3$–CO– | $Cl_2CHCO$ | H | F | H | $SCF_3$ | H | F |
| $CH_3$–CO– | $Cl_2CHCO$ | H | F | H | $SO_2CF_3$ | H | F |
| $CH_3$–CO– | $CH_3$–CHCO<br>\|<br>Cl | H | F | H | $SO_2CF_3$ | H | F |

Fortsetzung Tabelle 1

| $R^1$ | $R^2$ | $R^3$ | $R^4$ | $R^5$ | $R^6$ | $R^7$ | $R^8$ |
|---|---|---|---|---|---|---|---|
| $CH_3$–CO– | Br–$CH_2$CO | H | F | F | F | F | F |
| HO–N=CH– | H | H | Cl | H | Cl | H | H |
| HO–N=CH– | H | H | Cl | H | $CF_3$ | H | H |
| HO–N=CH– | H | H | Cl | Cl | Cl | H | H |
| HO–N=CH– | H | H | F | F | F | F | F |
| HO–N=CH– | $CH_3CO$ | H | Cl | Cl | Cl | H | H |
| HO–N=CH– | $CH_3CO$ | H | Cl | H | Cl | H | Cl |
| HO–N=CH– | $C_2H_5CO$ | H | Cl | H | $CF_3$ | H | Cl |
| HO–N=CH– | $C_2H_5CO$ | H | Cl | H | Cl | H | H |
| HO–N=CH– | $C_2H_5CO$ | $C_2H_5CO$ | Cl | H | $OCF_3$ | H | Cl |
| HO–N=CH– | $CH_3CO$ | $CH_3CO$ | Cl | H | $OCF_3$ | H | Cl |
| HO–N=CH– | $CF_3CO$ | $CF_3CO$ | Cl | H | $SCF_3$ | H | Cl |
| HO–N=CH– | $CF_3CO$ | H | Cl | H | $OCH_3$ | H | Cl |
| HO–N=CH– | $ClCH_2CO$ | H | Cl | H | $SCH_3$ | H | Cl |
| HO–N=CH– | $Cl_2CHCO$ | H | Cl | H | $SO_2CH_3$ | H | Cl |
| HO–N=CH– | $CH_3OCH_2CO$ | H | Cl | H | $SO_2Ch_3$ | H | Cl |
| HO–N=CH– | $CH_3OCH_2CO$ | H | Cl | H | $SO_2CF_3$ | H | Cl |
| HO–N=CH– | $CH_3SCH_2CO$ | H | Cl | Cl | Cl | H | H |
| $CH_3$O–N=CH– | H | H | $CF_3$ | H | $CF_3$ | H | H |
| $CH_3$O–N=CH– | H | H | F | H | F | H | F |
| $CH_3$O–N=CH– | H | H | F | H | F | H | F |
| $CH_3$O–N=CH– | $CH_3CO$ | H | F | F | F | F | F |
| $CH_3$O–N=CH– | $CH_3CO$ | H | Cl | H | Cl | H | Cl |
| $CH_3$O–N=CH– | $CH_3CO$ | H | Cl | H | Cl | H | H |
| $CH_3$O–N=CH– | $C_2H_5CO$ | H | Cl | Cl | Cl | H | H |
| $CH_3$O–N=CH– | $C_2H_5O$ | H | $CF_3$ | H | $CF_3$ | H | H |
| $CH_3$O–N=CH– | $C_2H_5CO$ | H | $CF_3$ | H | $CF_3$ | H | $CF_3$ |
| $CH_3$O–N=CH– | $CF_3CO$ | H | Cl | H | $CF_3$ | H | H |
| $CH_3$O–N=CH– | $C_6H_5CO$ | H | Cl | H | $CF_3$ | H | Cl |
| $CH_3$O–N=CH– | $ClCH_2CO$ | H | Cl | H | $OCF_3$ | H | Cl |
| $CH_3$O–N=CH– | $ICH_2CO$ | H | Cl | Cl | Cl | H | H |
| $CH_3$O–N=CH– | $BrCH_2CO$ | H | F | F | F | F | F |
| $CH_3$O–N=CH– | $C_2H_5OCO$ | H | Cl | H | $OCF_3$ | H | H |
| $CH_3$O–N=CH– | $CH_3CO$ | $CH_3$ | Cl | H | $SCF_3$ | H | Cl |
| $CH_3$O–N=CH– | $CH_3CO$ | H | Cl | H | $CF_3$ | H | Cl |
| $CH_3$O–N=CH– | $C_2H_5CO$ | H | Cl | H | $CF_3$ | H | Cl |
| $H_2NSO_2$– | H | H | $CF_3$ | H | $CF_3$ | H | H |
| $H_2NSO_2$– | H | H | Cl | H | Cl | H | Cl |
| $H_2NSO_2$– | H | H | Cl | H | Cl | H | H |
| $H_2NSO_2$– | $CH_3CO$ | H | Cl | Cl | Cl | H | Cl |
| $H_2NSO_2$– | $CH_3CO$ | H | F | F | F | F | F |
| $H_2NSO_2$– | $CH_3CO$ | H | F | F | $CF_3$ | F | F |
| $H_2NSO_2$– | $C_2H_5CO$ | H | F | F | $OCF_3$ | F | F |
| $H_2NSO_2$– | $C_2H_5CO$ | H | F | F | $SCF_3$ | F | F |
| $H_2NSO_2$– | $C_2H_5CO$ | H | Cl | H | $OCF_3$ | H | Cl |
| $H_2NSO_2$– | $CH_3CO$ | $CH_3CO$ | Cl | H | $SCF_3$ | H | Cl |
| $H_2NSO_2$– | $CH_3CO$ | $CH_3$ | Cl | H | $SOCF_3$ | H | Cl |
| $H_2NSO_2$– | $ClCH_2CO$ | H | Cl | H | $SO_2CF_3$ | H | Cl |
| $H_2NSO_2$– | $BrCH_2CO$ | H | Cl | H | $SO_2CH_3$ | H | H |
| $H_2NSO_2$– | $ICH_2CO$ | H | Cl | H | $SO_2CF_3$ | H | H |
| $H_2NSO_2$– | $C_6H_5CO$ | H | Cl | H | $CF_3$ | H | Cl |
| $H_2NSO_2$– | $C_2H_5CO$ | H | Cl | H | $CF_3$ | H | H |
| $H_2NSO_2$– | $CF_3CO$ | H | Cl | Cl | Cl | H | H |
| H | $C_2H_5CO$ | H | Cl | H | $OCF_3$ | H | Cl |
| H | $CH_3CO$ | H | Cl | H | $OCF_3$ | H | Cl |
| H | 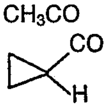 | H | Cl | H | $OCF_3$ | H | Cl |
| H | n–$C_3H_7CO$ | H | Cl | H | $OCF_3$ | H | Cl |
| H | $CH_3OCO$ | H | Cl | H | $OCF_3$ | H | Cl |
| H | $CH_3OCO$ | H | Cl | H | $SO_2CH_3$ | H | Cl |
| H | $CH_3CO$ | H | Cl | H | $SO_2CH_3$ | H | Cl |

Fortsetzung Tabelle 1

| $R^1$ | $R^2$ | $R^3$ | $R^4$ | $R^5$ | $R^6$ | $R^7$ | $R^8$ |
|---|---|---|---|---|---|---|---|
| H | $C_2H_5CO$ | H | Cl | H | $SO_2CF_3$ | H | Cl |
| H | 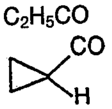 | H | Cl | H | $SO_2CH_3$ | H | Cl |
| H | n–$C_3H_7CO$ | H | Cl | H | $SO_2CF_3$ | H | Cl |
| H | n–$C_3H_7CO$ | H | Cl | H | $SO_2CClF_2$ | H | Cl |
| H | $CH_3CO$ | H | Cl | H | $SO_2CClF_2$ | H | Cl |
| H | $C_2H_5CO$ | H | Cl | H | $SO_2CClF_2$ | H | Cl |
| H | $CH_3CO$ | H | Br | H | $OCF_3$ | H | Br |
| H | $C_2H_5CO$ | H | Br | H | $OCF_3$ | H | H |
| H | n–$C_3H_7CO$ | H | Br | H | $OCF_3$ | H | H |
| H | 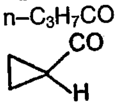 | H | Br | H | $OCF_3$ | H | H |
| H | $CH_3OCO$ | H | Br | H | $OCF_3$ | H | H |
| H | $CH_3CO$ | H | Br | H | $SCF_3$ | H | Br |
| H | $CH_3CO$ | H | Br | H | $SCF_3$ | H | H |
| H | $C_2H_5CO$ | H | Br | H | $SCF_3$ | H | H |
| H | n–$C_3H_7CO$ | H | Br | H | $SCF_3$ | H | H |
| H | $C_2H_5CO$ | H | Br | H | Br | H | Br |
| H | $C_2H_5CO$ | H | Cl | H | Cl | H | H |
| H | $C_2H_5CO$ | H | Br | H | Br | H | H |
| NO | $C_2H_5CO$ | H | Cl | H | $CF_3$ | H | Cl |
| NO | $CH_3CO$ | H | Cl | H | $CF_3$ | H | Cl |
| NO | 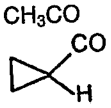 | H | Cl | H | $CF_3$ | H | Cl |
| NO | $CH_3OCO$ | H | Cl | H | $CF_3$ | H | Cl |
| NO | n–$C_3H_7CO$ | H | Cl | H | $CF_3$ | H | Cl |
| NO | $CH_3CO$ | $CH_3CO$ | Cl | H | $CF_3$ | H | Cl |
| NO | $C_2H_5CO$ | H | Cl | H | $OCF_3$ | H | H |
| NO | 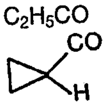 | H | Cl | H | $OCF_3$ | H | H |
| NO | n–$C_4H_9CO$ | H | Cl | H | $OCF_3$ | H | H |
| NO | $C_2H_5CO$ | H | Cl | H | $SCF_3$ | H | Cl |
| NO | n–$C_3H_7CO$ | H | Cl | H | $SCF_3$ | H | Cl |
| NO | 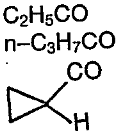 | H | Cl | H | $SCF_3$ | H | Cl |
| NO | $CH_3CO$ | H | Cl | H | $SCF_3$ | H | Cl |
| NO | $CH_3OCO$ | H | Cl | H | $SCF_3$ | H | Cl |
| NO | $CH_3OCO$ | H | Cl | H | $OCF_3$ | H | H |
| NO | $C_2H_5CO$ | H | Cl | Cl | Cl | H | Cl |
| NO | $CH_3CO$ | H | Cl | Cl | Cl | H | Cl |
| NO | $CH_3CO$ | H | Cl | Cl | Cl | H | H |
| NO | 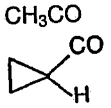 | H | Cl | Cl | Cl | H | Cl |
| NO | $CH_3OCO$ | H | Cl | Cl | Cl | H | Cl |
| NO | $CH_3CO$ | H | Cl | Cl | $CF_3$ | H | Cl |
| NO | $C_2H_5CO$ | H | Cl | Cl | $CF_3$ | H | Cl |
| NO | n–$C_3H_7CO$ | H | Cl | Cl | $CF_3$ | H | Cl |
| NO | 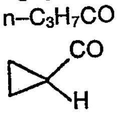 | H | Cl | Cl | $CF_3$ | H | Cl |
| NO | $CH_3OCO$ | H | Cl | Cl | $CF_3$ | H | Cl |
| NO | $C_2H_5CO$ | H | Cl | H | $OCF_3$ | H | Cl |
| NO | $CH_3CO$ | H | Cl | H | $OCF_3$ | H | Cl |
| NO | 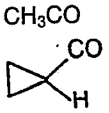 | H | Cl | H | $OCF_3$ | H | Cl |

Fortsetzung Tabelle 1

| $R^1$ | $R^2$ | $R^3$ | $R^4$ | $R^5$ | $R^6$ | $R^7$ | $R^8$ |
|---|---|---|---|---|---|---|---|
| NO | $n-C_3H_7CO$ | H | Cl | H | $OCF_3$ | H | Cl |
| NO | $CH_3OCO$ | H | Cl | H | $OCF_3$ | H | Cl |
| NO | $CH_3OCO$ | H | Cl | H | $OCF_3$ | H | Cl |
| NO | $CH_3CO$ | H | Cl | H | $SO_2CF_3$ | H | Cl |
| NO | $C_2H_5CO$ | H | Cl | H | $SO_2CF_3$ | H | Cl |
| NO | 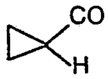 | H | Cl | H | $SO_2CF_3$ | H | Cl |
| NO | $n-C_3H_7CO$ | H | Cl | H | $SO_2CF_3$ | H | Cl |
| NO | $n-C_3H_7CO$ | H | Cl | H | $SO_2CClF_2$ | H | Cl |
| NO | $CH_3CO$ | H | Cl | H | $SO_2CClF_2$ | H | Cl |
| NO | $C_2H_5CO$ | H | Cl | H | $SO_2CClF_2$ | H | Cl |
| NO | $CH_3CO$ | H | Br | H | $OCF_3$ | H | Br |
| NO | $C_2H_5CO$ | H | Br | H | $OCF_3$ | H | H |
| NO | $n-C_3H_7CO$ | H | Br | H | $OCF_3$ | H | H |
| NO | 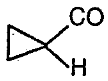 | H | Br | H | $OCF_3$ | H | H |
| NO | $CH_3OCO$ | H | Br | H | $OCF_3$ | H | H |
| NO | $CH_3CO$ | H | Br | H | $SCF_3$ | H | Br |
| NO | $CH_3CO$ | H | Br | H | $SCF_3$ | H | Br |
| NO | $C_2H_5CO$ | H | Br | H | $SCF_3$ | H | H |
| NO | $n-C_3H_7CO$ | H | Br | H | $SCF_3$ | H | H |
| NO | $C_2H_5CO$ | H | Br | H | Br | H | Br |
| NO | $C_2H_5CO$ | H | Cl | H | Cl | H | H |
| NO | $C_2H_5CO$ | H | Br | H | Br | H | H |
| $NO_2$ | $C_2H_5CO$ | H | Cl | H | $OCF_3$ | H | Cl |
| $NO_2$ | $CH_3CO$ | H | Cl | H | $OCF_3$ | H | Cl |
| $NO_2$ | 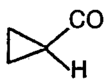 | H | Cl | H | $OCF_3$ | H | Cl |
| $NO_2$ | $n-C_3H_7CO$ | H | Cl | H | $OCF_3$ | H | Cl |
| $NO_2$ | $CH_3OCO$ | H | Cl | H | $OCF_3$ | H | Cl |
| $NO_2$ | $CH_3OCO$ | H | Cl | H | $SO_2CF_3$ | H | Cl |
| $NO_2$ | $CH_3CO$ | H | Cl | H | $SO_2CF_3$ | H | Cl |
| $NO_2$ | $C_2H_5CO$ | H | Cl | H | $SO_2CF_3$ | H | Cl |
| $NO_2$ | 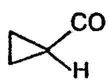 | H | Cl | H | $SO_2CF_3$ | H | Cl |
| $NO_2$ | $n-C_3H_7CO$ | H | Cl | H | $SO_2CF_3$ | H | Cl |
| $NO_2$ | $n-C_3H_7CO$ | H | Cl | H | $SO_2CClF_2$ | H | Cl |
| $NO_2$ | $CH_3CO$ | H | Cl | H | $SO_2CClF_2$ | H | Cl |
| $NO_2$ | $C_2H_5CO$ | H | Cl | H | $SO_2CClF_2$ | H | Cl |
| $NO_2$ | $CH_3CO$ | H | Br | H | $OCF_3$ | H | Br |
| $NO_2$ | $C_2H_5CO$ | H | Br | H | $OCF_3$ | H | H |
| $NO_2$ | $n-C_3H_7CO$ | H | Br | H | $OCF_3$ | H | H |
| $NO_2$ | 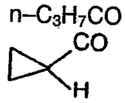 | H | Br | H | $OCF_3$ | H | H |
| $NO_2$ | $CH_3OCO$ | H | Br | H | $OCF_3$ | H | H |
| $NO_2$ | $CH_3CO$ | H | Br | H | $SCF_3$ | H | Br |
| $NO_2$ | $CH_3CO$ | H | Br | H | $SCF_3$ | H | H |
| $NO_2$ | $C_2H_5CO$ | H | Br | H | $SCF_3$ | H | H |
| $NO_2$ | $n-C_3H_7CO$ | H | Br | H | $SCF_3$ | H | H |
| $NO_2$ | $C_2H_5CO$ | H | Br | H | Br | H | Br |
| $NO_2$ | $C_2H_5CO$ | H | Cl | H | Cl | H | H |
| $NO_2$ | $C_2H_5CO$ | H | Br | H | Br | H | H |
| $NO_2$ | $C_2H_5CO$ | H | Cl | H | $CF_3$ | H | Cl |
| $NO_2$ | $CH_3CO$ | H | Cl | H | $CF_3$ | H | Cl |
| $NO_2$ | 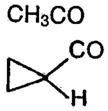 | H | Cl | H | $CF_3$ | H | Cl |

Fortsetzung Tabelle 1

| $R^1$ | $R^2$ | $R^3$ | $R^4$ | $R^5$ | $R^6$ | $R^7$ | $R^8$ |
|---|---|---|---|---|---|---|---|
| $NO_2$ | $CH_3OCO$ | H | Cl | H | $CF_3$ | H | Cl |
| $NO_2$ | n–$C_3H_7CO$ | H | Cl | H | $CF_3$ | H | Cl |
| $NO_2$ | $CH_3CO$ | $CH_3CO$ | Cl | H | $CF_3$ | H | Cl |
| $NO_2$ | $C_2H_5CO$ | H | Cl | H | $OCF_3$ | H | H |
| $NO_2$ | 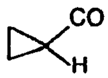 | H | Cl | H | $OCF_3$ | H | H |
| $NO_2$ | n–$C_4H_9CO$ | H | Cl | H | $OCF_3$ | H | H |
| $NO_2$ | $C_2H_5CO$ | H | Cl | H | $SCF_3$ | H | Cl |
| $NO_2$ | n–$C_3H_7CO$ | H | Cl | H | $SCF_3$ | H | Cl |
| $NO_2$ | 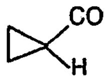 | H | Cl | H | $SCF_3$ | H | Cl |
| $NO_2$ | $CH_3CO$ | H | Cl | H | $SCF_3$ | H | Cl |
| $NO_2$ | $CH_3OCO$ | H | Cl | H | $SCF_3$ | H | Cl |
| $NO_2$ | $CH_3OCO$ | H | Cl | H | $OCF_3$ | H | H |
| $NO_2$ | $C_2H_5CO$ | H | Cl | Cl | Cl | H | Cl |
| $NO_2$ | $CH_3CO$ | H | Cl | Cl | Cl | H | Cl |
| $NO_2$ | $CH_3CO$ | H | Cl | Cl | Cl | H | H |
| $NO_2$ | 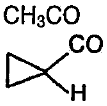 | H | Cl | Cl | Cl | H | Cl |
| $NO_2$ | $CH_3OCO$ | H | Cl | Cl | Cl | H | Cl |
| $NO_2$ | $CH_3CO$ | H | Cl | Cl | $CF_3$ | H | Cl |
| $NO_2$ | $C_2H_5CO$ | H | Cl | Cl | $CF_3$ | H | Cl |
| $NO_2$ | n–$C_3H_7CO$ | H | Cl | Cl | $CF_3$ | H | Cl |
| $NO_2$ | 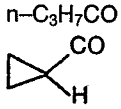 | H | Cl | Cl | $CF_3$ | H | Cl |
| $NO_2$ | $CH_3OCO$ | H | Cl | Cl | $CF_3$ | H | Cl |
| $PO(OC_2H_5)_2$ | $C_2H_5CO$ | H | Cl | H | $CF_3$ | H | Cl |
| $PO(OC_2H_5)_2$ | $CH_3CO$ | H | Cl | H | $CF_3$ | H | Cl |
| $PO(OC_2H_5)_2$ | 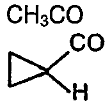 | H | Cl | H | $CF_3$ | H | Cl |
| $PO(OC_2H_5)_2$ | $CH_3CO$ | H | Cl | H | $CF_3$ | H | Cl |
| $PO(OC_2H_5)_2$ | n–$C_3H_7CO$ | H | Cl | H | $CF_3$ | H | Cl |
| $PO(OC_2H_5)_2$ | $CH_3CO$ | $CH_3CO$ | Cl | H | $CF_3$ | H | Cl |
| $PO(OC_2H_5)_2$ | $C_2H_5CO$ | H | Cl | H | $OCF_3$ | H | H |
| $PO(OC_2H_5)_2$ | 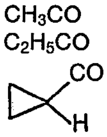 | H | Cl | H | $OCF_3$ | H | H |
| $PO(OC_2H_5)_2$ | n–$C_4H_9CO$ | H | Cl | H | $OCF_3$ | H | H |
| $PO(OC_2H_5)_2$ | $C_2H_5CO$ | H | Cl | H | $SCF_3$ | H | Cl |
| $PO(OC_2H_5)_2$ | n–$C_3H_7CO$ | H | Cl | H | $SCF_3$ | H | Cl |
| $PO(OC_2H_5)_2$ | 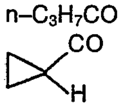 | H | Cl | H | $SCF_3$ | H | Cl |
| $PO(OC_2H_5)_2$ | $CH_3CO$ | H | Cl | H | $SCF_3$ | H | Cl |
| $PO(OC_2H_5)_2$ | $CH_3OCO$ | H | Cl | H | $SCF_3$ | H | Cl |
| $PO(OC_2H_5)_2$ | $CH_3OCO$ | H | Cl | H | $OCF_3$ | H | H |
| $PO(OC_2H_5)_2$ | $C_2H_5CO$ | H | Cl | Cl | Cl | H | Cl |
| $PO(OC_2H_5)_2$ | $CH_3CO$ | H | Cl | Cl | Cl | H | H |
| $PO(OC_2H_5)_2$ | $CH_3CO$ | H | Cl | Cl | Cl | H | H |
| $PO(OC_2H_5)_2$ | 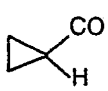 | H | Cl | Cl | Cl | H | Cl |
| $PO(OC_2H_5)_2$ | $CH_3CO$ | H | Cl | Cl | Cl | H | Cl |
| $PO(OC_2H_5)_2$ | $CH_3CO$ | H | Cl | Cl | $CF_3$ | H | Cl |
| $PO(OC_2H_5)_2$ | $C_2H_5CO$ | H | Cl | Cl | $CF_3$ | H | Cl |
| $PO(OC_2H_5)_2$ | $-C_3H_7CO$ | H | Cl | Cl | $CF_3$ | H | Cl |

Fortsetzung Tabelle 1

| $R^1$ | $R^2$ | $R^3$ | $R^4$ | $R^5$ | $R^6$ | $R^7$ | $R^8$ |
|---|---|---|---|---|---|---|---|
| $PO(OC_2H_5)_2$ | 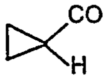 | H | Cl | Cl | $CF_3$ | H | Cl |
| $PO(OC_2H_5)_2$ | $C_2H_5CO$ | H | Cl | Cl | $CF_3$ | H | Cl |
| $PO(OC_2H_5)_2$ | $C_2H_5CO$ | H | Cl | H | $OCF_3$ | H | Cl |
| $PO(OC_2H_5)_2$ | $CH_3CO$ | Cl | Cl | H | $OCF_3$ | H | Cl |
| $PO(OC_2H_5)_2$ | 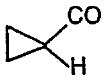 | H | Cl | H | $OCF_3$ | H | Cl |
| $PO(OC_2H_5)_2$ | $n{-}C_3H_7CO$ | H | Cl | H | $OCF_3$ | H | Cl |
| $PO(OC_2H_5)_2$ | $CH_3OCO$ | H | Cl | H | $OCF_3$ | H | Cl |
| $PO(OC_2H_5)_2$ | $CH_3OCO$ | H | Cl | H | $SO_2CF_3$ | H | Cl |
| $PO(OC_2H_5)_2$ | $CH_3CO$ | H | Cl | H | $SO_2CF_3$ | H | Cl |
| $PO(OC_2H_5)_2$ | $C_2H_5CO$ | H | Cl | H | $SO_2CF_3$ | H | Cl |
| $PO(OC_2H_5)_2$ | 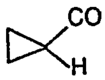 | H | Cl | H | $SO_3CF_3$ | H | Cl |
| $PO(OC_2H_5)_2$ | $n{-}C_3H_7CO$ | H | Cl | H | $SO_2CF_3$ | H | Cl |
| $PO(OC_2H_5)_2$ | $n{-}C_3H_7CO$ | H | Cl | H | $SO_2CClF_2$ | H | Cl |
| $PO(OC_2H_5)_2$ | $CH_3CO$ | H | Cl | H | $SO_2CClF_2$ | H | Cl |
| $PO(OC_2H_5)_2$ | $C_2H_5CO$ | H | Cl | H | $SO_2CClF_2$ | H | Cl |
| $PO(OC_2H_5)_2$ | $CH_3CO$ | H | Br | H | $OCF_3$ | H | Br |
| $PO(OC_2H_5)_2$ | $C_2H_5O$ | H | Br | H | $OCF_3$ | H | H |
| $PO(OC_2H_5)_2$ | $n{-}C_3H_7CO$ | H | Br | H | $OCF_3$ | H | H |
| $PO(OC_2H_5)_2$ | 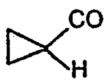 | H | Br | H | $OCF_2$ | H | H |
| $PO(OC_2H_5)_2$ | $CH_3OCO$ | H | Br | H | $OCF_3$ | H | H |
| $PO(OC_2H_5)_2$ | $CH_3CO$ | H | Br | H | $SCF_3$ | H | Br |
| $PO(OC_2H_5)_2$ | $CH_3CO$ | H | Br | H | $SCF_3$ | H | H |
| $PO(OC_2H_5)_2$ | $C_2H_5CO$ | H | Br | H | $SCF_3$ | H | H |
| $PO(OC_2H_5)_2$ | $n{-}C_3H_7CO$ | H | Br | H | $SCF_3$ | H | H |
| $PO(OC_2H_5)_2$ | $C_2H_5O$ | H | Br | H | Br | H | Br |
| $PO(OC_2H_5)_2$ | $C_2H_5CO$ | H | Cl | H | Cl | H | H |
| $PO(OC_2H_5)_2$ | $C_2H_5CO$ | H | Br | H | Br | H | H |
| Cl | $C_2H_5CO$ | H | Cl | H | $CF_3$ | H | Cl |
| Cl | $CH_3CO$ | H | Cl | H | $CF_3$ | H | Cl |
| Cl | 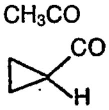 | H | Cl | H | $CF_3$ | H | Cl |
| Cl | $CH_3OCO$ | H | Cl | H | $CF_3$ | H | Cl |
| Cl | $n{-}C_3H_7CO{-}$ | H | Cl | H | $CF_3$ | H | Cl |
| Cl | $CH_3CO$ | $CH_3CO$ | Cl | H | $CF_3$ | H | Cl |
| Cl | $C_2H_5CO$ | H | Cl | H | $OCF_3$ | H | H |
| Cl | 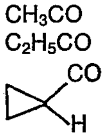 | H | Cl | H | $OCF_3$ | H | H |
| Cl | $n{-}C_4H_9CO$ | H | Cl | H | $OCF_3$ | H | H |
| Cl | $C_2H_5CO$ | H | Cl | H | $SCF_3$ | H | Cl |
| Cl | $n{-}C_3H_7CO$ | H | Cl | H | $SCF_3$ | H | Cl |
| Cl | 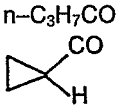 | H | Cl | H | $SCF_3$ | H | Cl |
| Cl | $CH_3CO$ | H | Cl | H | $SCF_3$ | H | Cl |
| Cl | $CH_3OCO$ | H | Cl | H | $SCF_3$ | H | Cl |
| Cl | $CH_3OCO$ | H | Cl | H | $OCF_3$ | H | H |
| Cl | $C_2H_5CO$ | H | Cl | Cl | Cl | H | Cl |
| Cl | $CH_3CO$ | H | Cl | Cl | Cl | H | Cl |
| Cl | $CH_3CO$ | H | Cl | Cl | Cl | H | H |
| Cl | 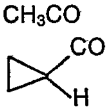 | H | Cl | Cl | Cl | H | Cl |

Fortsetzung Tabelle 1

| $R^1$ | $R^2$ | $R^3$ | $R^4$ | $R^5$ | $R^6$ | $R^7$ | $R^8$ |
|---|---|---|---|---|---|---|---|
| Cl | $CH_3OCO$ | H | Cl | Cl | Cl | H | Cl |
| Cl | $CH_3CO$ | H | Cl | Cl | $CF_3$ | H | Cl |
| Cl | $C_2H_5CO$ | H | Cl | Cl | $CF_3$ | H | Cl |
| Cl | n–$C_3H_7CO$ | H | Cl | Cl | $CF_3$ | H | Cl |
| Cl | 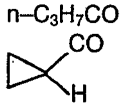 | H | Cl | Cl | $CF_3$ | H | Cl |
| Cl | $CH_3OCO$ | H | Cl | Cl | $CF_3$ | H | Cl |
| Cl | $C_2H_5CO$ | H | Cl | H | $OCF_3$ | H | Cl |
| Cl | $CH_3CO$ | H | Cl | H | $OCF_3$ | H | Cl |
| Cl | 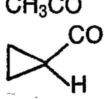 | H | Cl | H | $OCF_3$ | H | Cl |
| Cl | n–$C_3H_7CO$ | H | Cl | H | $OCF_3$ | H | Cl |
| Cl | $CH_3OCO$ | H | Cl | H | $OCF_3$ | H | Cl |
| Cl | $CH_3OCO$ | H | Cl | H | $SO_2CF_3$ | H | Cl |
| Cl | $CH_3CO$ | H | Cl | H | $SO_2CF_3$ | H | Cl |
| Cl | $C_2H_5CO$ | H | Cl | H | $SO_2CF_3$ | H | Cl |
| Cl | 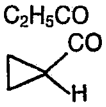 | H | Cl | H | $SO_2CF_3$ | H | Cl |
| Cl | n–$C_3H_7CO$ | H | Cl | H | $SO_2CF_3$ | H | Cl |
| Cl | n–$C_3H_7CO$ | H | Cl | H | $SO_2CClF_2$ | H | Cl |
| Cl | $CH_3CO$ | H | Cl | H | $SO_2CClF_2$ | H | Cl |
| Cl | $C_2H_5CO$ | H | Cl | H | $SO_2CClF_2$ | H | Cl |
| Cl | $CH_3CO$ | H | Br | $CF_3$ | $OCF_3$ | H | Br |
| Cl | $C_2H_5CO$ | H | Br | H | $OCF_3$ | H | H |
| Cl | n–$C_3H_7CO$ | H | Br | H | $OCF_3$ | H | H |
| Cl | 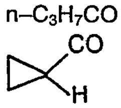 | H | Br | H | $OCF_3$ | H | H |
| Cl | $CH_3OCO$ | H | Br | H | $OCF_3$ | H | H |
| Cl | $CH_3CO$ | H | Br | H | $SCF_3$ | H | Br |
| Cl | $CH_3CO$ | H | Br | H | $SCF_3$ | H | H |
| Cl | $C_2H_5CO$ | H | Br | H | $SCF_3$ | H | H |
| Cl | n–$C_3H_7CO$ | H | Br | H | $SCF_3$ | H | H |
| Cl | $C_2H_5CO$ | H | Cl | H | H | H | Cl |
| Cl | $C_2H_5CO$ | H | Br | H | Br | H | Br |
| Cl | $C_2H_5CO$ | H | Cl | H | Cl | H | H |
| Cl | $C_2H_5CO$ | H | Br | H | Br | H | H |
| H | H–CO– | H | Cl | H | Cl | H | Cl |
| H | H–CO– | H | Cl | Cl | Cl | H | H |
| H | H–CO– | H | Cl | H | $OCF_3$ | H | Cl |
| H | H–CO– | H | Cl | H | $SCF_3$ | H | Cl |
| H | H–CO– | H | Cl | H | $OCF_3$ | H | H |
| H | H–CO– | H | Cl | H | $CF_3$ | H | Cl |
| H | H–CO– | H | Br | H | Br | H | Br |
| H | H–CO– | H | Cl | H | Br | H | Cl |
| H | H–CO– | H | Cl | H | $SO_2CF_3$ | H | H |
| H | H–CO– | H | Cl | H | $SO_2CF_3$ | H | Cl |
| $NO_2$ | H–CO– | H | Cl | H | Cl | H | Cl |
| $NO_2$ | H–CO– | H | Cl | Cl | Cl | H | H |
| $NO_2$ | H–CO– | H | Cl | H | $OCF_3$ | H | Cl |
| $NO_2$ | H–CO– | H | Cl | H | $OCF_3$ | H | H |
| $NO_2$ | H–CO– | H | Cl | H | $SCF_3$ | H | Cl |
| $NO_2$ | H–CO– | H | Cl | H | $SCF_3$ | H | H |
| $NO_2$ | H–CO– | H | Cl | H | $SO_2CF_3$ | H | H |
| $NO_2$ | H–CO– | H | Cl | H | $SO_2CF_3$ | H | Cl |
| $NO_2$ | H–CO– | H | Br | H | Br | H | Br |
| $NO_2$ | H–CO– | H | Cl | H | Br | H | Cl |
| $NO_2$ | H–CO– | H | Cl | H | Br | H | Br |
| $NO_2$ | H–CO– | H | Br | H | $OCF_3$ | H | Br |

Fortsetzung Tabelle 1

| $R^1$ | $R^2$ | $R^3$ | $R^4$ | $R^5$ | $R^6$ | $R^7$ | $R^8$ |
|---|---|---|---|---|---|---|---|
| $NO_2$ | H–CO– | H | Br | H | $OCF_3$ | H | H |
| $NO_2$ | H–CO– | H | Br | H | $SCF_3$ | H | Br |
| $NO_2$ | H–CO– | H | I | H | $OCF_3$ | H | H |
| $NO_2$ | $CH_3CO$ | H | Cl | H | Cl | H | Cl |
| $NO_2$ | $CH_3CO$ | H | Cl | Cl | Cl | H | H |
| $NO_2$ | $CH_3CO$ | H | Cl | H | $OCF_3$ | H | Cl |
| $NO_2$ | $CH_3CO$ | H | Br | H | Br | H | Br |
| $NO_2$ | $CH_3CO$ | H | Cl | H | Br | H | Cl |
| $NO_2$ | $CH_3CO$ | H | Cl | H | Br | H | Br |
| $NO_2$ | $CH_3CO$ | H | Br | H | Cl | H | Cl |
| $NO_2$ | $CH_3CO$ | H | Br | H | $OCF_3$ | H | H |
| $NO_2$ | $CH_3CO$ | H | Br | H | $OCF_3$ | H | Br |
| $NO_2$ | $CH_3CO$ | H | Br | H | $SCF_3$ | H | H |
| $NO_2$ | $CH_3CO$ | H | Br | H | $SCF_3$ | H | Br |
| $NO_2$ | $CH_3CO$ | H | Br | H | $SO_2CH_3$ | H | H |
| $NO_2$ | $CH_3CO$ | H | Br | H | $SO_2CF_3$ | H | Br |
| $NO_2$ | $CH_3CO$ | H | Br | H | $SO_2CF_2Cl$ | H | H |
| $NO_2$ | $CH_3CO$ | H | Br | H | $SO_2CF_2Cl$ | H | Br |
| $NO_2$ | $CH_3CO$ | H | Cl | H | $OCF_2Cl$ | H | H |
| $NO_2$ | $CH_3CO$ | H | Cl | H | $OCF_2CHF_2$ | H | Cl |
| $NO_2$ | $CH_3CO$ | H | Cl | H | $OCHF_2$ | H | H |
| $NO_2$ | $CH_3CO$ | H | Cl | H | $OCHF_2$ | H | Cl |
| $NO_2$ | $CH_3CO$ | H | Cl | H | $OCH_2CF_3$ | H | H |
| $NO_2$ | $CH_3CO$ | H | Cl | H | $OCH_2CF_3$ | H | Cl |
| $NO_2$ | $CH_3CO$ | H | Br | H | $OCH_2CF_3$ | H | H |
| $NO_2$ | $CH_3CO$ | H | Br | H | $OCH_2CF_3$ | H | Br |
| $NO_2$ | $CH_3CO$ | H | Br | H | $CF_3$ | H | H |
| $NO_2$ | $CH_3CO$ | H | Br | H | $CF_3$ | H | Br |
| $NO_2$ | $C_2H_5CO$ | H | Cl | H | $OCHF_2$ | H | Cl |
| $NO_2$ | $C_2H_5CO$ | H | Cl | H | $OCHF_2$ | H | H |
| $NO_2$ | $C_2H_5CO$ | H | Cl | H | $OCF_2CHF_2$ | H | H |
| $NO_2$ | $C_2H_5CO$ | H | Cl | H | $OCF_2CHF_2$ | H | Cl |
| $NO_2$ | $C_2H_5CO$ | H | Cl | H | $CHF_2$ | H | H |
| $NO_2$ | $C_2H_5CO$ | H | Cl | H | $CHF_2$ | H | Cl |
| $NO_2$ | $C_2H_5CO$ | H | Cl | H | $OCF_2CHFCl$ | H | H |
| $NO_2$ | $C_2H_5CO$ | H | Cl | H | $OCF_2CHFCl$ | H | Cl |
| $NO_2$ | $C_2H_5CO$ | H | Cl | H | Br | H | Cl |

Die erfindungsgemäss zu verwendenden substituierten 5-Amino-1-phenyl-pyrazole der Formel (I) sind teilweise bekannt (vgl.: J. Org. Chem. 36, 2972–2974 [1971]; J. Heterocycl. Chem. 7, 345–349, [1970]; C.A. 62: 13137c).

Noch nicht bekannt sind substituierte 5-Amino-1-phenylpyrazole der Formel (I′),

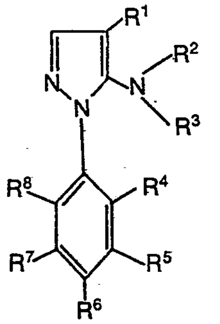

(I′)

in welcher

$R^1$, $R^2$, $R^3$, $R^4$, $R^5$, $R^6$, $R^7$ und $R^8$ die oben angegebene Bedeutung haben, wobei jedoch für den Fall, dass $R^1$ für Wasserstoff, für Methyl oder für gegebenenfalls substituiertes Phenyl steht, $R^4$ und $R^6$ nicht gleichzeitig für die Nitro-Gruppe stehen und wobei weiterhin die Verbindungen 5-Acetamido-1-(2,4,6-trinitrophenyl)-pyrazol und 1-(2-Nitrophenyl)-4-methyl-5-amino-pyrazol ausgenommen sind.

Man erhält die noch nicht bekannten substituierten 5-Amino-1-phenyl-pyrazole der Formel (I′), wenn man

(a) Phenylhydrazine der Formel (II),

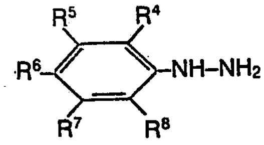

(II)

in welcher

$R^4$, $R^5$, $R^6$, $R^7$ und $R^8$ die oben angegebene Bedeutung haben,

mit Acrylnitril-Derivaten der Formel (III),

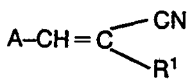
(III)

in welcher

$R^1$ die bei der Formel (I') angegebene Bedeutung hat und

A für Halogen, Hydroxy oder Alkoxy steht,

oder für den Fall, dass $R^1$ für Wasserstoff steht, auch mit 2-Halogenacrylnitrilen der Formel (IIIa),

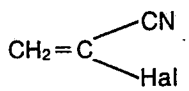
(IIIa)

in welcher

Hal für Halogen, insbesondere für Chlor oder Brom steht,

oder mit 2,3-Dihalogenpropionitrilen der Formel (IIIb),

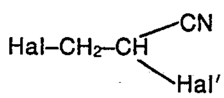
(IIIb)

in welcher

Hal und Hal' für gleiche oder unterschiedliche Halogenatome, insbesondere für Chlor oder Brom stehen,

entweder zunächst in einer 1. Stufe, gegebenenfalls in Gegenwart eines Verdünnungsmittels, sowie gegebenenfalls in Gegenwart eines Reaktionshilfsmittels umsetzt zu den Phenylhydrazin-Derivaten der Formel (IV),

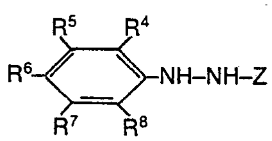
(IV)

in welcher

$R^4$, $R^5$, $R^6$, $R^7$ und $R^8$ die unter Formel (I') angegebene Bedeutung haben und

Z für einen der Reste

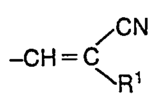
oder
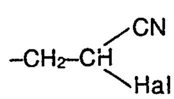

steht, wobei Hal für Halogen steht,

und diese in einer 2. Stufe gegebenenfalls in Gegenwart eines Verdünnungsmittels und gegebenenfalls in Gegenwart eines Säurebindemittels cyclisiert, oder direkt in einem Reaktionsschritt ohne Isolierung der Zwischenstufe der Formel (IV), gegebenenfalls in Gegenwart eines Verdünnungsmittels direkt cyclisiert zu den 5-Aminopyrazolen der Formel (Ia),

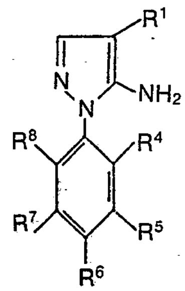
(Ia)

in welcher

$R^1$, $R^4$, $R^5$, $R^6$, $R^7$ und $R^8$ die bei der Formel (I') angegebene Bedeutung haben,

oder wenn man

(b) die nach Verfahren (a) erhältlichen 5-Aminopyrazole der Formel (Ia),

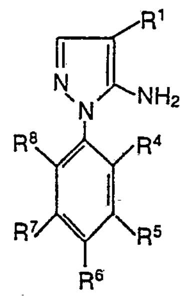
(Ia)

in welcher

$R^1$, $R^4$, $R^5$, $R^6$, $R^7$ und $R^8$ die bei der Formel (I') angegebene Bedeutung haben,

in allgemein üblicher Weise mit Acylierungsmitteln oder Alkylierungsmitteln der Formel (V),

$$R^{14}\text{–}A' \qquad (V)$$

in welcher

$R^{14}$ für geradkettiges oder verzweigtes Alkyl mit bis zu 4 Kohlenstoffatomen oder einen Rest

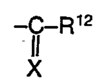
steht,

wobei X und $R^{12}$ die oben angegebene Bedeutung haben und

A' für Chlor, Brom, Iod, p-Toluolsulfonyloxy, Alkylsulfonyloxy oder Aryloxy steht,

oder mit Iso(thio)cyanaten der Formel (VI),

$$R^{15}\text{–}N{=}C{=}X \qquad (VI)$$

in welcher

$R^{15}$ für geradkettiges oder verzweigtes Alkyl mit bis zu 4 Kohlenstoffatomen oder für gegebenenfalls ein- bis dreifach, gleich oder verschieden substituiertes Phenyl steht, wobei als Substituenten in Frage kommen: Halogen, jeweils geradkettiges oder verzweigtes Alkyl, Alkoxy oder Halogenalkyl mit jeweils bis zu 4 Kohlenstoffatomen und im Fall des Halogenalkyl mit bis zu 9 gleichen oder verschiedenen Halogenatomen, und

X die oben angegebene Bedeutung hat,

gegebenenfalls in Gegenwart eines Verdünnungsmittels und gegebenenfalls in Gegenwart eines Säurebindemittels umsetzt, zu den am Stickstoff alkylierten bzw. acylierten 5-Amino-pyrazolen der Formel (Ib),

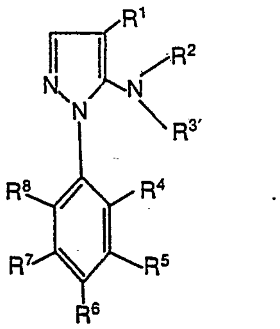

(Ib)

in welcher

$R^1$, $R^2$, $R^4$, $R^5$, $R^6$, $R^7$ und $R^8$ die bei der Formel (I') angegebene Bedeutung haben und

$R^{3'}$ für die gleichen Reste steht, wie das oben angegebene $R^3$ mit Ausnahme des Wasserstoffrestes,

oder wenn man

(c) die nach Verfahren (a) oder (b) erhältlichen in 4-Stellung unsubstituierten 5-Amino-pyrazole der Formel (Ic),

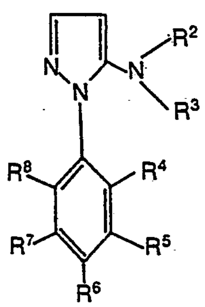

(Ic)

in welcher

$R^2$, $R^3$, $R^4$, $R^5$, $R^6$, $R^7$ und $R^8$ die bei der Formel (I') angegebene Bedeutung haben,

ebenfalls in allgemein üblicher Art und Weise mit elektrophilen Agenzien der Formel (VII),

$$R^{1'}\text{–}E \qquad (VII)$$

in welcher

$R^{1'}$ für Chlor, Brom, Nitroso, Nitro, Formyl, Hydroxysulfonyl, Chlorsulfonyl sowie für geradkettiges oder verzweigtes Alkyl oder Alkanoyl mit jeweils bis zu 6 Kohlenstoffatomen oder für gegebenenfalls einfach oder mehrfach gleich oder verschieden substituiertes Benzoyl steht, wobei als Substituenten in Frage kommen: Halogen, jeweils geradkettiges oder verzweigtes Alkyl, Alkoxy oder Halogenalkyl mit jeweils bis zu 4 Kohlenstoffatomen und im Fall des Halogenalkyl mit bis zu 9 gleichen oder verschiedenen Halogenatomen, und

E für Halogen, Hydroxy, Alkyl- oder Arylsulfonyloxy, für Alkanoyloxy oder Aroyloxy steht,

oder mit anderen üblichen elektrophilen Reagenzien gegebenenfalls in Gegenwart eines Verdünnungsmittels und gegebenenfalls in Gegenwart eines Katalysators oder Reaktionshilfsmittels in 4-Stellung substituiert, oder wenn man

(d) die nach Verfahren (c) erhältlichen 4-Chlorsulfonyl-5-amino-pyrazole der Formel (Id),

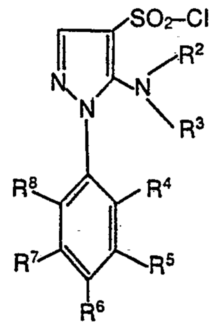

(Id)

in welcher

$R^2$, $R^3$, $R^4$, $R^5$, $R^6$, $R^7$ und $R^8$ die bei der Formel (I') angegebene Bedeutung haben,

mit Aminen der Formel (VIII),

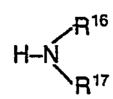

(VIII)

in welcher

$R^{16}$ und $R^{17}$ unabhängig voneinander für Wasserstoff oder für geradkettiges oder verzweigtes Alkyl mit bis zu 4 Kohlenstoffatomen stehen,

oder mit einem Alkalimetallfluorid der Formel (IX),

$$Me^{\oplus}\ F^{\ominus} \qquad (IX),$$

in welcher

$Me^{\oplus}$ für ein Alkalimetallkation steht,

gegebenenfalls in Gegenwart eines Verdünnungsmittels und gegebenenfalls in Gegenwart eines Säurebindemittels an der Chlorsulfonylgruppe in 4-Stellung substituiert, oder wenn man

(e) die nach den Verfahren (c) oder (f) erhältlichen 4-Acyl-5-amino-pyrazole der Formel (Ie)

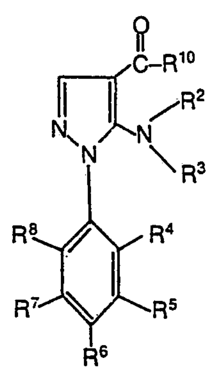

(Ie)

in welcher

$R^2$, $R^3$, $R^4$, $R^5$, $R^6$, $R^7$ und $R^8$ die bei der Formel (I') angegebene Bedeutung haben,

$R^{10}$ die oben angegebene Bedeutung hat,

mit Hydroxylamin-Derivaten der Formel (X)

$$H_2N\text{–}OR^{11} \qquad (X)$$

in welcher
$R^{11}$ die oben angegebene Bedeutung hat,
oder mit deren Hydrosalzen, gegebenenfalls in Gegenwart eines Verdünnungsmittels und gegebenenfalls in Gegenwart eines Säurebindemittels umsetzt, oder wenn man
(f) 4-Cyano-5-amino-pyrazole der Formel (XI),

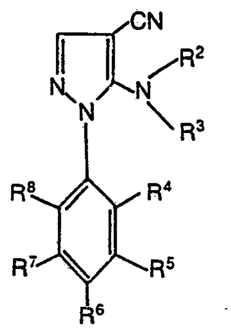

(XI)

in welcher
$R^2$, $R^3$, $R^4$, $R^5$, $R^6$, $R^7$ und $R^8$ die bei der Formel (I') angegebene Bedeutung haben,
mit Ameisensäure und Raney-Nickel umsetzt zu den entsprechenden 5-Amino-4-formyl-pyrazolen der Formel (If)

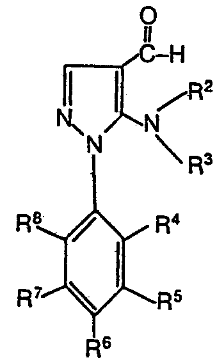

(If)

in welcher
$R^2$, $R^3$, $R^4$, $R^5$, $R^6$, $R^7$ und $R^8$ die bei der Formel (I') angegebene Bedeutung haben.

Mit den unter (e) genannten Hydrosalzen von Hydroxylamin-Derivaten sind die Hydrohalogenide gemeint, z.B. die Hydrochloride (HCl-Additionssalze).

Verwendet man als Ausgangsstoffe beispielsweise 2-Chloracrylnitril und 2,6-Dichlor-4-trifluormethylphenylhydrazin, so lässt sich der Reaktionsablauf des Herstellungsverfahrens (a) durch das folgende Formelschema darstellen:

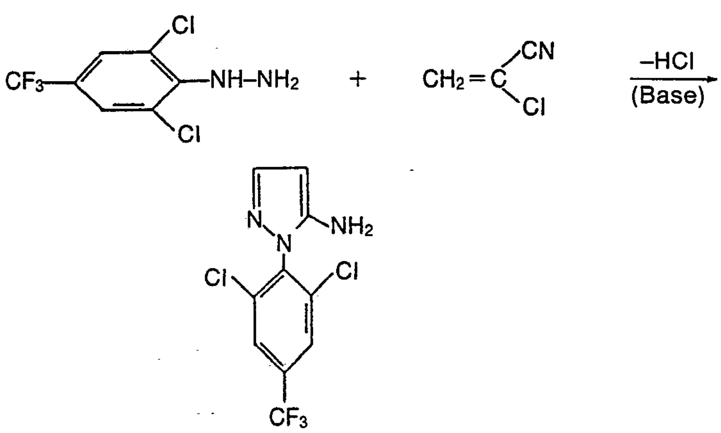


Verwendet man als Ausgangsstoffe beispielsweise 5-Amino-1-(2,4,6-trichlorphenyl)-pyrazol und Propionylchlorid, so lässt sich der Reaktionsverlauf des Herstellungsverfahrens (b) durch das folgende Formelschema wiedergeben:

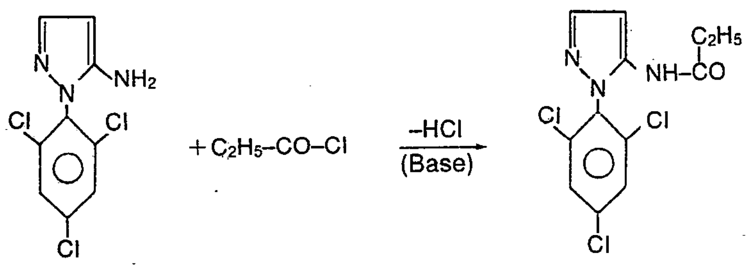


Verwendet man als Ausgangsstoffe beispielsweise 5-Propionamido-1-(2,3,4-trichlorphenyl)-pyrazol und Salpetersäure, so lässt sich der Reaktionsablauf des Herstellungsverfahrens (c) durch das folgende Formelschema darstellen:

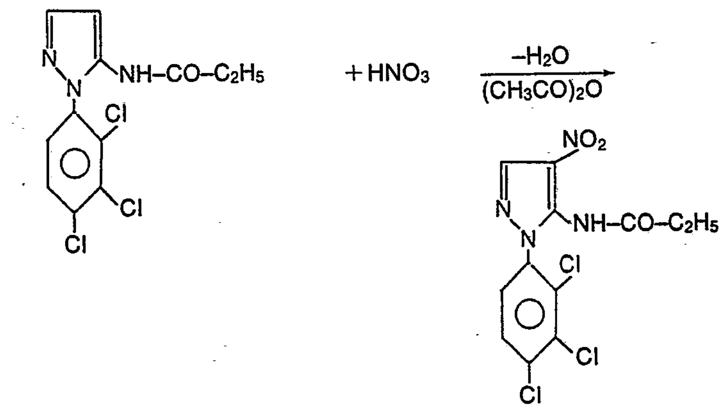


Verwendet man als Ausgangsstoffe beispielsweise
5-Acetamido-4-chlorsulfonyl-1-(2,4,6-trichlorphenyl)-pyrazol

und Diethylamin, so lässt sich der Reaktionsablauf des Herstellungsverfahrens (d) durch das folgende Formelschema darstellen:

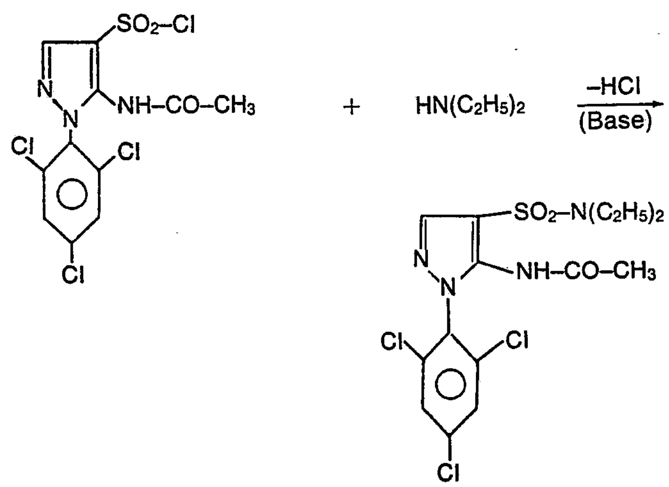


Verwendet man als Ausgangsstoffe beispielsweise
5-Acetamido-4-acetyl-1-(pentafluorphenyl)-pyrazol

und O-Methyl-hydroxylamin-Hydrochlorid, so lässt sich der Reaktionsablauf des Herstellungsverfahrens (e) durch das folgende Formelschema darstellen:

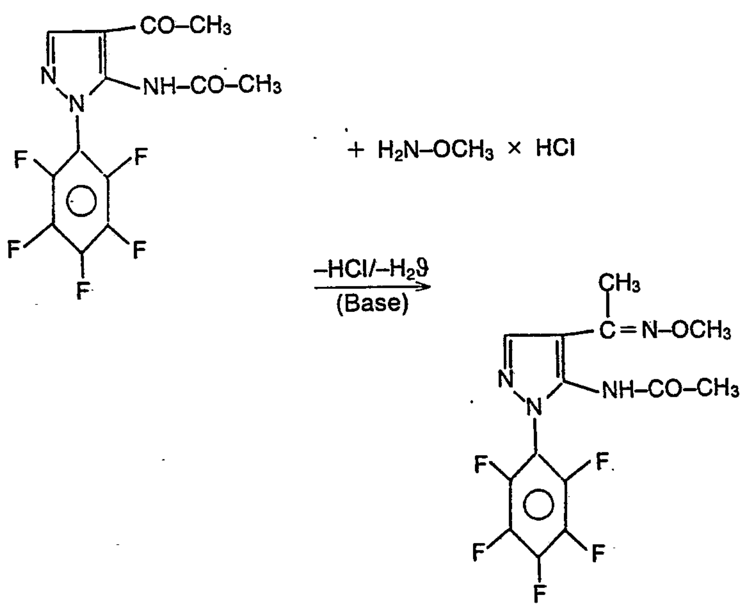

Verwendet man als Ausgangsstoffe beispielsweise
5-Amino-4-cyano-1-(2,3,4-trichlorphenyl)-pyrazol
und Ameisensäure sowie Raney-Nickel als Reduktionsmittel, so lässt sich der Reaktionsablauf des erfindungsgemässen Verfahrens (f) durch das folgende Formelschema darstellen:

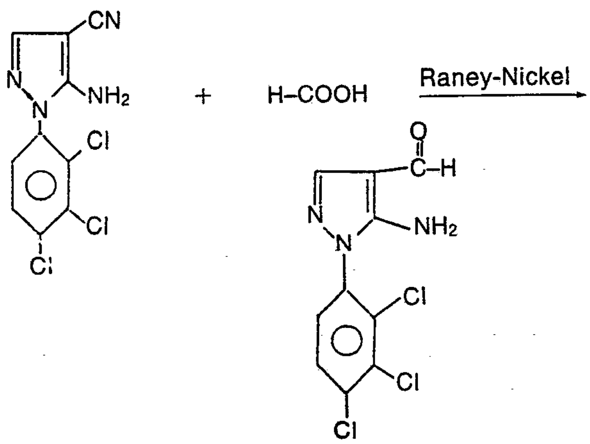


Die zur Durchführung des Herstellungsverfahrens (a) als Ausgangsstoffe benötigten Phenylhydrazine sind durch die Formel (II) allgemein definiert. In dieser Formel (II) stehen $R^4$, $R^5$, $R^6$, $R^7$ und $R^8$ vorzugsweise für diejenigen Substituenten, die schon bei der Beschreibung der erfindungsgemäss verwendbaren Stoffe der Formel (I) als bevorzugt für diese Reste genannt wurden.

Die Phenylhydrazine der Formel (II) sind grösstenteils bekannt oder können nach bekannten Verfahren in einfacher, analoger Weise hergestellt werden (vgl.: z.B. Houben-Weyl ‹Methoden der organischen Chemie› Band X,2, S. 203, Thieme Verlag Stuttgart 1967), indem man beispielsweise die bekannten Aniline der Formel (XII),

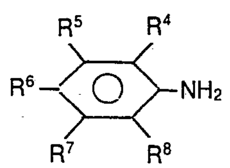

(XII)

in welcher
$R^4$, $R^5$, $R^6$, $R^7$ und $R^8$ die oben angegebene Bedeutung haben,
mit Natriumnitrit, in Gegenwart einer Säure, wie beispielsweise Schwefelsäure, und dann mit Zinn-(II)-chlorid, ebenfalls in Gegenwart einer Säure, wie beispielsweise Salzsäure, bei Temperaturen zwischen −20 °C und +80 °C umsetzt.

Die weiterhin zur Durchführung des Herstellungsverfahrens (a) als Ausgangsstoffe benötigten Acrylnitril-Derivate sind durch die Formel (III) allgemein definiert. In dieser Formel (III) steht $R^1$ vorzugsweise für diejenigen Reste, die schon bei der Beschreibung der erfindungsgemäss verwendbaren Stoffe der Formel (I) als bevorzugt für diese Substituenten angegeben wurden. A steht vorzugsweise für Chlor oder Brom, für Hydroxy, Methoxy oder Ethoxy.

Die Acrylnitril-Derivate der Formel (III) sind bekannt (vgl.: z.B. DE-A-3 129 429, EP-A-34 945; J. Chem. Soc. D 1970, 1255; Can. J. Chem. 48, 2104–2109 (1970); J. Heterocyclic Chem. 19, 1267–1273 (1982); Can. J. Chem. 51, 1239–1244 [1973]) oder können nach literaturbekannten Verfahren in einfacher analoger Weise erhalten werden. Die 2-Halogen-acrylnitrile der Formel (IIIa) und die 2,3-Dihalogen-Propionitrile der Formel (IIIb) sind ebenfalls bekannt (vgl. z.B. J. Prakt. Chemie 321, 93 [1979]; J. Heterocyclic Chem. 19, 1265 [1982]; J. Heterocyclic Chem. 19, 1267 [1982]).

Die zur Durchführung des Herstellungsverfahrens (b) als Ausgangsstoffe benötigten 5-Aminopyrazole sind durch die Formel (Ia) allgemein definiert. In dieser Formel (Ia) stehen $R^1$, $R^4$, $R^5$, $R^6$, $R^7$ und $R^8$ vorzugsweise für diejenigen Reste, die schon bei der Beschreibung der erfindungsgemäss verwendbaren Stoffe der Formel (I) als bevorzugt für diese Reste genannt wurden.

Die 5-Amino-pyrazole der Formel (Ia) sind noch nicht bekannt. Man erhält sie nach Herstellungsverfahren (a).

Die weiterhin zur Durchführung des Herstellungsverfahrens (b) als Ausgangsstoffe benötigten Alkylierungs- und Acylierungsmittel sind durch die Formel (V) allgemein definiert. Die Alkylierungs- und Acylierungsmittel der Formel (V) sind allgemein bekannte Verbindungen der organischen Chemie.

Die alternativ zur Durchführung des Herstellungsverfahrens (b) als Ausgangsstoffe verwendbaren Iso(thio)cyanate sind durch die Formel (VI) allgemein definiert.

Die Iso(thio)cyanate der Formel (VII) sind ebenfalls allgemein bekannte Verbindungen der organischen Chemie.

Die zur Durchführung des Herstellungsverfahrens (c) als Ausgangsstoffe benötigten 5-Aminopyrazole sind durch die Formel (Ic) allgemein definiert. In dieser Formel (Ic) stehen $R^2$, $R^3$, $R^4$, $R^5$, $R^6$, $R^7$ und $R^8$ vorzugsweise für diejenigen Reste, die schon bei der Beschreibung der erfindungsgemäss verwendbaren Stoffe der Formel (I) als bevorzugt für diese Substituenten genannt wurden. Die 5-Amino-pyrazole der Formel (Ic) sind noch nicht bekannt. Man erhält sie nach Herstellungsverfahren (a) oder (b).

Die weiterhin zur Durchführung des Herstellungsverfahrens (c) als Ausgangsstoffe benötigten elektrophilen Agenzien sind durch die Formel (VII) allgemein definiert. In dieser Formel (VII) kommen als Substituenten von Benzoyl in $R^{1'}$ insbesondere Fluor, Chlor, Brom, Methyl, Methoxy und Trifluormethyl in Frage.

Weiterhin verwendbare elektrophile Reagenzien sind Sulfurylchlorid, Phosphoroxychlorid/Dimethylformamid, Nitriersäure und andere üblicherweise zu elektrophilen Substitutionen verwendeten Stoffe.

Die elektrophilen Agenzien der Formel (VII) ebenso wie die weiterhin üblichen elektrophilen Reagenzien sind allgemein bekannte Verbindungen.

Die zur Durchführung des Herstellungsverfahrens (d) als Ausgangsstoffe benötigten 4-Chlorsulfonyl-5-amino-pyrazole sind durch die Formel (Id) allgemein definiert. In dieser Formel (Id) stehen $R^2$, $R^3$, $R^4$, $R^5$, $R^6$, $R^7$ und $R^8$ vorzugsweise für diejenigen Reste, die schon bei der Beschreibung der erfindungsgemäss verwendbaren Stoffe der Formel (I) als bevorzugt für diese Substituenten genannt wurden. Die 4-Chlorsulfonyl-5-amino-pyrazole sind noch nicht bekannt. Man erhält sie nach Herstellungsverfahren (c).

Die weiterhin zur Durchführung des Herstellungsverfahrens (d) als Ausgangsstoffe benötigten Amine sind durch die Formel (VIII) allgemein definiert.

Die alternativ zur Durchführung des Herstellungsverfahrens (d) als Ausgangsstoffe benötigten Alkalimetallfluoride sind durch die Formel (IX) allgemein definiert. In dieser Formel (IX) steht $M^{\oplus}$ vorzugsweise für ein Natrium- oder Kaliumkation.

Die Amine der Formel (VIII) ebenso wie die Alkalimetallfluoride der Formel (IX) sind allgemein bekannte Verbindungen.

Die zur Durchführung des Herstellungsverfahrens (e) als Ausgangsstoffe benötigten 4-Acyl-5-amino-pyrazole sind durch die Formel (Ie) allgemein definiert. In dieser Formel (Ie) stehen $R^2$, $R^3$, $R^4$, $R^5$, $R^6$, $R^7$, $R^8$ und $R^{10}$ vorzugsweise für diejenigen Reste, die schon bei der Beschreibung der erfindungsgemäss verwendbaren Stoffe der Formel (I) als bevorzugt für diese Substituenten genannt wurden. Die 4-Acyl-5-amino-pyrazole der Formel (Ie) sind noch nicht bekannt. Man erhält sie nach Herstellungsverfahren (c) oder (f).

Die weiterhin zur Durchführung des Herstellungsverfahrens (e) als Ausgangsstoffe benötigten Hydroxylamin-Derivate sind durch die Formel (X) allgemein definiert. In dieser Formel (X) steht $R^{11}$ vorzugsweise für diejenigen Reste, die schon bei der Beschreibung der erfindungsgemäss verwendbaren Stoffe der Formel (I) als bevorzugt für diesen Substituenten genannt wurde. Die Hydroxylamin-Derivate der Formel (X) und deren Hydrosalze sind allgemein bekannte Verbindungen.

Die zur Durchführung des Herstellungsverfahrens (f) als Ausgangsstoffe benötigten 4-Cyano-5-amino-pyrazole sind durch die Formel (XI) allgemein definiert. In dieser Formel (XI) stehen $R^2$, $R^3$, $R^4$, $R^5$, $R^6$, $R^7$ und $R^8$ vorzugsweise für diejenigen Reste, die schon bei der Beschreibung der erfindungsgemäss verwendbaren Stoffe der Formel (I) als bevorzugt für diese Substituenten genannt wurden. Die 4-Cyano-5-amino-pyrazole der Formel (XI) sind teilweise bekannt (vgl. z.B. EP-A-26 034; EP-A-34 945; EP-A-53 687; DE-A-3 226 496 und DE-A-3 226 513) teilweise sind sie Gegenstand einer eigenen älteren Patentanmeldung (EP-A-0 138 149). Man erhält sie beispielsweise in analoger Weise zu Herstellungsverfahren (a), wenn man Phenylhydrazine der Formel (II),

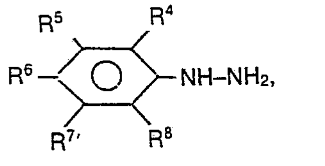
(II)

in welcher

$R^4$, $R^5$, $R^6$, $R^7$ und $R^8$ die oben angegebene Bedeutung haben,

mit den bekannten Ethoxymethylenmalodinitrilen der Formel (XII),

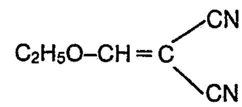
(XII)

gegebenenfalls in Gegenwart eines Verdünnungsmittels wie beispielsweise Ethanol und gegebenenfalls in Gegenwart eines Säurebindemittels wie beispielsweise Natriumacetat bei Temperaturen zwischen −20 °C und +150 °C umsetzt.

Als Verdünnungsmittel zur Durchführung des Herstellungsverfahrens (a) kommen sowohl für die 1. als auch für die 2. Reaktionsstufe inerte organische Lösungsmittel in Frage. Vorzugsweise verwendet man Alkohole, wie Methanol, Ethanol, Propanol, Butanol, Ethylenglykol oder Ethylenglykolmonomethyl- oder -ethylether.

Als Reaktionshilfsmittel zur Durchführung der 1. Stufe des Herstellungsverfahrens (a) kommen organische oder anorganische Säuren in Frage. Vorzugsweise verwendet man Schwefelsäure oder Essigsäure, gegebenenfalls auch in Gegenwart einer Puffersubstanz, wie beispielsweise Natriumacetat.

Die Reaktionstemperaturen können bei der Durchführung der 1. Stufe des Herstellungsverfahrens (a) in gewissen Bereichen variiert werden. Im allgemeinen arbeitet man zwischen −30 °C und +50 °C, vorzugsweise zwischen −20 °C und +20 °C.

Als Säurebindemittel zur Durchführung der 2. Stufe des Herstellungsverfahrens (a) kommen alle üblicherweise verwendbaren anorganischen und organischen Basen in Frage. Vorzugsweise verwendet man Alkalimetallcarbonate oder Hydrogencarbonate, wie Natriumcarbonat, Kaliumcarbonat oder Kaliumhydrogencarbonat.

Die Reaktionstemperaturen können bei der Durchführung der 2. Stufe des Herstellungsverfahrens (a) ebenso wie bei der einstufigen Reaktionsführung in einem grösseren Bereich variiert werden. Im allgemeinen arbeitet man zwischen 0 °C und 200 °C, vorzugsweise zwischen +50 °C und +150 °C.

Zur Durchführung des Herstellungsverfahrens (a) setzt man sowohl bei der einstufigen als auch bei der zweistufigen Reaktionsführung pro Mol Phenylhydrazin der Formel (II) im allgemeinen 1,0 bis 3,0 Mol, vorzugsweise 1,0 bis 1,5 Mol an Acrylnitril-Derivat der Formel (III) bzw. (IIIa) oder an 2,3-Dichlorpropionitril der Formel (IIIb) und im Fall des zweistufigen Verfahrens gegebenenfalls in der 1. Stufe 1,0 bis 10,0 Mol an Reaktionshilfsmittel und gegebenenfalls in der 2. Stufe 1,0 bis 10,0 Mol an Säurebindemittel ein.

Die Aufarbeitung und Isolierung der Reaktionsprodukte erfolgt nach üblichen Verfahren, beispielsweise durch Entfernen des organischen Verdünnungsmittels, Ausfällen des Reaktionsproduktes in Wasser, Absaugen und Trocknen des so erhaltenen Produktes.

Zur Durchführung des Herstellungsverfahrens (b) kommen als Verdünnungsmittel ebenfalls inerte organische Lösungsmittel in Frage. Vorzugsweise verwendet man aliphatische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Nitrile, wie Acetonitril oder Propionitril, Amide, wie Dimethylformamid, Dimethylacetamid, N-Methylpyrrolidon oder Hexamethylphosphorsäuretriamid. Verwendet man Acylierungs- oder Alkylierungsmittel der Formel (V) oder (VI) in flüssiger Form, so ist es auch möglich, diese in entsprechendem Überschuss als Verdünnungsmittel einzusetzen.

Als Säurebindemittel zur Durchführung des Herstellungsverfahrens (b) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des Herstellungsverfahrens (b) in einem grösseren Bereich variiert werden. Im allgemeinen arbeitet man zwischen −20 °C und +150 °C, vorzugsweise zwischen 0 °C und +100 °C.

Zur Durchführung des Herstellungsverfahrens (b) setzt man pro Mol 5-Amino-pyrazol der Formel (Ia) im allgemeinen 1,0 bis 20,0 Mol, vorzugsweise 1,0 bis 15,0 Mol an Acylierungs- oder Alkylierungsmittel der Formel (V) oder (VI) und gegebenenfalls 1,0 bis 3,0 Mol, vorzugsweise 1,0 bis 2,0 Mol an Säurebindemittel ein. Die Reaktionsdurchführung, Aufarbeitung und Isolierung der Reaktionsprodukte der Formel (Ib) erfolgt in allgemein üblicher Art und Weise.

Als Verdünnungsmittel zur Durchführung des Herstellungsverfahrens (c) kommen alle üblicherweise für derartige elektrophile Substitutionen verwendbaren Lösungsmittel in Frage. Vorzugsweise verwendet man die als Reagenzien in Frage kommenden Säuren oder Gemische, wie beispielsweise Schwefelsäure, Chlorsulfonsäure, Salpetersäure, Nitriersäure, Sulfurylchlorid, Phosphoroxychlorid/Dimethylformamid oder Nitriersäure gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff als Verdünnungsmittel in Frage.

Als Katalysatoren oder Reaktionshilfsmittel zur Durchführung des Herstellungsverfahrens (c) kommen ebenfalls die für derartige Reaktionen üblichen Katalysatoren in Frage; vorzugsweise verwendet man saure Katalysatoren wie beispielsweise Schwefelsäure, Eisen-III-chlorid oder andere Lewis-Säuren oder Acetanhydrid.

Die Reaktionstemperaturen können bei der Durchführung des Herstellungsverfahrens (c) in einem grösseren Bereich variiert werden. Im allgemeinen arbeitet man zwischen −50 °C und +200 °C, vorzugsweise zwischen −20 °C und +150 °C.

Zur Durchführung des Herstellungsverfahrens (c) setzt man pro Mol 5-Amino-pyrazol der Formel (Ic) im allgemeinen 1,0 bis 10,0 Mol, vorzugsweise 1,0 bis 5,0 Mol an elektrophilem Agens der Formel (VII) und gegebenenfalls 0,1 bis 10 Mol an Katalysator oder Reaktionshilfsmittel ein. Die Reaktionsführung, Aufarbeitung und Isolierung der Reaktionsprodukte der Formel (I) erfolgt in allgemein üblicher Art und Weise.

Als Verdünnungsmittel zur Durchführung des Herstellungsverfahrens (d) kommen ebenfalls inerte organische Lösungsmittel in Frage. Vorzugsweise verwendet man die bei dem Herstellungsverfahren (b) genannten Lösungsmittel. Für die Umsetzung mit einem Alkalimetallfluorid der Formel (IX) kommt gegebenenfalls auch Wasser oder wässrige Gemische mit einem der bei Verfahren (b) genannten Lösungsmittel in Frage.

Als Säurebindemittel zur Durchführung des Herstellungsverfahrens (d) kommen ebenfalls alle üblicherweise verwendbaren organischen oder anorganischen Basen in Frage. Vorzugsweise verwendet man Alkalimetallhydroxide, -carbonate oder -hydrogencarbonate, wie beispielsweise Kaliumcarbonat oder Natriumhydrogencarbonat. Es kommen auch tertiäre organische Basen, wie Triethylamin oder Pyridin in Frage.

Die Reaktionstemperaturen können bei dem Herstellungsverfahren (d) ebenfalls in einem grösseren Bereich variiert werden. Im allgemeinen arbeitet man zwischen −20 °C und +120 °C, vorzugsweise zwischen 0 °C und +90 °C.

Zur Durchführung des Herstellungsverfahrens (d) setzt man pro Mol 4-Chlorsulfonyl-5-amino-pyrazol der Formel (Id) im allgemeinen 1,0 bis 3,0 Mol, vorzugsweise 1,0 bis 2,0 Mol an Amin der Formel (VIII) oder Alkalimetallfluorid der Formel (IX) und gegebenenfalls 1,0 bis 3,0 Mol, vorzugsweise 1,0 bis 2,0 Mol an Säurebindemittel ein. Die Reaktionsführung, Aufarbeitung und Isolierung der Reaktionsprodukte der Formel (I) erfolgt in üblicher Art und Weise.

Als Verdünnungsmittel zur Durchführung des Herstellungsverfahrens (e) kommen ebenfalls inerte organische Lösungsmittel in Frage. Vorzugsweise verwendet man Alkohole, wie beispielsweise Methanol, Ethanol oder Propanol.

Als Säurebindemittel zur Durchführung des Herstellungsverfahrens (e) kommen ebenfalls alle

üblicherweise verwendbaren organischen und anorganischen Basen in Frage. Vorzugsweise verwendet man die bei Verfahren (b) genannten Säurebindemittel.

Die Reaktionstemperaturen können bei der Durchführung des Herstellungsverfahrens (e) ebenfalls in einem grösseren Bereich variiert werden. Im allgemeinen arbeitet man zwischen +20 °C und +150 °C, vorzugsweise zwischen +20 °C und +120 °C.

Zur Durchführung des Herstellungsverfahrens (e) setzt man pro Mol 4-Acyl-5-amino-pyrazol der Formel (Ie) im allgemeinen 1,0 bis 3,0 Mol, vorzugsweise 1,0 bis 2,0 Mol an Hydroxylamin-Derivat der Formel (X) und gegebenenfalls 1,0 bis 3,0 Mol an Säurebindemittel ein. Die Reaktionsführung, Aufarbeitung und Isolierung der Reaktionsprodukte der Formel (I) erfolgt in allgemein üblicher Art und Weise.

Als Verdünnungsmittel zur Durchführung des Herstellungsverfahrens (f) kommen inerte organische Lösungsmittel oder auch wässrige Systeme in Frage. Vorzugsweise verwendet man die als reaktionspartner verwendete Ameisensäure in entsprechendem Überschuss, gegebenenfalls in Mischung mit Wasser als Verdünnungsmittel.

Die Reaktionstemperaturen können bei der Durchführung des Herstellungsverfahrens (f) ebenfalls in einem grösseren Bereich variiert werden. Im allgemeinen arbeitet man zwischen 0 °C und +150 °C, vorzugsweise zwischen +20 °C und 130 °C.

Zur Durchführung des Herstellungsverfahrens (f) setzt man pro Mol 4-Cyano-5-amino-pyrazol der Formel (XI) im allgemeinen 0,1 bis 3 Mol, vorzugsweise 0,5 bis 2 Mol an Raney-Nickel und im allgemeinen 10 bis 30 Mol, vorzugsweise 1,0 bis 15 Mol an Ameisensäure ein.

Die Reaktionsführung, Aufarbeitung und Isolierung der Reaktionsprodukte der Formel (I) erfolgt in üblicher Weise in Analogie zu bekannten Verfahren (vgl. z.B. Chem. Pharm. Bull. 24, 3120 [1976]).

Die erfindungsgemäss verwendbaren Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäss verwendbaren Stoffe als totale oder selektive Herbizide wirken, hängt im wesentlichen von der angewendeten Menge ab.

Die erfindungsgemäss verwendbaren Wirkstoffe können z.B. bei den folgenden Pflanzen verwendet werden.

Dikotyle Unkräuter der Gattungen: Sinapsis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monoktyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäss verwendbaren Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäss verwendbaren Wirkstoffe der Formel (I) neben einer besonders guten allgemeinherbiziden Wirksamkeit auch eine deutlich verbesserte Kulturpflanzenselektivität in wichtigen Kulturen und können als selektive Unkrautbekämpfungsmittel sowohl in dikotylen Kulturen, wie beispielsweise Baumwollpflanzungen, Sojabohnen oder Erdnüssen, als auch in monokotylen Kulturen, insbesondere Getreide, wie beispielsweise Weizen, eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäss verwendbaren Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B.
1-Amino-6-ethylthio-3-(2,2-dimethylpropyl)-1,3,5-triazin-2,4(1H,3H)-dion
oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und
4-Amino-6-(1,1-dimethylethyl)-3-methylthio-1,2,4-triazin-5(4H)-on
zur Unkrautbekämpfung in Sojabohnen, in Frage. Auch Mischungen mit
N,N-Dimethyl-N'-(3-trifluormethylphenyl)-harnstoff,
N,N-Dimethyl-N'-(3-chlor-4-methylphenyl)-harnstoff,
N,N-Dimethyl-N'-(4-isopropylphenyl)-harnstoff,
4-Amino-6-t-butyl-3-ethylthio-1,2,4-triazin-5(4H)-on,
2,4-Dichlorphenoxyessigsäure,
2,4-Dichlorphenoxypropionsäure,
(2-Methyl-4-chlorphenoxy)-essigsäure,
(4-Chlor-2-methyl-phenoxy)-propionsäure,
Chloressigsäure-N-(methoxymethyl)-2,6-diethylanilid,
2-Ethyl-6-methyl-N-(1-methyl-2-methoxyethyl)-chloracetanilid,
2,6-Dinitro-4-trifluormethyl-N,N-dipropylanilin,
2-[4-(3,5-Dichlorpyrid-2-yloxy)-phenoxy]-propionsäure-(2-benzyloxyethylester),
-(trimethylsilylmethylester) oder -(2,2-diethoxyethylester)
sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfrass, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Giessen, Spritzen, Sprühen, Streuen.

Die erfindungsgemäss verwendbaren Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem grösseren Bereich schwanken. Es hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,01 und 5 kg pro ha.

Die Herstellung und die Verwendung der erfindungsgemäss verwendbaren Wirkstoffe geht aus den nachfolgenden Beispielen hervor.

Herstellungsbeispiele
Beispiel 1

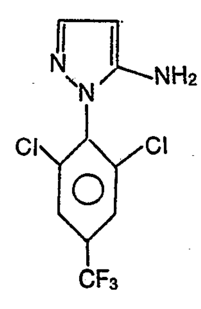

(Verfahren a)

24,5 g (0,1 Mol) 2,6-Dichlor-4-trifluormethylphenylhydrazin werden mit 20 mg Ethylendiamintetraessigsäure-Dinatriumsalz (= Titriplex II) in 150 ml Methanol bei Rückflusstemperatur tropfenweise und mit 25 ml (27,6 g/0,3 Mol) 2-Chloracrylnitril versetzt. Nach beendeter Zugabe erhitzt man weitere 8 Stunden auf Rückflusstemperatur, tropft dann 9 ml (0,16 Mol) 96%-ige Schwefelsäure zu und erhitzt weitere 6 Stunden auf Rückflusstemperatur. Die abgekühlte Reaktionsmischung wird mit 33,5 gt (0,3 Mol) wasserfreiem Natriumcarbonat versetzt. Nach 4 Stunden entfernt man das Lösungsmittel im Vakuum, nimmt den Rückstand in 500 ml Wasser auf und rührt 10 Stunden bei Raumtemperatur. Man filtriert den ausgefallenen Niederschlag ab, wäscht mit Wasser nach und trocknet im Vakuum bei 50 °C.

Man erhält 28,5 g (96% der Theorie)
5-Amino-1-(2,6-dichlor-4-trifluormethylphenyl)-pyrazol
vom Schmelzpunkt 103–105 °C.

Beispiel 2:

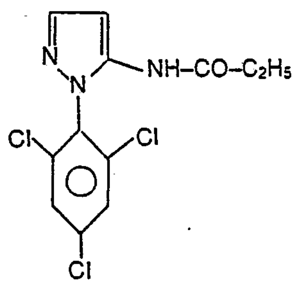

(Verfahren b)

13,2 g (0,05 Mol) 5-Amino-1-(2,4,6-trichlorphenyl)-pyrazol in 100 ml Dichlormethan werden bei Raumtemperatur unter Rühren nacheinander mit 5 ml (5,3 g/0,05 Mol) 98%-igem Propionylchlorid und dann mit 5 ml (5,0 g/0,063 Mol) wasserfreiem Pyridin versetzt. Dabei steigt die Temperatur auf 40 °C. Nach beendeter Zugabe rührt man weitere 16 Stunden bei Raumtemperatur, gibt 50 ml Dichlormethan zu, wäscht je zweimal mit 100 ml Wasser, 100 ml gesättigter Natriumhydrogencarbonatlösung und 100 ml Natriumchloridlösung, trocknet über Magnesiumsulfat und entfernt das Lösungsmittel im Vakuum. Der feste Rückstand wird mit wenig Hexan gewaschen und getrocknet.

Man erhält 12,5 g (81% der Theorie) an 5-Propionamido-1-(2,4,6-trichlorphenyl)-pyrazol vom Schmelzpunkt 125 °C.

Beispiel 3:

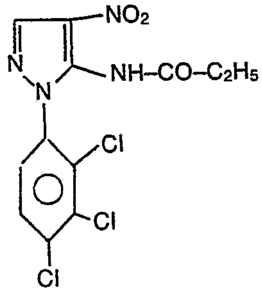

(Verfahren c)

6,4 g (0,02 Mol) 5-Propionamido-1-(2,3,4-trichlorphenyl)-pyrazol in 20 ml Eisessig werden bei 10 °C nacheinander zuerst mit 2 ml (2,17 g/0,021 Mol) Acetanhydrid und dann mit 0,9 ml (1,3 g/0,02 Mol) 98%-iger Salpetersäure versetzt. Nach beendeter Zugabe rührt man 16 Stunden bei 25 °C. Zur Aufarbeitung engt man im Vakuum ein, nimmt den Rückstand in 20 ml Diethylether auf, wäscht dreimal mit insgesamt 50 bis 100 ml konzentrierter Natriumhydrogencarbonatlösung und zweimal mit 50 ml gesättigter Natriumchloridlösung, entfernt das Lösungsmittel im Wasserstrahlvakuum, wäscht den festen Rückstand mit wenig Wasser und trocknet im Hochvakuum bei 30 °C bis 40 °C. Man erhält 5,5 g (76% der Theorie) an
4-Nitro-5-propionamido-1-(2,3,4-trichlorphenyl)-pyrazol
vom Schmelzpunkt 79–81 °C.

Beispiel 4:

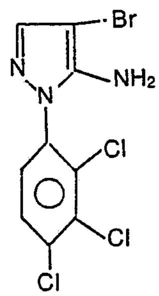

(Verfahren c)

2,6 g (0,01 Mol) 5-Amino-1-(2,3,4-trichlorphenyl)-pyrazol in 10 ml Eisessig werden bei 20 °C tropfenweise unter Rühren mit einer Lösung von 1,6 g (0,02 Mol) Brom in 5 ml Eisessig versetzt. Nach beendeter Zugabe rührt man weitere 3 Stunden bei 20 °C, fügt dann 30 ml Wasser und 3 g (0,022 Mol) Natriumacetat-Trihydrat zu, rührt eine weitere Stunde, saugt den kristallinen Niederschlag ab, wäscht mit Wasser und trocknet bei 50 °C bis 60 °C im Vakuum. Man erhält 3,2 g (94% der Theorie) an
5-Amino-4-brom-1-(2,3,4-trichlorphenyl)-pyrazol
vom Schmelzpunkt 129 °C.

Beispiel 5:

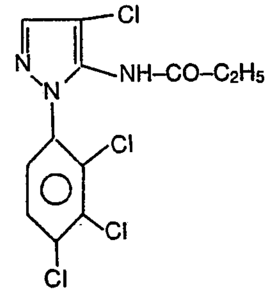

(Verfahren c)

2,7 g (0,0085 Mol) 5-Propionamido-1-(2,3,4-trichlorphenyl)-pyrazol in 20 ml Dichlormethan werden bei 0 °C bis 5 °C tropfenweise mit 1,2 g (0,009 Mol) Sulfurylchlorid versetzt. Nach beendeter Zugabe rührt man weitere 16 Stunden bei Raumtemperatur, verdünnt mit 30 ml Dichlormethan, wäscht mehrfach mit Wasser, gesättigter Natriumbicarbonatlösung und gesättigter Natriumchloridlösung, trocknet über Natriumsulfat, engt im Vakuum ein und trocknet bei 50 °C im Hochvakuum. Man erhält 2,5 g (83% der Theorie) an

4-Chlor-5-propionamido-1-(2,3,4-trichlorphenyl)-pyrazol

vom Schmelzpunkt 122 °C.

Beispiel 6:

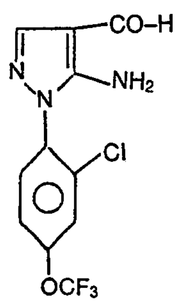

(Verfahren f)

12 g (0,042 Mol) 5-Amino-4-cyano-1-(2-chlor-4-trifluormethoxy-phenyl)-pyrazol werden mit 5 g Raney-Nickel in 50 ml 75%-iger wässriger Ameisensäure 1 Stunde unter Rückfluss gekocht; die noch warme Lösung wird abgesaugt, der Rückstand mit Wasser nachgewaschen, das Filtrat mehrfach mit Ether extrahiert, die vereinigten Etherphasen mit gesättigter Natriumhydrogencarbonatlösung gewaschen, über Natriumsulfat getrocknet und im Vakuum eingeengt. Man erhält 8,0 g (62% der Theorie) an

5-Amino-1-(2-chlor-4-trifluormethoxy-phenyl)-4-formyl-pyrazol

als Öl.

$^1$H-NMR δ[ppm] = 5.7 (s, 2H) – Singulett, 7.3–7.6 (m, 3H) – Multiplett, 7,85 (s, 1H) – Singulett, 9,6 (s, 1H) – Singulett.

Beispiel 7:

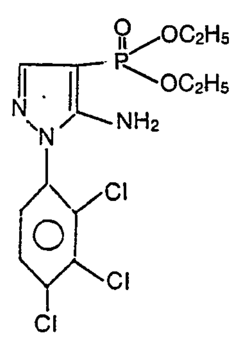

(Verfahren a)

6,3 g (0,03 Mol) 2,3,4-Trichlorphenylhydrazin, 5,8 g (0,025 Mol) Ethoxymethylen-diethylphosphonoacetonitril und 1 g (0,012 Mol) Natriumacetat, wasserfrei werden in 10 ml Eisessig suspendiert und 48 Stunden bei Raumtemperatur gerührt. Die Reaktionsmischung wird mit 50 ml Dichlormethan und 100 ml Wasser versetzt. Die organische Phase wird abgetrennt und je einmal mit 50 ml gesättigter Natriumhydrogencarbonat-Lösung und 30 ml gesättigter Kochsalzlösung gewaschen. Nach dem Trocknen der Lösung über Magnesiumsulfat wird das Lösungsmittel im Vakuum abdestilliert, der ölige Rückstand in 15 ml Ethoxyethanol gelöst und 5 Stunden unter Rückfluss erhitzt. Danach kühlt man auf Raumtemperatur ab, versetzt die Lösung mit 30 ml Wasser und rührt so lange bis der anfangs ölige Niederschlag kristallisiert. Die schwach gelb gefärbten Kristalle werden abgesaugt, mit Wasser gewaschen und im Vakuum bei 50–60 °C getrocknet.

Man erhält 5,6 g (46,8% der Theorie)

5-Amino-4-diethylphosphono-1-(2,3,4-trichlorphenyl)-pyrazol

vom Schmelzpunkt 114 °C.

Herstellung der Ausgangsverbindung:

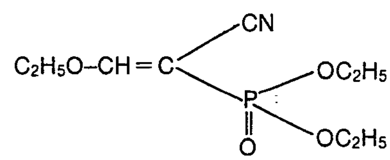

35,4 g (0,2 Mol) Diethylphosphonoacetonitril (vgl. Houben-Weyl ‹Methoden der organischen Chemie› Band E2, S. 345, 4. Auflage, Thieme Verlag Stuttgart, 1982), 42 ml (0,45 Mol) Essigsäureanhydrid und 56 ml (0,34 Mol) o-Ameisensäuretriethylester werden 2 Stunden bei 110 °C gerührt. Danach destilliert man über eine kurze Kolonne bei Normaldruck die leichtsiedenden Verbindungen ab, wobei die Sumpftemperatur bis auf 140 °C ansteigt. Man hält noch 4 Stunden bei dieser Temperatur und destilliert dann die unumgesetzten Ausgangsverbindungen zuerst im Wasserstrahl- und dann im Ölpumpenvakuum ab. Im Sumpf verbleiben 20,5 g (44% der Theorie) Ethoxymethylen-diethylphosphonoacetonitril als Öl.

$^1$H-NMR ($CDCl_3$) = δ = 7.63 ppm (d, 1H) – Dublett, 4.32 ppm (q, 2H) – Quartett, 4.15 ppm (m, 4H) – Multiplett, 1.45–1.35 ppm (m, 9H) – Multiplett.

In entsprechender Weise und gemäss den allgemeinen Herstellungsangaben erhält man die folgenden Verbindungen der allgemeinen Formel (I)

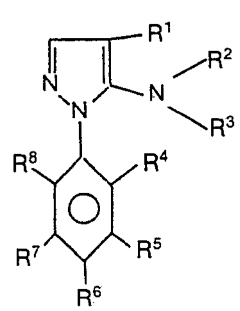

(I)

Tabelle 2

| Beisp. Nr. | $R^1$ | $R^2$ | $R^3$ | $R^4$ | $R^5$ | $R^6$ | $R^7$ | $R^8$ | physikal. Eigenschaften |
|---|---|---|---|---|---|---|---|---|---|
| 8 | $NO_2$ | $C_2H_5CO$ | H | Cl | H | Cl | H | Cl | Fp:54 °C |
| 9 | $NO_2$ | $C_2H_5CO$ | H | Cl | H | $CF_3$ | H | Cl | Fp:48 °C |
| 10 | $NO_2$ | $C_2H_5CO$ | H | Cl | H | $CF_3$ | H | H | Fp:115 °C (Zers.) |
| 11 | $NO_2$ | $C_2H_5CO$ | H | Cl | H | $SCF_3$ | H | Cl | Fp:96–99 °C |
| 12 | $NO_2$ | $C_2H_5CO$ | H | Cl | H | $OCF_3$ | H | H | Fp:65 °C |
| 13 | $NO_2$ | $C_2H_5CO$ | H | Cl | Cl | $CF_3$ | Cl | Cl | Fp:54–60 °C |
| 14 | $NO_2$ | $C_2H_5CO$ | H | $CF_3$ | H | Cl | H | H | Öl |
| 15 | $NO_2$ | H | H | Cl | H | Cl | H | Cl | Fp:218 °C |
| 16 | $NO_2$ | $CH_3CO$ | H | Cl | H | $CF_3$ | H | Cl | Fp:50 °C |
| 17 | $NO_2$ | $CH_3CO$ | H | Cl | H | $SCF_3$ | H | Cl | Fp:58–61 °C |
| 18 | $NO_2$ | $CH_3(CH_2)_2CO$ | H | Cl | H | $CF_3$ | H | Cl | Fp:44 °C |
| 19 | $NO_2$ | $CH_3OCO$ | H | Cl | H | $CF_3$ | H | Cl | Fp:141–43 °C |
| 20 | $NO_2$ | $ClCH_2CO$ | H | Cl | H | $CF_3$ | H | Cl | Fp:130–33 °C |
| 21 | $NO_2$ | $C_2H_5CO$ | H | Cl | H | Cl | H | H | Fp:63 °C |
| 22 | H | H | H | Cl | H | Cl | H | Cl | Fp:113 °C |
| 23 | H | H | H | Cl | H | $CF_3$ | H | H | Fp:88–92 °C |
| 24 | H | H | H | Cl | H | $SCF_3$ | H | Cl | Fp:79 °C |
| 25 | H | H | H | Cl | Cl | Cl | H | H | Fp:152 °C |
| 26 | H | H | H | Cl | Cl | $CF_3$ | Cl | Cl | Fp:103–10 °C |
| 27 | H | H | H | Cl | H | $OCF_3$ | H | H | Fp:73–76 °C |
| 28 | H | $C_2H_5CO$ | H | Cl | Cl | Cl | H | H | Fp:55 °C |
| 29 | H | $C_2H_5CO$ | H | Cl | H | $CF_3$ | H | H | Fp:106–10 °C |
| 30 | H | $C_2H_5CO$ | H | Cl | H | $SCF_3$ | H | Cl | Fp:136 °C |
| 31 | H | $C_2H_5CO$ | H | Cl | H | $CF_3$ | H | Cl | Fp:146 °C |
| 32 | H | $C_2H_5CO$ | H | Cl | H | $OCF_3$ | H | H | Öl |
| 33 | H | $C_2H_5CO$ | H | Cl | Cl | $CF_3$ | Cl | Cl | Fp:154–58 °C |
| 34 | H | $C_2H_5CO$ | H | $CF_3$ | H | Cl | H | H | Öl |
| 35 | H | $C_2H_5CO$ | H | Cl | H | Cl | H | H | Öl |
| 36 | H | $C_2H_5CO$ | H | Cl | H | $SO_2CF_2Cl$ | H | Cl | Fp:134 °C |
| 37 | H | $CH_3CO$ | $C_2H_5CO$ | Cl | Cl | Cl | H | H | Fp:142 °C |
| 38 | H | $CH_3CO$ | $CH_3CO$ | Cl | Cl | Cl | H | H | Fp:142 °C |
| 39 | H | $CH_3OCO$ | H | Cl | H | $CF_3$ | H | Cl | Fp:123–126 °C |
| 40 | H | $CH_3CO$ | H | Cl | H | $CF_3$ | H | Cl | Fp:173–76 °C |
| 41 | H | $CH_3CO$ | H | Cl | H | $SCF_3$ | H | Cl | Fp:131 °C |
| 42 | H | $CH_3(CH_2)_2CO$ | H | Cl | H | $CF_3$ | H | Cl | Fp:128–31 °C |
| 43 | H | $ClCH_2CO$ | H | Cl | H | $CF_3$ | H | Cl | Fp:149–50 °C |
| 44 | Br | $C_2H_5CO$ | H | Cl | Cl | Cl | H | H | Fp:78 °C |
| 45 | I | H | H | Cl | Cl | Cl | H | H | Fp:162 °C |
| 46 | $CH_3O$<br>$N=CH-$ | H | H | Cl | Cl | Cl | H | H | Fp:156–59 °C |
| 47 | $HON=CH-$ | H | H | Cl | H | $OCF_3$ | H | H | Fp:180 °C |
| 48 | $(C_2H_5O)_2P-$<br>‖<br>O | $C_2H_5CO$ | H | Cl | Cl | Cl | H | H | Öl |
| 49 | $NO_2$ | $C_2H_5CO$ | H | Cl | H | $SO_2CCl_2F$ | H | Cl | Fp:156–58 °C |
| 50 | $NO_2$ | H | H | Cl | H | $CF_3$ | H | Cl | Fp:189–90 °C |
| 51 | $NO_2$ | H | H | Cl | H | $SO_2CCl_2F$ | H | Cl | Fp:208–09 °C |

Fortsetzung Tabelle 2

| Beisp. Nr. | $R^1$ | $R^2$ | $R^3$ | $R^4$ | $R^5$ | $R^6$ | $R^7$ | $R^8$ | Schmelzpunkt (°C) |
|---|---|---|---|---|---|---|---|---|---|
| 52 | H | $C_2H_5CO$ | $CH_3$ | Cl | H | Cl | H | Cl | 150–51 |
| 53 | $NO_2$ | $C_2H_5CO$ | $CH_3$ | Cl | H | Cl | H | Cl | 192–98 |
| 54 | H | $C_2H_5CO$ | H | Cl | H | $SCF_3$ | H | H | Oil |

Fortsetzung Tabelle 2

| Beisp. Nr. | $R^1$ | $R^2$ | $R^3$ | $R^4$ | $R^5$ | $R^6$ | $R^7$ | $R^8$ | Schmelzpunkt (°C) |
|---|---|---|---|---|---|---|---|---|---|
| 55 | H | 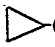CO | H | Cl | H | $CF_3$ | H | Cl | 69–71 |
| 56 | H | $C_{11}H_{23}CO$ | H | Cl | H | $CF_3$ | H | Cl | 63–66 |
| 57 | $SCCl_2F$ | H | H | Cl | H | Cl | H | Cl | 102 |
| 58 | $SCCl_2F$ | $C_2H_5CO$ | H | Cl | H | Cl | H | Cl | 128–29 |
| 59 | $NO_2$ | H | H | Cl | H | $OCF_3$ | H | H | 50 |
| 60 | $NO_2$ | 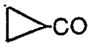CO | H | Cl | H | $CF_3$ | H | Cl | 165–68 |
| 61 | $NO_2$ | $C_{11}H_{23}CO$ | H | Cl | H | $CF_3$ | H | Cl | 60–70 |
| 62 | $NO_2$ | $C_2H_5CO$ | H | Cl | H | $SCF_3$ | H | H | 90–93 |
| 63 | Cl | $C_2H_5CO$ | H | Cl | H | $CF_3$ | H | Cl | 63–65 |
| 64 | H | $C_2H_5CO$ | H | Cl | H | $OCF_3$ | H | Cl | 50–56 |
| 65 | $SOCCl_2F$ | $C_2H_5CO$ | H | Cl | H | Cl | H | Cl | 143–46 |
| 66 | $NO_2$ | $C_2H_5CO$ | H | Cl | H | $SO_2CF_3$ | H | H | 60–64 |
| 67 | $SO_2CCl_2F$ | $C_2H_5CO$ | H | Cl | H | Cl | H | Cl | 136–39 |
| 68 | H | $C_2H_5CO$ | H | Cl | Cl | $CF_3$ | H | Cl | 129–32 |
| 69 | NO | H | H | Cl | H | $CF_3$ | H | Cl | 227 |
| 70 | $NO_2$ | H | H | Cl | H | $SO_2CF_3$ | H | H | 68–70 |
| 71 | $NO_2$ | $C_2H_5CO$ | H | Cl | H | $OCF_3$ | H | Cl | 130–35 |
| 72 | $NO_2$ | $C_2H_5CO$ | H | Cl | H | $SO_2CF_3$ | H | Cl | 132–34 |
| 73 | $NO_2$ | $C_2H_5CO$ | H | Cl | Cl | $CF_3$ | H | Cl | 50–52 |
| 74 | $(C_2H_5O)_2P(O)$ | H | H | Cl | H | $CF_3$ | H | Cl | Oil |
| 75 | $NO_2$ | H | H | Cl | H | $SCF_3$ | H | H | 56–60 |
| 76 | $NO_2$ | H | H | Cl | H | $SCF_3$ | H | Cl | 145–49 |
| 77 | $NO_2$ | H | H | Cl | H | $OCF_3$ | H | Cl | 206–08 |
| 78 | $NO_2$ | H | H | Cl | H | $SO_2CF_3$ | H | Cl | 249–53 |
| 79 | $NO_2$ | H | H | Cl | Cl | $CF_3$ | H | Cl | 78–85 |
| 80 | H | $CH_3NHCO$ | H | Cl | H | $CF_3$ | H | Cl | 178–82 |
| 81 | H | 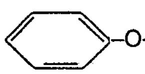–O–CO | H | Cl | H | $CF_3$ | H | Cl | 65–67 |
| 82 | H | $(CH_3)_2NCO$ | H | Cl | H | $CF_3$ | H | Cl | 137–38 |
| 83 | H | $C_2H_5CO$ | H | Cl | Cl | Cl | H | Cl | 62–67 |
| 84 | $NO_2$ | $C_2H_5CO$ | H | Cl | Cl | Cl | H | Cl | 60–67 |
| 85 | H | $ClCH_2CH_2–CO$ | H | Cl | H | $CF_3$ | H | Cl | 120–24 |
| 86 | $NO_2$ | $CH_3NHCO$ | H | Cl | H | $CF_3$ | H | Cl | 212–14 |
| 87 | $NO_2$ | 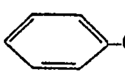–O–CO | H | Cl | H | $CF_3$ | H | Cl | 48–52 |
| 88 | $NO_2$ | H | H | Cl | Cl | Cl | H | Cl | 83–89 |
| 89 | $NO_2$ | H | $CH_3$ | Cl | H | Cl | H | Cl | 167–75 |
| 90 | H | H | $CH_3$ | Cl | H | Cl | H | Cl | 186–88 |
| 91 | H | $ClCH_2–CO$ | H | Cl | H | $CF_3$ | H | H | 103–05 |
| 92 | H | $ClCH_2–CO$ | H | Cl | H | $OCF_3$ | H | H | 84–87 |
| 93 | $NO_2$ | H | H | Cl | H | $CF_3$ | H | H | 55–58 |
| 94 | $NO_2$ | $ClCH_2CH_2–CO$ | H | Cl | H | $CF_3$ | H | Cl | 135–40 |
| 95 | $NO_2$ | $ClCH_2–CO$ | H | Cl | H | $OCF_3$ | H | H | Oil |
| 96 | $NO_2$ | $ClCH_2–CO$ | H | Cl | H | $CF_3$ | H | H | 111–13 |
| 97 | H | $C_2H_5CO$ | H | Cl | F | $CF_3$ | F | Cl | 121–23 |
| 98 | $NO_2$ | $C_2H_5CO$ | H | Cl | F | $CF_3$ | F | Cl | 135–37 |
| 99 | H | $C_2H_5CO$ | H | Cl | F | Cl | F | Cl | 190–93 |
| 100 | $NO_2$ | H | $CH_3$ | Cl | H | $CF_3$ | H | H | 120–23 |
| 101 | $NO_2$ | $C_2H_5CO$ | $CH_3$ | Cl | H | $CF_3$ | H | Cl | 140–54 |
| 102 | $NO_2$ | H | H | Cl | F | $CF_3$ | F | Cl | 158–65 |
| 103 | $NO_2$ | $C_2H_5CO$ | H | Cl | F | Cl | F | Cl | 130–33 |
| 104 | $NO_2$ | $CH_2{=}CH–CO$ | H | Cl | H | $CF_3$ | H | Cl | 55–65 |
| 105 | $NO_2$ | H | H | Cl | F | Cl | F | Cl | 210–20 |
| 106 | H | HCO | H | Cl | H | $CF_3$ | H | Cl | 144–50 |
| 107 | $NO_2$ | $C_2H_5CO$ | H | Cl | H | $OCH_3$ | H | Cl | 165–74 |
| 108 | $NO_2$ | HCO | H | Cl | H | $CF_3$ | H | Cl | 144–50 |
| 109 | $NO_2$ | H | $CH_3$ | Cl | H | $CF_3$ | H | Cl | 120–40 |

Fortsetzung Tabelle 2

| Beisp. Nr. | $R^1$ | $R^2$ | $R^3$ | $R^4$ | $R^5$ | $R^6$ | $R^7$ | $R^8$ | Schmelzpunkt (°C) |
|---|---|---|---|---|---|---|---|---|---|
| 110 | $SCCl_2F$ | H | H | Cl | H | $CF_3$ | H | Cl | 99–105 |
| 111 | $SOCCl_2F$ | H | H | Cl | H | $CF_3$ | H | Cl | 55–62 |
| 112 | H | $CH_3CO$ | H | Cl | Cl | $CF_3$ | H | Cl | 158–62 |
| 113 | $SO_2CCl_2F$ | H | H | Cl | H | $CF_3$ | H | Cl | 135–38 |
| 114 | $NO_2$ | $CH_3CO$ | H | Cl | Cl | $CF_3$ | H | Cl | 124–33 |
| 115 | H | $CH_3CO$ | H | Cl | H | $CF_3$ | H | H | 50–52 |
| 116 | $NO_2$ | $CH_3CO$ | H | Cl | H | $CF_3$ | H | H | 62–65 |
| 117 | H | H | $CH_3$ | Cl | H | $CF_3$ | H | Cl | 135–37 |
| 118 | H | 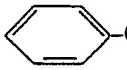-CO | H | Cl | H | $CF_3$ | H | Cl | 62–64 |
| 119 | $NO_2$ | $ClCH_2CH_2CH_2CO$ | H | Cl | H | $CF_3$ | H | Cl | 60–63 |
| 120 | $NO_2$ | 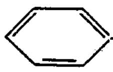-CO | H | Cl | H | $CF_3$ | H | Cl | 157–65 |
| 121 | H | $C_2H_5CO$ | H | Cl | H | Br | H | H | 86–88 |
| 122 | H | H | H | Br | H | Br | H | Br | 141–43 |
| 123 | H | $C_2H_5CO$ | H | Br | H | Br | H | Br | 147–50 |
| 124 | H | H | H | Cl | H | Br | H | Cl | 102 |
| 125 | H | H | H | Br | H | Cl | H | Br | 98 |
| 126 | $NO_2$ | H | H | Cl | H | Br | H | H | 95–98 |
| 127 | H | H | H | J | H | J | H | H | 138–40 |
| 128 | $NO_2$ | $C_2H_5CO$ | H | Br | H | Br | H | Br | 92–94 |
| 129 | H | $C_2H_5CO$ | H | Br | H | Cl | H | Br | 138–40 |
| 130 | H | $C_2H_5CO$ | H | Cl | H | Br | H | Cl | 142–48 |
| 131 | H | H | H | Br | H | Cl | H | Cl | 105–07 |
| 132 | $NO_2$ | $C_2H_5CO$ | H | Br | H | Cl | H | Br | 146–48 |
| 133 | H | $ClCH_2CO$ | H | Br | H | Cl | H | Br | 127–30 |
| 134 | H | H | H | Br | H | F | H | H | 58–60 |
| 135 | $NO_2$ | H | H | Br | H | Cl | H | Br | 230 |
| 136 | H | H | H | Br | Cl | Cl | H | Br | 120–25 |
| 137 | $NO_2$ | $C_2H_5CO$ | H | Cl | H | Br | H | Cl | 94 |
| 138 | $NO_2$ | H | H | Cl | H | Br | H | Cl | 198 |
| 139 | H | $C_2H_5CO$ | H | Br | H | Br | H | Cl | 114–18 |
| 140 | $NO_2$ | $ClCH_2CO$ | H | Br | H | Cl | H | Br | 115–20 |
| 141 | H | $C_2H_5CO$ | H | J | H | J | H | H | Oil |
| 142 | H | H | H | Br | Cl | Br | H | Cl | 170 |
| 143 | $NO_2$ | $C_2H_5CO$ | H | Br | H | Br | H | Cl | 89–93 |
| 144 | $NO_2$ | H | H | Br | H | Br | H | Cl | 208–10 |
| 145 | H | $C_2H_5CO$ | H | Br | Cl | Cl | H | Br | 165 |
| 146 | H | $C_2H_5CO$ | H | Br | Cl | Br | H | Cl | 140–50 |
| 147 | $NO_2$ | $C_2H_5CO$ | H | J | H | J | H | H | 55–60 |
| 148 | $NO_2$ | $C_2H_5CO$ | H | Br | Cl | Cl | H | Br | 130–40 |
| 149 | $NO_2$ | $C_2H_5CO$ | H | Br | H | F | H | H | 72–74 |
| 150 | $NO_2$ | $C_2H_5CO$ | H | Br | H | $OCF_3$ | H | H | 83–85 |
| 151 | Br | H | H | Cl | H | Cl | H | Cl | 119 |
| 152 | $SCH_3$ | H | H | Cl | H | Cl | H | Cl | 114 |
| 153 | Br | H | H | $OCH_3$ | H | Cl | H | Cl | 67–70 |
| 154 | $SCH_3$ | $C_2H_5CO$ | H | Cl | H | Cl | H | Cl | 92–95 |
| 155 | $SO_2CH_3$ | $C_2H_5CO$ | H | Cl | H | Cl | H | Cl | 146–50 |
| 156 | –CHO | H | H | Cl | H | $OCF_3$ | H | H | Oil |
| 157 | –CH | $C_2H_5CO$ | H | Cl | H | $OCF_3$ | H | H | Oil |
| 158 | $-CH=NOCH_3$ | H | H | Cl | H | $OCF_3$ | H | H | 138–39 |
| 159 | H | $ClCH_2CH_2CH_2CO$ | H | Cl | H | $CF_3$ | H | Cl | Oil |

Anwendungsbeispiele:

In den folgenden Anwendungsbeispielen wird die nachstehend aufgeführte Verbindung als Vergleichssubstanz herangezogen:

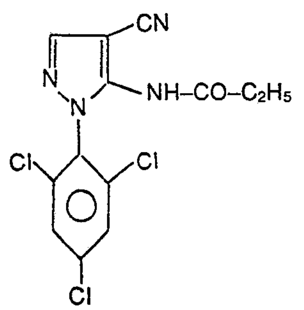

4-Cyano-5-propionamido-1-(2,4,6-trichlorphenyl)-pyrazol
(bekannt aus DE-A-3 226 513)

Beispiel A
Pre-emergence-Test
Lösungsmittel: 5 Gewichtsteile Aceton
Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Bei diesem Test zeigt beispielsweise die Verbindung gemäss Herstellungsbeispiel 3 eine deutliche Überlegenheit sowohl in der herbiziden Wirksamkeit als auch in der Kulturpflanzenselektivität gegenüber dem Stand der Technik.

Beispiel B
Post-emergence-Test
Lösungsmittel: 5 Gewichtsteile Aceton
Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5–15 cm haben so, dass die jeweils gewünschten Wirkstoffmengen pro Flächeneinheit ausgebracht werden. Die Konzentration der Spritzbrühe wird so gewählt, dass in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0% = keine Wirkung (wie unbehandelte Kontrolle)
100% = totale Vernichtung

Bei diesem Test zeigt beispielsweise die Verbindung gemäss Herstellungsbeispiel 3 eine deutliche Überlegenheit sowohl in der herbiziden Wirksamkeit als auch in der Kulturpflanzenselektivität gegenüber dem Stand der Technik.

**Patentansprüche**

1. Herbizide Mittel, gekennzeichnet durch einen Gehalt an mindestens einem 5-Amino-1-phenylpyrazol der allgemeinen Formel (I')

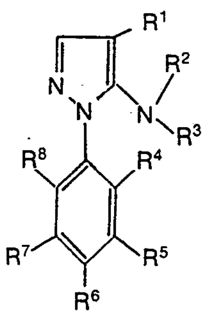

(I')

in welcher

$R^1$ für Wasserstoff, Nitroso, Nitro, Halogen, jeweils geradkettiges oder verzweigtes Alkyl oder Halogenalkyl mit jeweils bis zu 6 Kohlenstoffatomen und im Fall des Halogenalkyl bis zu 9 gleichen oder verschiedenen Halogenatomen, ferner für gegebenenfalls einfach oder mehrfach, gleich oder verschieden durch Halogen, Nitro, Alkyl, Alkoxy oder Halogenalkyl mit jeweils bis zu 4 Kohlenstoffatomen substituiertes Phenyl oder für einen der Reste

$$-S(O)_n-R^9;\ -\overset{O}{\overset{\|}{C}}-R^{10};\ -\overset{O}{\overset{\|}{P}}(OR^{11})_2 \text{ oder } -\overset{R^{10}}{\overset{|}{C}}=N-OR^{11}$$

steht,

$R^2$ für Wasserstoff oder einen Rest

$$-\overset{X}{\overset{\|}{C}}-R^{12} \text{ steht,}$$

$R^3$ unabhängig von $R^2$ für die gleichen Reste wie $R^2$ steht und zusätzlich für geradkettiges oder verzweigtes Alkyl mit bis zu 4 Kohlenstoffatomen steht,

$R^4$ und $R^6$ unabhängig voneinander für Cyano, Nitro, Halogen oder für jeweils geradkettiges oder verzweigtes Alkyl, Alkoxy oder Alkoxycarbonyl mit jeweils bis zu 4 Kohlenstoffatomen ausserdem für jeweils geradkettiges oder verzweigtes Halogenalkyl oder Halogenalkoxy mit jeweils bis zu 4 Kohlenstoffatomen und bis zu 9 gleichen oder

verschiedenen Halogenatomen oder für einen Rest $-S(O)_n-R^{13}$ stehen, und

$R^5$, $R^7$ und $R^8$ unabhängig voneinander und von $R^4$ und $R^6$ für die gleichen Reste wie $R^4$ und $R^6$ stehen und zusätzlich für Wasserstoff stehen, wobei

$R^9$ für Wasserstoff, Hydroxy, Fluor, Chlor, Brom, Amino, für jeweils geradkettiges oder verzweigtes Alkylamino, Dialkylamino, Alkyl oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen sowie für gegebenenfalls einfach oder mehrfach, gleich oder verschieden substituiertes Phenyl steht, wobei als Substituenten in Frage kommen: Halogen, jeweils geradkettiges oder verzweigtes Alkyl, Alkoxy oder Halogenalkyl mit jeweils bis zu 4 Kohlenstoffatomen und im Fall des Halogenalkyl mit bis zu 9 gleichen oder verschiedenen Halogenatomen,

$R^{10}$ für Wasserstoff, für geradkettiges oder verzweigtes Alkyl mit bis zu 4 Kohlenstoffatomen oder für gegebenenfalls einfach oder mehrfach, gleich oder verschieden substituiertes Phenyl steht, wobei als Phenylsubstituenten die bei $R^9$ genannten in Frage kommen,

$R^{11}$ für Wasserstoff, für Halogenalkyl mit bis zu 4 Kohlenstoffatomen und bis zu 6 gleichen oder verschiedenen Halogenatomen, für jeweils geradkettiges oder verzweigtes Alkyl, Alkenyl oder Alkinyl mit jeweils bis zu 8 Kohlenstoffatomen, sowie für geradkettiges oder verzweigtes Phenylalkyl mit bis zu 4 Kohlenstoffatomen im Alkylteil steht,

$R^{12}$ für Wasserstoff, für jeweils geradkettiges oder verzweigtes Alkyl mit 1–12 C-Atomen, sowie für Alkenyl, Alkinyl, Alkoxyalkyl, Alkylthioalkyl, Alkoxy, Alkylthio, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen, ausserdem für gegebenenfalls einfach oder mehrfach, gleich oder verschieden durch Halogen, niederes Alkyl oder niederes Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten die bei $R^9$ genannten in Frage kommen;

$R^{13}$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht,

X für Sauerstoff oder Schwefel steht und

n für eine Zahl 0, 1 oder 2 steht,

wobei jedoch für den Fall, dass $R^1$ für Wasserstoff, für Methyl oder für gegebenenfalls substituiertes Phenyl steht, $R^4$ und $R^6$ nicht gleichzeitig für die Nitro-Gruppe stehen und wobei weiterhin die Verbindungen 5-Acetamido-1-(2,4,6-trinitrophenyl)-pyrazol und 1-(2-Nitrophenyl)-4-methyl-5-amino-pyrazol ausgenommen sind.

2. Verfahren zur Herstellung von herbiziden Mitteln, dadurch gekennzeichnet, dass man 5-Amino-1-phenyl-pyrazole der allgemeinen Formel (I') gemäss Anspruch 1 mit Streckmitteln und/oder oberflächenaktiven Mitteln vermischt.

3. Verwendung von 5-Amino-1-phenyl-pyrazolen der allgemeinen Formel (I)

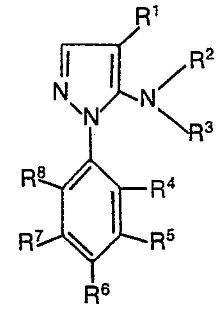

(I)

in welcher

$R^1$ für Wasserstoff, Nitroso, Nitro, Halogen, jeweils geradkettiges oder verzweigtes Alkyl oder Halogenalkyl mit jeweils bis zu 6 Kohlenstoffatomen und im Fall des Halogenalkyl bis zu 9 gleichen oder verschiedenen Halogenatomen, ferner für gegebenenfalls einfach oder mehrfach, gleich oder verschieden durch Halogen, Nitro, Alkyl, Alkoxy oder Halogenalkyl mit jeweils bis zu 4 Kohlenstoffatomen substituiertes Phenyl oder für einen der Reste $-S(O)_n-R^9$;

$$-S(O)_n-R^9;\ -\overset{\overset{O}{\|}}{C}-R^{10};\ -\overset{\overset{O}{\|}}{P}(OR^{11})_2 \text{ oder } -\overset{\overset{R^{10}}{|}}{C}=N-OR^{11}$$

steht,

$R^2$ für Wasserstoff oder einen Rest

$$-\overset{\overset{X}{\|}}{C}-R^{12} \quad \text{steht,}$$

$R^3$ unabhängig von $R^2$ für die gleichen Reste wie $R^2$ steht und zusätzlich für geradkettiges oder verzweigtes Alkyl mit bis zu 4 Kohlenstoffatomen steht,

$R^4$ und $R^6$ unabhängig voneinander für Cyano, Nitro, Halogen oder für jeweils geradkettiges oder verzweigtes Alkyl, Alkoxy oder Alkoxycarbonyl mit jeweils bis zu 4 Kohlenstoffatomen, ausserdem für jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder für einen Rest $-S(O)_n-R^{13}$ stehen, und

$R^5$, $R^7$ und $R^8$ unabhängig voneinander und von $R^4$ und $R^6$ für die gleichen Reste wie $R^4$ und $R^6$ stehen und zusätzlich für Wasserstoff stehen, wobei

$R^9$ für Wasserstoff, Hydroxy, Fluor, Chlor, Brom, Amino, für jeweils geradkettiges oder verzweigtes Alkylamino, Dialkylamino, Alkyl oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen sowie für gegebenenfalls einfach oder mehrfach, gleich oder verschieden substituiertes

Phenyl steht, wobei als Substituenten in Frage kommen: Halogen, jeweils geradkettiges oder verzweigtes Alkyl, Alkoxy oder Halogenalkyl mit jeweils bis zu 4 Kohlenstoffatomen und im Fall des Halogenalkyl mit bis zu 9 gleichen oder verschiedenen Halogenatomen,

$R^{10}$ für Wasserstoff, für geradkettiges oder verzweigtes Alkyl mit bis zu 4 Kohlenstoffatomen oder für gegebenenfalls einfach oder mehrfach, gleich oder verschieden substituiertes Phenyl steht, wobei als Phenylsubstituenten die bei $R^9$ genannten in Frage kommen,

$R^{11}$ für Wasserstoff, für Halogenalkyl mit bis zu 4 Kohlenstoffatomen und bis zu 6 gleichen oder verschiedenen Halogenatomen, für jeweils geradkettiges oder verzweigtes Alkyl, Alkenyl oder Alkinyl mit jeweils bis zu 8 Kohlenstoffatomen, sowie für geradkettiges oder verzweigtes Phenylalkyl mit bis zu 4 Kohlenstoffatomen im Alkylteil steht,

$R^{12}$ für Wasserstoff, für jeweils geradkettiges oder verzweigtes Alkyl mit 1–12 C-Atomen, sowie für Alkenyl, Alkinyl, Alkoxyalkyl, Alkylthioalkyl, Alkoxy, Alkylthio, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen, ausserdem für gegebenenfalls einfach oder mehrfach, gleich oder verschieden durch Halogen, niederes Alkyl oder niederes Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten die bei $R^9$ genannten in Frage kommen;

$R^{13}$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht,

X für Sauerstoff oder Schwefel steht und

n für eine Zahl 0, 1 oder 2 steht,

zur Bekämpfung von Unkraut.

4. Verfahren zur Bekämpfung von Unkraut, dadurch gekennzeichnet, dass man 5-Amino-1-phenyl-pyrazole der allgemeinen Formel (I) gemäss Anspruch 3 auf Unkraut oder seinen Lebensraum einwirken lässt.

5. 5-Amino-1-phenyl-pyrazole der Formel (I′)

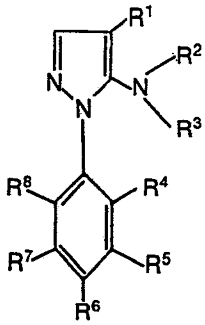

(I′)

in welcher

$R^1$, $R^2$, $R^3$, $R^4$, $R^5$, $R^6$, $R^7$ und $R^8$ die in Anspruch 1 angegebene Bedeutung haben,

wobei jedoch für den Fall, dass $R^1$ für Wasserstoff, für Methyl oder für gegebenenfalls substituiertes Phenyl steht, $R^4$ und $R^6$ nicht gleichzeitig für die Nitro-Gruppe stehen und wobei weiterhin die Verbindungen 5-Acetamido-1-(2,4,6-trinitrophenyl)-pyrazol und 1-(2-Nitrophenyl)-4-methyl-5-amino-pyrazol ausgenommen sind.

6. Phenylhydrazin-Derivate der Formel

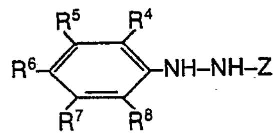

(IV)

in welcher

$R^4$, $R^5$, $R^6$, $R^7$ und $R^8$ die in Anspruch 1 angegebene Bedeutung haben und

Z für einen der Reste

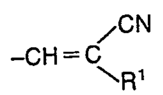

oder

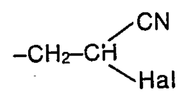

steht, wobei

Hal für Halogen steht und

$R^1$ die in Anspruch 1 angegebene Bedeutung hat,

wobei jedoch für den Fall, dass $R^1$ für Wasserstoff, für Methyl oder für gegebenenfalls substituiertes Phenyl steht, $R^4$ und $R^6$ nicht gleichzeitig für die Nitro-Gruppe stehen.

7. Verfahren zur Herstellung von 5-Amino-1-phenyl-pyrazolen der Formel (I′),

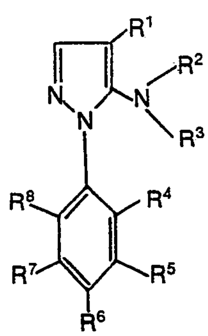

(I′)

in welcher

$R^1$, $R^2$, $R^3$, $R^4$, $R^5$, $R^6$, $R^7$ und $R^8$ die in Anspruch 1 angegebene Bedeutung haben,

wobei jedoch für den Fall, dass $R^1$ für Wasserstoff, für Methyl oder für gegebenenfalls substituiertes Phenyl steht, $R^4$ und $R^6$ nicht gleichzeitig für die Nitro-Gruppe stehen und wobei weiterhin die Verbindungen 5-Acetamido-1-(2,4,6-trinitrophenyl)-pyrazol und 1-(2,Nitrophenyl)-4-methyl-5-amino-pyrazol ausgenommen sind, dadurch gekennzeichnet, dass man

(a) Phenylhydrazine der Formel (II),

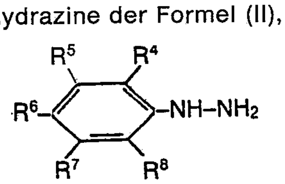

(II)

in welcher
$R^4$, $R^5$, $R^6$, $R^7$ und $R^8$ die bei der Formel (I′) angegebene Bedeutung haben,
mit Acrylnitril-Derivaten der Formel (III)

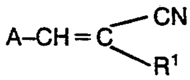
(III)

in welcher
$R^1$ die bei der Formel (I′) angegebene Bedeutung hat und
A für Halogen, Hydroxy oder Alkoxy steht,
oder für den Fall, dass $R^1$ für Wasserstoff steht, auch mit 2-Halogenacrylnitrilen der Formel (IIIa),

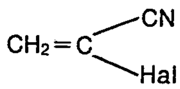
(IIIa)

in welcher
Hal für Halogen steht,
oder mit 2,3-Dihalogenpropionitrilen der Formel (IIIb),

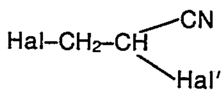
(IIIb)

in welcher
Hal und Hal′ für gleiche oder unterschiedliche Halogenatome stehen,
entweder zunächst in einer 1. Stufe, gegebenenfalls in Gegenwart eines Verdünnungsmittels, sowie gegebenenfalls in Gegenwart eines Reaktionshilfsmittels umsetzt zu den Phenylhydrazin-Derivaten der Formel (IV),

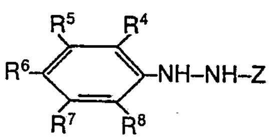
(IV)

in welcher
$R^4$, $R^5$, $R^6$, $R^7$ und $R^8$ die bei der Formel (I′) angegebene Bedeutung haben und
Z für einen der Reste

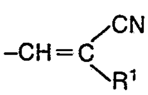 oder 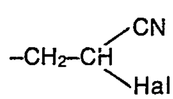 steht,

wobei Hal für Halogen steht und $R^1$ die bei der Formel (I′) angegebene Bedeutung hat,
und diese in einer 2. Stufe, gegebenenfalls in Gegenwart eines Verdünnungsmittels und gegebenenfalls in Gegenwart eines Säurebindemittels cyclisiert, oder direkt in einem Reaktionsschritt ohne Isolierung der Zwischenstufe der Formel (IV), gegebenenfalls in Gegenwart eines Verdünnungsmittels cyclisiert zu den 5-Amino-pyrazolen der Formel (Ia),

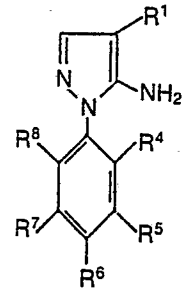
(Ia)

in welcher
$R^1$, $R^4$, $R^5$, $R^6$, $R^7$ und $R^8$ die bei der Formel (I′) angegebene Bedeutung haben,
oder dass man
(b) die nach Verfahren (a) erhältlichen 5-Amino-pyrazole der Formel (Ia),

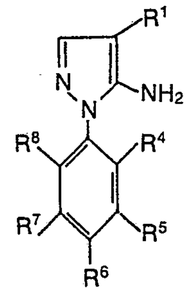
(Ia)

in welcher
$R^1$, $R^4$, $R^5$, $R^6$, $R^7$ und $R^8$ die bei der Formel (I′) angegebene Bedeutung haben,
mit Acylierungsmitteln oder Alkylierungsmitteln der Formel (V),

$$R^{14}\text{–}A' \qquad (V)$$

in welcher
$R^{14}$ für geradkettiges oder verzweigtes Alkyl mit bis zu 4 Kohlenstoffatomen oder einen Rest

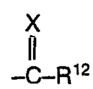 steht,

wobei X und $R^{12}$ die in Anspruch 1 angegebene Bedeutung haben und
A′ für Chlor, Brom, Iod, p-Toluolsulfonyloxy, Alkylsulfonyloxy oder Acyloxy steht,
oder mit Iso(thio)cyanaten der Formel (VI),

$$R^{15}\text{–}N=C=X \qquad (VI)$$

in welcher
$R^{15}$ geradkettiges oder verzweigtes Alkyl mit bis zu 4 Kohlenstoffatomen oder für gegebenenfalls ein- bis dreifach, gleich oder verschieden substituiertes Phenyl steht, wobei als Substituenten in Frage kommen: Halogen, jeweils geradkettiges oder verzweigtes Alkyl, Alkoxy oder Halogenalkyl mit jeweils bis zu 4 Kohlenstoffatomen und im Fall des Halogenalkyl mit bis zu 9 gleichen oder verschiedenen Halogenatomen, und
X die oben angegebene Bedeutung hat,
gegebenenfalls in Gegenwart eines Verdünnungsmittels und gegebenenfalls in Gegenwart eines

Säurebindemittels umsetzt, zu den am Stickstoff alkylierten bzw. acylierten 5-Amino-pyrazolen der Formel (Ib),

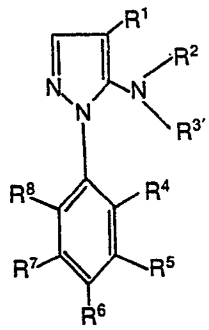

(Ib)

in welcher

$R^1$, $R^2$, $R^4$, $R^5$, $R^6$, $R^7$ und $R^8$ die bei der Formel (I′) angegebene Bedeutung haben und

$R^{3'}$ für die gleichen Reste steht, wie das oben angegebene $R^3$ mit Ausnahme des Wasserstoffrestes,

oder dass man

(c) die nach Verfahren (a) oder (b) erhältlichen in 4-Stellung unsubstituierten 5-Amino-pyrazole der Formel (Ic),

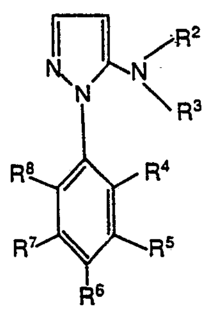

(Ic)

in welcher

$R^2$, $R^3$, $R^4$, $R^5$, $R^6$, $R^7$ und $R^8$ die bei der Formel (I′) angegebene Bedeutung haben,

mit elektrophilen Agenzien der Formel (VII),

$$R^{1'}\text{–}E \qquad \text{(VII)}$$

in welcher

$R^{1'}$ für Chlor, Brom, Nitroso, Nitro, Formyl, Hydroxysulfonyl, Chlorsulfonyl sowie für geradkettiges oder verzweigtes Alkyl oder Alkanoyl mit jeweils bis zu 6 Kohlenstoffatomen oder für gegebenenfalls einfach oder mehrfach gleich oder verschieden substituiertes Benzoyl steht, wobei als Substituenten in Frage kommen: Halogen, jeweils geradkettiges oder verzweigtes Alkyl, Alkoxy oder Halogenalkyl mit jeweils bis zu 4 Kohlenstoffatomen und im Fall des Halogenalkyl mit bis zu 9 gleichen oder verschiedenen Halogenatomen, und

E für Halogen, Hydroxy, Alkyl- oder Arylsulfonyloxy, für Alkanoyloxy oder Aroyloxy steht,

oder mit anderen üblichen elektrophilen Reagenzien gegebenenfalls in Gegenwart eines Verdünnungsmittels und gegebenenfalls in Gegenwart eines Katalysators oder Reaktionshilfsmittels in 4-Stellung substituiert, oder dass man

(d) die nach Verfahren (c) erhältlichen 4-Chlorsulfonyl-5-amino-pyrazole der Formel (Id),

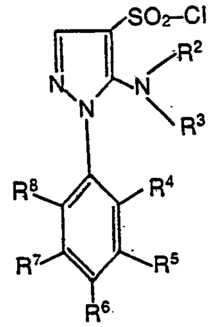

(Id)

in welcher

$R^2$, $R^3$, $R^4$, $R^5$, $R^6$, $R^7$ und $R^8$ die bei der Formel (I′) angegebene Bedeutung haben,

mit Aminen der Formel (VIII),

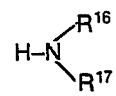

(VIII)

in welcher

$R^{16}$ und $R^{17}$ unabhängig voneinander für Wasserstoff oder für geradkettiges oder verzweigtes Alkyl mit bis zu 4 Kohlenstoffatomen stehen,

oder mit einem Alkalimetallfluorid der Formel (IX),

$$Me^{\oplus}F^{\ominus} \qquad \text{(IX)}$$

in welcher

$Me^{\oplus}$ für ein Alkalimetallkation steht,

gegebenenfalls in Gegenwart eines Verdünnungsmittels und gegebenenfalls in Gegenwart eines Säurebindemittels an der Chlorsulfonylgruppe in 4-Stellung substituiert oder dass man

(e) die nach den Verfahren (c) oder (f) erhältlichen 4-Acyl-5-amino-pyrazole der Formel (Ie)

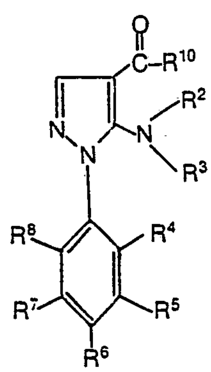

(Ie)

in welcher

$R^2$, $R^3$, $R^4$, $R^5$, $R^6$, $R^7$ und $R^8$ die bei der Formel (I′) angegebene Bedeutung haben und

$R^{10}$ die oben angegebene Bedeutung hat,

mit Hydroxylamin-Derivaten der Formel (X)

$$H_2N\text{–}OR^{11} \qquad \text{(X)}$$

in welcher

$R^{11}$ die oben angegebene Bedeutung hat,

oder mit deren Hydrosalzen, gegebenenfalls in Gegenwart eines Verdünnungsmittels und gegebenenfalls in Gegenwart eines Säurebindemittels umsetzt, oder dass man

(f) 4-Cyano-5-amino-pyrazole der Formel (XI),

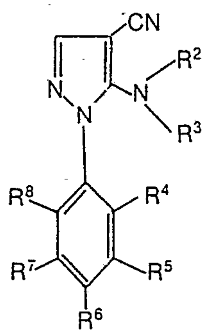

(XI)

in welcher

$R^2$, $R^3$, $R^4$, $R^5$, $R^6$, $R^7$, $R^8$ die bei der Formel (I′) angegebene Bedeutung haben,

mit Ameisensäure und Raney-Nickel umsetzt zu den entsprechenden 5-Amino-4-formyl-pyrazolen der Formel (If)

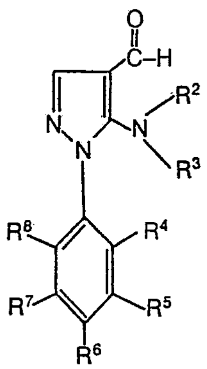

(If)

in welcher

$R^2$, $R^3$, $R^4$, $R^5$, $R^6$, $R^7$ und $R^8$ die bei der Formel (I′) angegebene Bedeutung haben.

8. Verfahren zur Herstellung von Phenylhydrazin-Derivaten der Formel (IV),

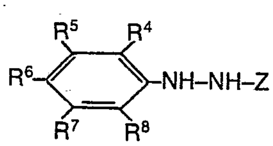

(IV)

in welcher

$R^4$, $R^5$, $R^6$, $R^7$ und $R^8$ die in Anspruch 1 angegebene Bedeutung haben und

Z für einen der Reste

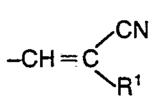

oder

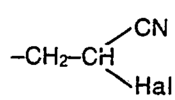

steht, wobei Hal für Halogen steht und

$R^1$ die in Anspruch angegebene Bedeutung hat, wobei jedoch für den Fall, dass $R^1$ für Wasserstoff, für Methyl oder für gegebenenfalls substituiertes Phenyl steht, $R^4$ und $R^6$ nicht gleichzeitig für die Nitro-Gruppe stehen,

dadurch gekennzeichnet, dass man

(a) Phenylhydrazine der Formel (II)

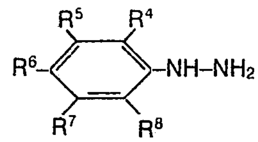

(II)

in welcher

$R^4$, $R^5$, $R^6$, $R^7$ und $R^8$ die oben angegebene Bedeutung haben

mit Acrylnitril-Derivaten der Formel (III)

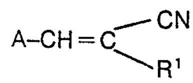

(III)

in welcher

$R^1$ die oben angegebene Bedeutung hat und

A für Halogen, Hydroxy oder Alkoxy steht,

oder für den Fall, dass $R^1$ für Wasserstoff steht, auch mit 2-Halogenacrylnitrilen der Formel (IIIa),

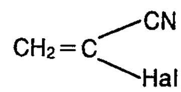

(IIIa)

in welcher

Hal für Halogen steht,

oder mit 2,3-Dihalogenpropionitrilen der Formel (IIIb),

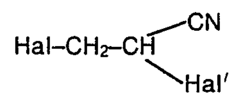

(IIIb)

in welcher

Hal und Hal′ für gleiche oder unterschiedliche Halogenatome stehen,

gegebenenfalls in Gegenwart eines Verdünnungsmittels, sowie gegebenenfalls in Gegenwart eines Reaktionshilfsmittels umsetzt.

9. 5-Amino-1-(2,6-dichlor-4-trifluormethylphenyl)-4-nitro-pyrazol.

10. 5-Amino-1-(2,6-dichlor-4-trifluormethylsulfonylphenyl)-4-nitro-pyrazol.

11. 5-Amino-1-(2,3,6-trichlor-4-trifluormethylphenyl)-4-nitro-pyrazol.

12. 5-(1-Chlorpropionamido)-1-(2,6-dichlor-4-trifluormethylphenyl)-4-nitro-pyrazol.

**Claims**

1. Herbicidal agents, characterized in that they contain at least one 5-amino-1-phenyl-pyrazole of the general formula (I′)

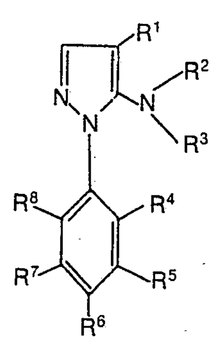

(I′)

in which

$R^1$ represents hydrogen, nitroso, nitro, halogen, alkyl or halogenoalkyl, each of which is straight-chain or branched and each of which has up to 6 carbon atoms and, in the case of halogenoalkyl, up to 9 identical or different halogen atoms, or represents phenyl which is optionally monosubstituted or polysubstituted by identical or different substituents from amongst halogen, nitro, alkyl, alkoxy or halogenoalkyl, each having up to 4 carbon atoms, or represents one of the radicals

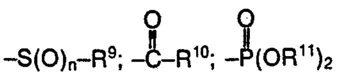 or 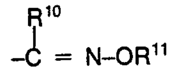

$R^2$ represents hydrogen or a radical 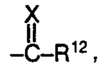

$R^3$ independently of $R^2$ represents the same radicals as $R^2$, and additionally represents straight-chain or branched alkyl having up to 4 carbon atoms,

$R^4$ and $R^6$ independently of one another represent cyano, nitro, halogen, or alkyl, alkoxy or alkoxycarbonyl, each of which is straight-chain or branched and each of which has up to 4 carbon atoms, and furthermore represents halogenoalkyl or halogenoalkoxy, each of which is straight-chain or branched and each of which has up to 4 carbon atoms and up to 9 identical or different halogen atoms, or represents a radical $-S(O)_n-R^{13}$, and

$R^5$, $R^7$ and $R^8$ independently of one another and of $R^4$ and $R^6$ represent the same radicals as $R^4$ and $R^6$ and additionally represent hydrogen and

$R^9$ represents hydrogen, hydroxyl, fluorine, chlorine, bromine, amino or alkylamino, dialkylamino, alkyl or halogenoalkyl, each of which is straight-chain or branched and each of which has up to 4 carbon atoms in the individual alkyl parts, and, in the case of halogenoalkyl, up to 9 identical or different halogen atoms, and represents phenyl which is optionally monosubstituted or polysubstituted by identical or different substituents, the following being suitable substituents: halogen, alkyl, alkoxy or halogenoalkyl, each of which is straight-chain or branched and each of which has up to 4 carbon atoms and, in the case of halogenoalkyl, up to 9 identical or different halogen atoms,

$R^{10}$ represents hydrogen, straight-chain or branched alkyl having up to 4 carbon atoms, or phenyl which is optionally monosubstituted or polysubstituted by identical or different substituents, suitable phenyl substituents being those mentioned in the case of $R^9$,

$R^{11}$ represents hydrogen, halogenoalkyl having up to 4 carbon atoms and up to 6 identical or different halogen atoms, alkyl, alkenyl or alkinyl, each of which is straight-chain or branched and each of which has up to 8 carbon atoms, and straight-chain or branched phenylalkyl having up to 4 carbon atoms in the alkyl part,

$R^{12}$ represents hydrogen, straight-chain or branched alkyl having 1–12 carbon atoms, or alkenyl, alkinyl, alkoxyalkyl, alkylthioalkyl, alkoxy, alkylthio, alkylamino, dialkylamino or halogenoalkyl, each of which has up to 4 carbon atoms in the individual alkyl parts and, in the case of halogenoalkyl, up to 9 identical or different halogen atoms, and furthermore represents cycloalkyl which has 3 to 7 carbon atoms and is optionally monosubstituted or polysubstituted by identical or different substituents from amongst halogen, lower alkyl or lower halogenoalkyl, and represents phenyl, phenoxy, phenylthio or phenylamino, each of which is optionally monosubstituted or polysubstituted by identical or different substituents, suitable phenyl substituents being those stated in the case of $R^9$;

$R^{13}$ represents amino, and alkyl, alkylamino, dialkylamino or halogenoalkyl, each of which is straight-chain or branched and each of which has up to 4 carbon atoms in the individual alkyl parts and, in the case of halogenoalkyl, up to 9 identical or different halogen atoms,

X represents oxygen or sulphur and

n represents the number 0, 1 or 2,

but in the event that $R^1$ represents hydrogen, methyl or optionally substituted phenyl, $R^4$ and $R^6$ do not simultaneously represent the nitro group and furthermore with the exception of the compounds 5-acetamido-1-(2,4,6-trinitrophenyl)-pyrazole and 1-(2-nitrophenyl)-4-methyl-5-amino-pyrazole.

2. Process for preparing herbicides, characterized in that 5-amino-1-phenyl-pyrazoles of the general formula (I') according to Claim 1 are mixed with extenders and/or surfactants.

3. Use of 5-amino-1-phenyl-pyrazoles of the general formula (I)

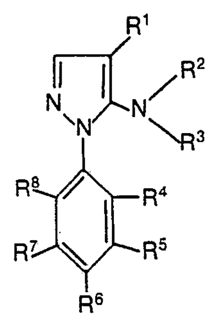

(I)

in which

$R^1$ represents hydrogen, nitroso, nitro, halogen, alkyl or halogenoalkyl, each of which is straight-chain or branched and each of which has up to 6 carbon atoms and, in the case of halogenoalkyl, up to 9 identical or different halogen atoms, or furthermore represents phenyl which is optionally monosubstituted or polysubstituted by identical or different substituents from amongst halogen, nitro, alkyl, alkoxy or halogenoalkyl, each having up to 4 carbon atoms, or represents one of the radicals

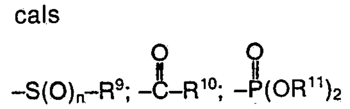 or 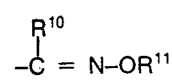

$R^2$ represents hydrogen or a radical 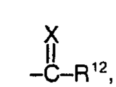

$R^3$ independently of $R^2$ represents the same radicals as $R^2$, and additionally represents straight-chain or branched alkyl having up to 4 carbon atoms,

$R^4$ and $R^6$ independently of one another represent cyano, nitro, halogen, or alkyl, alkoxy or alkoxycarbonyl, each of which is straight-chain or branched and each of which has up to 4 carbon atoms, and furthermore represents halogenoalkyl or halogenoalkoxy, each of which is straight-chain or branched and each of which has up to 4 carbon atoms and up to 9 identical or different halogen atoms, or represents a radical $-S(O)_n-R^{13}$, and

$R^5$, $R^7$ and $R^8$ independently of one another and of $R^4$ and $R^6$ represent the same radicals as $R^4$ and $R^6$ and additionally represent hydrogen and

$R^9$ represents hydrogen, hydroxyl, fluorine, chlorine, bromine or amino, or alkylamino, dialkylamino, alkyl or halogenoalkyl, each of which is straight-chain or branched and each of which has up to 4 carbon atoms in the individual alkyl parts, and, in the case of halogenoalkyl, up to 9 identical or different halogen atoms, and represents phenyl which is optionally monosubstituted or polysubstituted by identical or different substituents, the following being suitable substituents: halogen, or alkyl, alkoxy or halogenoalkyl, each of which is straight-chain or branched and each of which has up to 4 carbon atoms and, in the case of halogenoalkyl, up to 9 identical or different halogen atoms,

$R^{10}$ represents hydrogen, straight-chain or branched alkyl having up to 4 carbon atoms, or phenyl which is optionally monosubstituted or polysubstituted by identical or different substituents, suitable phenyl substituents being those mentioned in the case of $R^9$,

$R^{11}$ represents hydrogen, halogenoalkyl having up to 4 carbon atoms and up to 6 identical or different halogen atoms, alkyl, alkenyl or alkinyl, each of which is straight-chain or branched and each of which has up to 8 carbon atoms, and straight-chain or branched phenylalkyl having up to 4 carbon atoms in the alkyl part,

$R^{12}$ represents hydrogen, straight-chain or branched alkyl having 1–12 carbon atoms, or alkenyl, alkinyl, alkoxyalkyl, alkylthioalkyl, alkoxy, alkylthio, alkylamino, dialkylamino or halogenoalkyl, each of which has up to 4 carbon atoms in the individual alkyl parts and, in the case of halogenoalkyl, up to 9 identical or different halogen atoms, and furthermore represents cycloalkyl which has 3 to 7 carbon atoms and is optionally monosubstituted or polysubstituted by identical or different substituents from amongst halogen, lower alkyl or lower halogenoalkyl, and represents phenyl, phenoxy, phenylthio or phenylamino, each of which is optionally monosubstituted or polysubstituted by identical or different substituents, suitable phenyl substituents being those stated in the case of $R^9$;

$R^{13}$ represents amino, and alkyl, alkylamino, dialkylamino or halogenoalkyl, each of which is straight-chain or branched and each of which has up to 4 carbon atoms in the individual alkyl parts and, in the case of halogenoalkyl, up to 9 identical or different halogen atoms,

X represents oxygen or sulphur and

n represents the number 0, 1 or 2,

for combating weeds.

4. Process for combating weeds, characterized in that 5-amino-1-phenyl-pyrazoles of the general formula (I) according to Claim 3 are allowed to act on weeds or their environment.

5. 5-Amino-1-phenyl-pyrazoles of the formula (I')

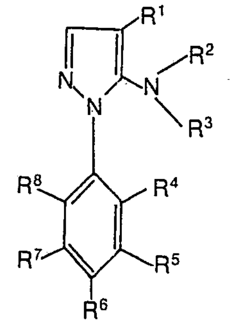

(I')

in which

$R^1$, $R^2$, $R^3$, $R^4$, $R^5$, $R^6$, $R^7$ and $R^8$ have the meaning given in Claim 1, except that, where $R^1$ represents hydrogen, methyl or optionally substituted phenyl, $R^4$ and $R^6$ do not simultaneously represent the nitro group and furthermore with the exception of the compounds 5-acetamido-1-(2,4,6-trinitrophenyl)-pyrazole and 1-(2-nitrophenyl)-4-methyl-5-aminopyrazole.

6. Phenylhydrazine derivatives of the formula

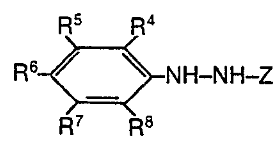

(IV)

in which

$R^4$, $R^5$, $R^6$, $R^7$ and $R^8$ have the meaning given in Claim 1 and

Z represents one of the radicals

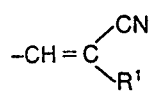

or

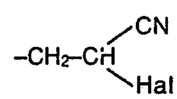

wherein

Hal represents halogen

and R′ has the meaning given in Claim 1, where, however, in the event that $R^1$ represents hydrogen, methyl or optionally substituted phenyl, $R^4$ and $R^6$ do not simultaneously represent the nitro group.

7. Process for the preparation of 5-amino-1-phenyl-pyrazoles of the formula (I')

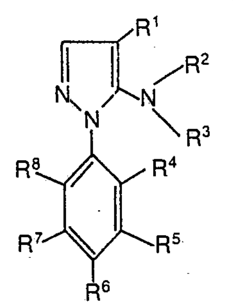

(I')

in which

$R^1$, $R^2$, $R^3$, $R^4$, $R^5$, $R^6$, $R^7$ and $R^8$ have the meaning given in Claim 1, except that, where $R^1$ represents hydrogen, methyl or optionally substituted phenyl, $R^4$ and $R^6$ do not simultaneously represent the nitro group, and furthermore with the exception of the compounds 5-acetamido-1-(2,4,6-trinitrophenyl)-pyrazole and 1-(2-nitrophenyl)-4-methyl-5-amino-pyrazole,

characterized in that

(a) phenylhydrazines of the formula (II)

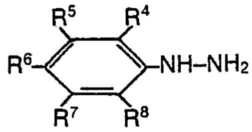

(II)

in which

$R^4$, $R^5$, $R^6$, $R^7$ and $R^8$ have the meaning given in the formula (I'), are reacted with acrylonitrile derivatives of the formula (III)

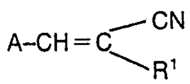

(III)

in which

$R^1$ has the meaning given in the formula (I') and

A represents halogen, hydroxyl or alkoxy,

or, in the case in which $R^1$ represents hydrogen, also with 2-halogenoacrylonitriles of the formula (IIIa)

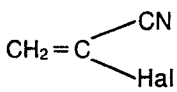

(IIIa)

in which

Hal represents halogen,

or with 2,3-dihalogenopropionitriles of the formula (IIIb)

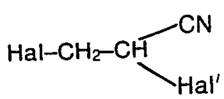

(IIIb)

in which

Hal and Hal' represent identical or different halogen atoms,

the reaction either being carried out to give the phenylhydrazine derivatives of the formula (IV)

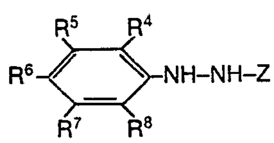

(IV)

in which

$R^4$, $R^5$, $R^6$, $R^7$ and $R^8$ have the meaning given in the formula (I') and

Z represents one of the radicals

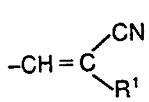 or 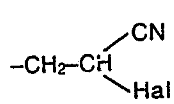

wherein Hal represents halogen and $R^1$ has the meaning given in the formula (I'), initially in a first stage, if appropriate in the presence of a diluent and, if appropriate in the presence of a reaction auxiliary, and the product being subjected to a cyclisation reaction in a second stage, if appropriate in the presence of a diluent and, if appropriate, in the presence of an acid-binding agent, or the cyclisation being carried out directly in one reaction step without isolation of the intermediate of the formula (IV), if appropriate in the presence of a diluent, to give the 5-amino-pyrazoles of the formula (Ia)

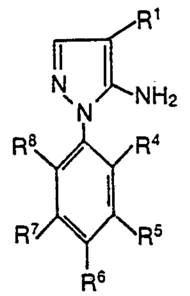

(Ia)

in which

$R^1$, $R^4$, $R^5$, $R^6$, $R^7$ and $R^8$ have the meaning given in the formula (I')

or in that

(b) the 5-amino-pyrazoles of the formula (Ia)

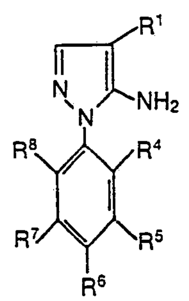

(Ia)

in which

$R^1$, $R^4$, $R^5$, $R^6$, $R^7$ and $R^8$ have the meaning given in the formula (I')

which are obtainable by process (a) are reacted in a generally customary manner with acylating agents or alkylating agents of the formula (V)

$$R^{14}\text{–}A' \qquad (V)$$

in which

$R^{14}$ represents straight-chain or branched alkyl having up to 4 carbon atoms or a radical

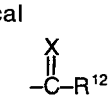

wherein

X and $R^{12}$ have the meaning given in Claim 1, and

A' represents chlorine, bromine, iodine, p-toluene-sulphonyloxy, alkylsulphonyloxy or acyloxy or with iso(thio)cyanates of the formula (VI)

$$R^{15}\text{–}N=C=X \qquad (VI)$$

in which

$R^{15}$ represents straight-chain or branched alkyl having up to 4 carbon atoms or phenyl which is optionally monosubstituted to trisubstituted by identical or different substituents from amongst

halogen or alkyl, alkoxy or halogenoalkyl, in each case straight-chain or branched and in each case having up to 4 carbon atoms and, in the case of halogenoalkyl, having up to 9 identical or different halogen atoms, and

X has the above meaning,

if appropriate in the presence of a diluent and, if appropriate in the presence of an acid-binding agent, to give the 5-amino-pyrazoles alkylated or acylated at the nitrogen, of the formula (Ib)

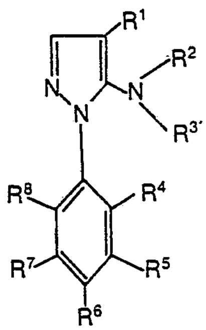

(Ib)

in which

$R^1$, $R^2$, $R^4$, $R^5$, $R^6$, $R^7$ and $R^8$ have the meaning given in the formula (I') and

$R^{3'}$ represents the same radicals as $R^3$ given above, with the exception of the hydrogen radical, or in that

(c) the 5-amino-pyrazoles of the formula (Ic)

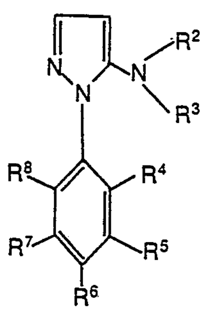

(Ic)

in which

$R^2$, $R^3$, $R^4$, $R^5$, $R^6$, $R^7$ and $R^8$ have the meaning given in the formula (I'),

which are unsubstituted in the 4-position and are obtainable by process (a) or (b), are substituted in the 4-position, likewise in a generally customary manner, using electrophilic agents of the formula (VII)

$$R^{1'}-E \qquad (VII)$$

in which

$R^{1'}$ represents chlorine, bromine, nitroso, nitro, formyl, hydroxysulphonyl or chlorosulphonyl and alkyl or alkanoyl, in each case straight-chain or branched and in each case having up to 6 carbon atoms, or for benzoyl which is optionally monosubstituted or polysubstituted by identical or different substituents, suitable substituents being halogen, or alkyl, alkoxy or halogenoalkyl, in each case straight-chain or branched and in each case having up to 4 carbon atoms and, in the case of halogenoalkyl, having up to 9 identical or different halogen atoms, and

E represents a halogen, hydroxyl, alkyl- or arylsulfonyloxy, alkanoyloxy or aroyloxy,

or using other customary electrophilic reagents, if appropriate in the presence of a diluent and, if appropriate, in the presence of a catalyst or a reaction auxiliary, or in that

(d) the 4-chlorosulphonyl-5-amino-pyrazoles obtainable by process (c) of the formula (Id)

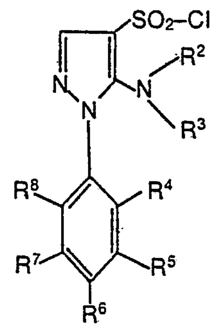

(Id)

in which

$R^2$, $R^3$, $R^4$, $R^5$, $R^6$, $R^7$ and $R^8$ have the meaning given in the formula (I'), are substituted at the chlorosulphonyl group in the 4-position, using amines of the formula (VIII)

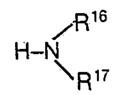

(VIII)

in which

$R^{16}$ and $R^{17}$ independently of one another represent hydrogen or straight-chain or branched alkyl having up to 4 carbon atoms,

or using an alkali metal fluoride of the formula (IX)

$$Me^{\oplus} F^{\ominus} \qquad (IX)$$

in which

$Me^{\oplus}$ represents an alkali metal cation, if appropriate in the presence of a diluent and, if appropriate, in the presence of an acid-binding agent, or in that

(e) the 4-acyl-5-amino-pyrazoles of the formula (Ie)

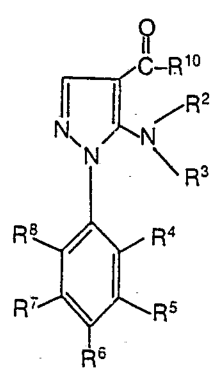

(Ie)

in which

$R^2$, $R^3$, $R^4$, $R^5$, $R^6$, $R^7$ and $R^8$ have the meaning given in the formula (I'),

and $R^{10}$ has the above meaning

which are obtainable by the process (c) or (f), are reacted with hydroxylamine derivatives of the formula (X)

$H_2N-OR^{11}$ (X)

in which

$R^{11}$ has the meaning given above,

or with their hydro-salts, if appropriate in the presence of a diluent and, if appropriate, in the presence of an acid-binding agent, or in that

(f) 4-cyano-5-amino-pyrazoles of the formula (XI)

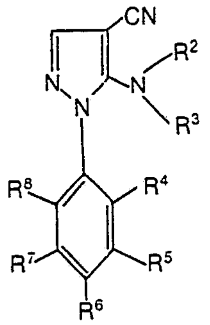

(XI)

in which

$R^2$, $R^3$, $R^4$, $R^5$, $R^6$, $R^7$ and $R^8$ have the meaning given in the formula (I′)

are reacted with formic acid and Raney nickel to give the corresponding 5-amino-4-formyl-pyrazoles of the formula (If)

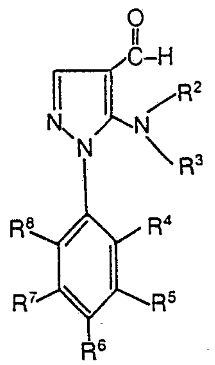

(If)

in which

$R^2$, $R^3$, $R^4$, $R^5$, $R^6$, $R^7$ and $R^8$ have the meaning given in the formula (I′).

8. Process for the preparation of phenylhydrazine derivatives of the formula (IV)

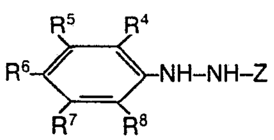

(IV)

in which

$R^4$, $R^5$, $R^6$, $R^7$ and $R^8$ have the meaning given in Claim 1 and

Z represents one of the radicals

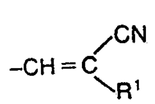

or

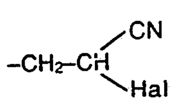

wherein Hal represents halogen and

$R^1$ has the meaning given in Claim 1,

where, however, in the event that $R^1$ represents hydrogen, methyl or optionally substituted phenyl, $R^4$ and $R^6$ do not simultaneously represent the nitro group,

characterized in that

(a) phenylhydrazines of the formula (II)

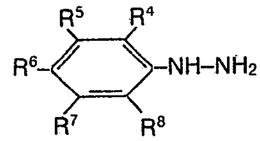

(II)

in which

$R^4$, $R^5$, $R^6$, $R^7$ and $R^8$ have the meaning given above,

are reacted with acrylonitrile derivatives of the formula (III)

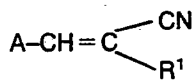

(III)

in which

$R^1$ has the meaning given above and

A represents halogen, hydroxyl or alkoxy

or, in the case in which $R^1$ represents hydrogen, also with 2-halogenoacrylonitriles of the formula (IIIa)

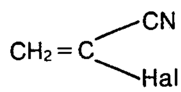

(IIIa)

in which

Hal represents halogen,

or with 2,3-dihalogenopropionitriles of the formula (IIIb)

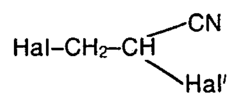

(IIIb)

in which

Hal and Hal′ represent identical or different halogen atoms,

if appropriate in the presence of a diluent, and if appropriate in the presence of a reaction auxiliary.

9. 5-amino-1-(2,6-dichloro-4-trifluoromethylphenyl)-4-nitro-pyrazole.

10. 5-Amino-1-(2,6-dichloro-4-trifluoromethylsulphonylphenyl)-4-nitro-pyrazole.

11. 5-Amino-1-(2,3,6-trichloro-4-trifluoromethylphenyl)-4-nitro-pyrazole.

12. 5-(1-Chloropropionamido)-1-(2,6-dichloro-4-trifluoromethylphenyl)-4-nitro-pyrazole.

## Revendications

1. Compositions herbicides, caractérisées par une teneur en au moins un 5-amino-1-phénylpyrazole de formule générale (I′)

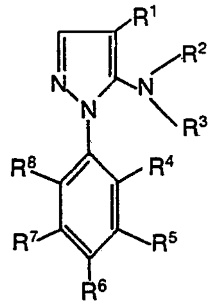

(I′)

dans lequelle

$R^1$ représente l'hydrogène, un groupe nitroso, nitro, un halogène, un groupe alkyle ou halogénalkyle, chacun à chaîne droite ou à chaîne ramifiée avec jusqu'à 6 atomes de carbone et, dans le cas des groupes halogénalkyle, jusqu'à 9 atomes d'halogènes identiques ou différents, en outre un groupe phényle portant éventuellement un ou plusieurs substituants, identiques ou différents, halogéno, nitro, alkyle, alkoxy ou halogénalkyle ayant chacun jusqu'à 4 atomes de carbone, ou l'un des restes

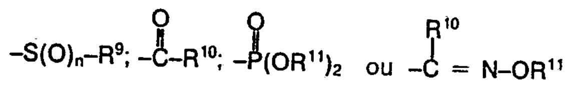

$R^2$ représente l'hydrogène ou un reste 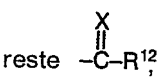

$R^3$ représente, indépendamment de $R^2$, les mêmes restes que $R^2$ et représente en outre un groupe alkyle à chaîne droite ou à chaîne ramifiée ayant jusqu'à 4 atomes de carbone,

$R^4$ et $R^6$ représentent, indépendamment l'un de l'autre, un groupe cyano, nitro, un halogène ou un groupe alkyle, alkoxy ou alkoxycarbonyle, chacun à chaîne droite ou à chaîne ramifiée avec jusqu'à 4 atomes de carbone, en outre un groupe halogénalkyle ou halogénalkoxy chacun à chaîne droite ou à chaîne ramifiée avec jusqu'à 4 atomes de carbone et jusqu'à 9 atomes d'halogènes identiques ou différents, ou un reste $-S(O)_n-R^{13}$, et

$R^5$, $R^7$ et $R^8$ représentent, indépendamment les uns des autres et de $R^4$ et $R^6$, les mêmes restes que $R^4$ et $R^6$ et représentent en outre l'hydrogène,

$R^9$ représente l'hydrogène, un groupe hydroxy, le fluor, le chlore, le brome, un groupe amino, un groupe alkylamino, dialkylamino, alkyle ou halogénalkyle chacun à chaîne droite ou à chaîne ramifiée avec jusqu'à 4 atomes de carbone dans les parties alkyliques individuelles et, dans le cas du groupe halogénalkyle, avec jusqu'à 9 atomes d'halogènes identiques ou différents, ainsi qu'un groupe phényle portant éventuellement un ou plusieurs substituants identiques ou différents, et on considère alors comme substituants: un halogène, un groupe alkyle, alkoxy ou halogénalkyle chacun à chaîne droite ou à chaîne ramifiée avec jusqu'à 4 atomes de carbone et, dans le cas du groupe halogénalkyle, avec jusqu'à 9 atomes d'halogènes identiques ou différents,

$R^{10}$ représente l'hydrogène, un groupe alkyle à chaîne droite ou à chaîne ramifiée ayant jusqu'à 4 atomes de carbone ou un groupe phényle portant éventuellement un ou plusieurs substituants identiques ou différents, et on considère alors comme substituants du groupe phényle ceux qui sont mentionnés dans le cas de $R^9$,

$R^{11}$ représente l'hydrogène, un groupe halogénalkyle ayant jusqu'à 4 atomes de carbone et jusqu'à 6 atomes d'halogènes identiques ou différents, un groupe alkyle, alcényle ou alcynyle chacun à chaîne droite ou à chaîne ramifiée avec jusqu'à 8 atomes de carbone, ainsi qu'un groupe phénylalkyle à chaîne droite ou à chaîne ramifiée avec jusqu'à 4 atomes de carbone dans la partie alkylique,

$R^{12}$ représente l'hydrogène, un groupe, à chaîne droite ou ramifiée, alkyle avec 1 à 12 atomes de carbone ainsi qu'alcényle, alcynyle, alkoxyalkyle, alkylthioalkyle, alkoxy, alkylthio, alkylamino, dialkylamino ou halogénalkyle ayant chacun jusqu'à 4 atomes de carbone dans les parties alkyliques individuelles et, dans le cas du groupe halogénalkyle, avec jusqu'à 9 atomes d'halogènes identiques ou différents, en outre un groupe cycloalkyle de 3 à 7 atomes de carbone portant éventuellement un ou plusieurs substituants, identiques ou différents, halogéno, alkyle inférieur ou halogénalkyle inférieur, ainsi qu'un groupe phényle, phénoxy, phénylthio ou phénylamino portant chacun, le cas échéant, un ou plusieurs substituants identiques ou différents, et on considère alors comme substituants du groupe phényle ceux qui ont été mentionnés dans le cas de $R^9$;

$R^{13}$ est un groupe amino, ainsi qu'un groupe alkyle, alkylamino, dialkylamino ou halogénalkyle chacun à chaîne droite ou à chaîne ramifiée, avec jusqu'à 4 atomes de carbone dans les parties alkyliques individuelles et, dans le cas du groupe halogénalkyle, avec jusqu'à 9 atomes d'halogènes identiques ou différents,

X représente l'oxygène ou le soufre et

n est le nombre 0, 1 ou 2,

sous réserve que lorsque $R^1$ représente l'hydrogène, le groupe méthyle ou un groupe phényle éventuellement substitué, $R^4$ et $R^6$ ne représentent pas simultanément le groupe nitro, et à l'exclusion en outre des composés

5-acétamido-1-(2,4,6-trinitrophényl)pyrazole

et

1-(2-nitrophényl)-4-méthyl-5-aminopyrazole.

2. Procédé de préparation de compositions herbicides, caractérisé en ce qu'on mélange des 5-amino-1-phénylpyrazoles de formule générale (I') selon la revendication 1 avec des diluants et/ou des agents tensio-actifs.

3. Utilisation de 5-amino-1-phénylpyrazoles de formule générale (I)

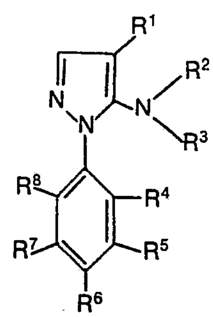

(I)

dans laquelle

$R^1$ représente l'hydrogène, un groupe nitroso, nitro, un halogène, un groupe alkyle ou halogénalkyle, chacun à chaîne droite ou à chaîne ramifiée, avec jusqu'à 6 atomes de carbone et, dans le cas des groupes halogénalkyle, jusqu'à 9 atomes d'halogènes identiques ou différents, en outre un groupe phényle portant éventuellement un ou plusieurs substituants, identiques ou différents, halogéno, nitro, alkyle, alkoxy ou halogénalkyle ayant chacun jusqu'à 4 atomes de carbone, ou l'un des restes

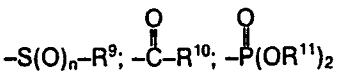 ou 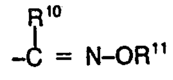

$R^2$ représente l'hydrogène ou un reste 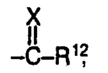

$R^3$ représente, indépendamment de $R^2$, les mêmes restes que $R^2$ et représente en outre un groupe alkyle à chaîne droite ou à chaîne ramifiée ayant jusqu'à 4 atomes de carbone,

$R^4$ et $R^6$ représentent, indépendamment l'un de l'autre, un groupe cyano, nitro, un halogène ou un groupe alkyle, alkoxy ou alkoxycarbonyle, chacun à chaîne droite ou à chaîne ramifiée avec jusqu'à 4 atomes de carbone, en outre un groupe halogénalkyle ou halogénalkoxy chacun à chaîne droite ou à chaîne ramifiée avec jusqu'à 4 atomes de carbone et jusqu'à 9 atomes d'halogènes identiques ou différents, ou un reste $-S(O)_n-R^{13}$, et

$R^5$, $R^7$ et $R^8$ représentent, indépendamment les uns des autres et de $R^4$ et $R^6$, les mêmes restes que $R^4$ et $R^6$ et représentent en outre l'hydrogène,

$R^9$ représente l'hydrogène, un groupe hydroxy, le fluor, le chlore, le brome, un groupe amino, un groupe alkylamino, dialkylamino, alkyle ou halogénalkyle chacun à chaîne droite ou à chaîne ramifiée avec jusqu'à 4 atomes de carbone dans les parties alkyliques individuelles et, dans le cas du groupe halogénalkyle, avec jusqu'à 9 atomes d'halogènes identiques ou différents, ainsi qu'un groupe phényle portant éventuellement un ou plusieurs substituants identiques ou différents, et on considère alors comme substituants: un halogène, un groupe alkyle, alkoxy ou halogénalkyle chacun à chaîne droite ou à chaîne ramifiée avec jusqu'à 4 atomes de carbone et, dans le cas du groupe halogénalkyle, avec jusqu'à 9 atomes d'halogènes identiques ou différents,

$R^{10}$ représente l'hydrogène, un groupe alkyle à chaîne droite ou à chaîne ramifiée ayant jusqu'à 4 atomes de carbone ou un groupe phényle portant éventuellement un ou plusieurs substituants identiques ou différents, et on considère alors comme substituants du groupe phényle ceux qui sont mentionnés dans le cas de $R^9$,

$R^{11}$ représente l'hydrogène, un groupe halogénalkyle ayant jusqu'à 4 atomes de carbone et jusqu'à 6 atomes d'halogènes identiques ou différents, un groupe alkyle, alcényle ou alcynyle chacun à chaîne droite ou à chaîne ramifiée avec jusqu'à 8 atomes de carbone, ainsi qu'un groupe phénylalkyle à chaîne droite ou à chaîne ramifiée avec jusqu'à 4 atomes de carbone dans la partie alkylique,

$R^{12}$ représente l'hydrogène, un groupe, à chaîne droite ou ramifiée, alkyle avec 1 à 12 atomes de carbone ainsi qu'alcényle, alcynyle, alkoxyalkyle, alkylthioalkyle, alkoxy, alkylthio, alkylamino, dialkylamino ou halogénalkyle ayant chacun jusqu'à 4 atomes de carbone dans les parties alkyliques individuelles et, dans le cas du groupe halogénalkyle, avec jusqu'à 9 atomes d'halogènes identiques ou différents, en outre un groupe cycloalkyle de 3 à 7 atomes de carbone portant éventuellement un ou plusieurs substituants, identiques ou différents, halogéno, alkyle inférieur ou halogénalkyle inférieur, ainsi qu'un groupe phényle, phénoxy, phénylthio ou phénylamino portant chacun, le cas échéant, un ou plusieurs substituants identiques ou différents, et on considère alors comme substituants du groupe phényle ceux qui ont été mentionnés dans le cas de $R^9$;

$R^{13}$ est un groupe amino, ainsi qu'un groupe alkyle, alkylamino, dialkylamino ou halogénalkyle chacun à chaîne droite ou à chaîne ramifiée, avec jusqu'à 4 atomes de carbone dans les parties alkyliques individuelles et, dans le cas du groupe halogénalkyle, avec jusqu'à 9 atomes d'halogènes identiques ou différents,

X représente l'oxygène ou le soufre et

n est le nombre 0, 1 ou 2,

pour combattre une mauvaise herbe.

4. Procédé pour combattre une mauvaise herbe, caractérisé en ce qu'on fait agir sur la mauvaise herbe ou sur son milieu des 5-amino-1-phénylpyrazoles de formule générale (I) suivant la revendication 3.

5. 5-amino-1-phénylpyrazoles de formule (I')

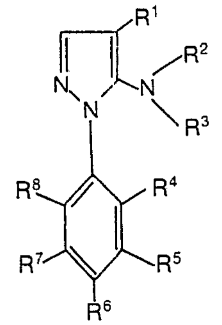

(I')

dans laquelle

$R^1$, $R^2$, $R^3$, $R^4$, $R^5$, $R^6$, $R^7$ et $R^8$ ont la définition indiquée dans la revendication 1,

sous réserve que lorsque $R^1$ représente l'hydrogène, le groupe méthyle ou un groupe phényle éventuellement substitué, $R^4$ et $R^6$ ne représentent pas simultanément le groupe nitro, et à l'exclusion en outre des composés 5-acétamido-1-(2,4,6-trinitrophényl)pyrazole et 1-(2-nitrophényl)-4-méthyl-5-aminopyrazole.

6. Dérivés de phénylhydrazine de formule

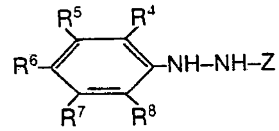

(IV)

dans laquelle

$R^4$, $R^5$, $R^6$, $R^7$ et $R^8$ ont la définition indiquée dans la revendication 1 et

Z représente l'un des restes

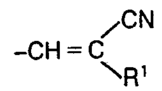 ou 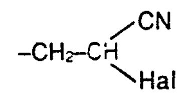

dans lesquels

Hal représente un halogène et

$R^1$ a la définition indiquée dans la revendication 1,

sous réserve que lorsque $R^1$ représente l'hydrogène, le groupe méthyle ou un groupe phényle éventuellement substitué, $R^4$ et $R^6$ ne représentent pas simultanément le groupe nitro.

7. Procédé de production de 5-amino-1-phénylpyrazoles de formule (I')

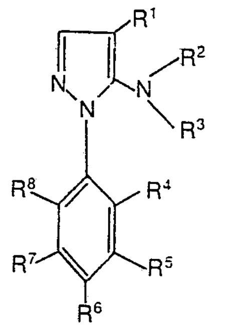

(I')

dans laquelle

$R^1$, $R^2$, $R^3$, $R^4$, $R^5$, $R^6$, $R^7$ et $R^8$ ont la définition indiquée dans la revendication 1,

sous réserve que lorsque $R^1$ représente l'hydrogène, le groupe méthyle ou un groupe phényle éventuellement substitué, $R^4$ et $R^6$ ne représentent pas simultanément le groupe nitro, et en outre à l'exclusion des composés 5-acétamido-1-(2,4,6-trinitrophényl)pyrazole et 1-(2-nitrophényl)-4-méthyl-5-aminopyrazole,

caractérisé en ce que

(a) on fait réagir tout d'abord dans une première étape des phénylhydrazines de formule (II)

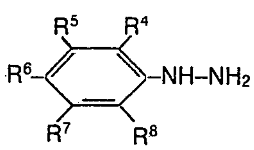

(II)

dans laquelle

$R^4$, $R^5$, $R^6$, $R^7$ et $R^8$ ont la définition indiquée pour la formule (I'),

avec des dérivés d'acrylonitrile de formule (III)

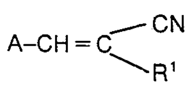

(III)

dans laquelle

$R^1$ a la définition indiquée pour la formule (I') et

A représente un halogène, un groupe hydroxy ou alkoxy,

ou pour le cas où $R^1$ représente l'hydrogène, également avec des 2-halogénacrylonitriles de formule (IIIa)

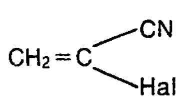

(IIIa)

dans laquelle

Hal représente un halogène,

ou avec des 2,3-dihalogénopropionitriles de formule (IIIb)

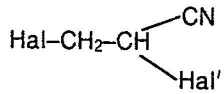

(IIIb)

dans laquelle

Hal et Hal' représent des atomes d'halogènes identiques ou différents,

le cas échéant en présence d'un diluant de même qu'en la présence éventuelle d'une substance auxiliaire de réaction pour former les dérivés de phénylhydrazine de formule (IV)

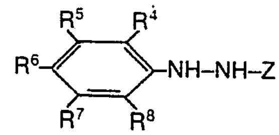

(IV)

dans laquelle

$R^4$, $R^5$, $R^6$, $R^7$ et $R^8$ ont la définition indiquée pour la formule (I'),

Z représente l'un des restes

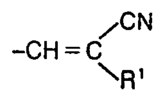

ou

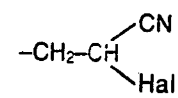

où Hal est un halogène et $R^1$ a la définition indiquée pour la formule (I') et on les cyclise dans une seconde étape, le cas échéant en présence d'un diluant et en la présence éventuelle d'un accepteur d'acide, ou bien on effectue une cyclisation directe en une étape réactionnelle sans isolement du stade intermédiaire (IV), le cas échéant en présence d'un diluant, en les 5-aminopyrazoles de formule (Ia)

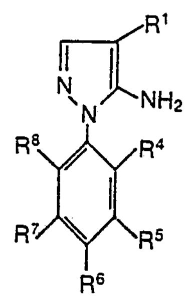

(Ia)

dans laquelle

$R^1$, $R^4$, $R^5$, $R^6$, $R^7$ et $R^8$ ont la définition indiquée pour la formule (I'),

ou en ce que

(b) on fait réagir les 5-aminopyrazoles obtenus selon le procédé (a), de formule (Ia)

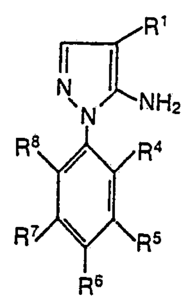

(Ia)

dans laquelle

$R^1$, $R^4$, $R^5$, $R^6$, $R^7$ et $R^8$ ont la définition indiquée pour la formule (I′)

avec des agents acylants ou des agents alkylants de formule (V)

$$R^{14}\text{–}A' \qquad (V)$$

dans laquelle

$R^{14}$ est un groupe alkyle à chaîne droite ou à chaîne ramifiée ayant jusqu'à 4 atomes de carbone ou un reste

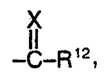

dans lequel

X et $R^{12}$ ont la définition indiquée dans la revendication 1 et

A′ représente le chlore, le brome, l'iode, un groupe p-toluènesulfonyloxy, alkylsulfonyloxy ou acyloxy,

ou avec des iso(thio)cyanates de formule (VI)

$$R^{15}\text{–}N{=}C{=}X \qquad (VI)$$

dans laquelle

$R^{15}$ est un groupe alkyle à chaîne droite ou à chaîne ramifiée ayant jusqu'à 4 atomes de carbone ou un groupe phényle portant éventuellement un à trois substituants identiques ou différents, et on considère alors comme substituants: un halogène, un groupe alkyle, alkoxy ou halogénalkyle chacun à chaîne droite ou à chaîne ramifiée avec jusqu'à 4 atomes de carbone et, dans le cas du groupe halogénalkyle, avec jusqu'à 9 atomes d'halogènes identiques ou différents, et

X a la définition indiquée ci-dessus,

éventuellement en présence d'un diluant et, le cas échéant en présence d'un accepteur d'acide, pour former les 5-aminopyrazoles alkylés ou acylés sur l'atome d'azote, de formule (Ib)

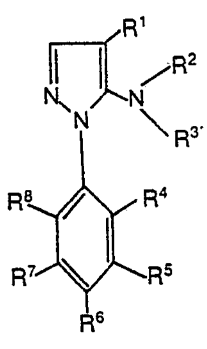

(Ib)

dans laquelle

$R^1$, $R^2$, $R^4$, $R^5$, $R^6$, $R^7$ et $R^8$ ont la définition indiquée pour la formule (I′) et

$R^{3'}$ représente les mêmes restes que le symbole $R^3$ indique ci-dessus à l'exception du reste hydrogène,

ou en ce que

(c) on substitue en position 4 les 5-aminopyrazoles non substitués, obtenus selon les procédés (a) ou (b), de formule (Ic)

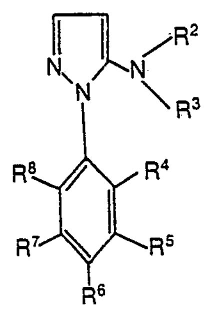

(Ic)

dans laquelle

$R^2$, $R^3$, $R^4$, $R^5$, $R^6$, $R^7$ et $R^8$ ont la définition indiquée pour la formule (I′),

avec des agents électrophiles de formule (VII)

$$R^{1'}\text{–}E \qquad (VII)$$

dans laquelle

$R^{1'}$ représente le chlore, le brome, un groupe nitroso, nitro, formyle, hydroxysulfonyle, chlorosulfonyle ainsi qu'un groupe alkyle ou alcanoyle à chaîne droite ou à chaîne ramifiée ayant chacun jusqu'à 6 atomes de carbone ou un groupe benzoyle portant éventuellement un ou plusieurs substituants identiques ou différents, et on considère alors comme substituants: un halogène, un groupe alkyle, alkoxy ou halogénalkyle chacun à chaîne droite ou à chaîne ramifiée avec jusqu'à 4 atomes de carbone et, dans le cas du groupe halogénalkyle, avec jusqu'à 9 atomes d'halogènes identiques ou différents, et

E représente un halogène, un groupe hydroxy, alkylsulfonyle ou arylsulfonyle, un groupe alcanoyloxy ou aroyloxy,

ou avec d'autres réactifs électrophiles classiques, éventuellement en présence d'un diluant et, le cas échéant en présence d'un catalyseur ou d'une substance auxiliaire de réaction, ou en ce que

(d) on substitue en position 4 sur le groupe chlorosulfonyle des 4-chlorosulfonyl-5-aminopyrazoles obtenus selon le procédé (c), de formule (Id)

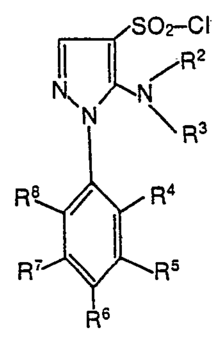

(Id)

dans laquelle

$R^2$, $R^3$, $R^4$, $R^5$, $R^6$, $R^7$ et $R^8$ ont la définition indiquée pour la formule (I′),

avec des amines de formule (VIII)

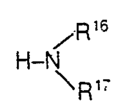

(VIII)

dans laquelle

$R^{16}$ et $R^{17}$ représentent, indépendamment l'un de l'autre, l'hydrogène ou un groupe alkyle à chaîne droite ou ramifiée ayant jusqu'à 4 atomes de carbone,

ou avec un fluorure de métal alcalin de formule (IX)

$$Me^{\oplus}F^{\ominus} \quad (IX)$$

dans laquelle

$Me^{\oplus}$ représente un cation de métal alcalin,

éventuellement en présence d'un diluant et, le cas échéant en présence d'un accepteur d'acide, ou en ce que

(e) on fait réagir les 4-acyl-5-aminopyrazoles obtenus selon les procédés (c) ou (f), de formule (Ie)

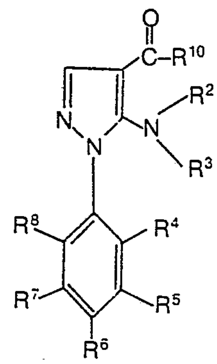

(Ie)

dans laquelle

$R^2$, $R^3$, $R^4$, $R^5$, $R^6$, $R^7$ et $R^8$ ont la définition indiquée pour la formule (I') et

$R^{10}$ a la définition indiquée ci-dessus,

avec des dérivés d'hydroxylamine de formule (X)

$$H_2N-OR^{11} \quad (X)$$

dans laquelle

$R^{11}$ a la définition indiquée ci-dessus,

ou avec leurs sels d'acides, éventuellement en présence d'un diluant et en la présence éventuelle d'un accepteur d'acide,

ou en ce que

(f) on fait réagir des 4-cyano-5-aminopyrazoles de formule (XI)

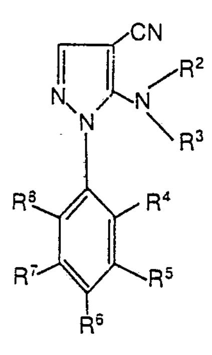

(XI)

dans laquelle

$R^2$, $R^3$, $R^4$, $R^5$, $R^6$, $R^7$, $R^8$ ont la définition indiquée pour la formule (I'),

avec l'acide formique et le nickel de Raney pour former les 5-amino-4-formylpyrazoles correspondants de formule (If)

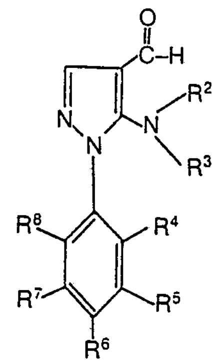

(If)

dans laquelle

$R^2$, $R^3$, $R^4$, $R^5$, $R^6$, $R^7$ et $R^8$ ont la définition indiquée pour la formule (I').

8. Procédé de production de dérivés de phénylhydrazine de formule (IV)

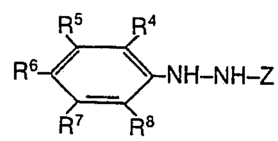

(IV)

dans laquelle

$R^4$, $R^5$, $R^6$, $R^7$ et $R^8$ ont la définition indiquée dans la revendication 1 et

Z représente l'un des restes

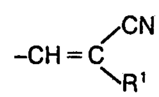

ou

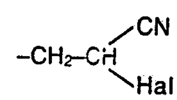

où Hal représente un halogène et

$R^1$ a la définition indiquée dans la revendication 1,

sous réserve que lorsque $R^1$ représente l'hydrogène, le groupe méthyle ou un groupe phényle éventuellement substitué, $R^4$ et $R^6$ ne représentent pas simultanément le groupe nitro,

caractérisé en ce que

(a) on fait réagir des phénylhydrazines de formule (II)

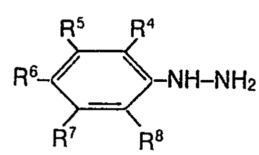

(II)

dans laquelle

$R^4$, $R^5$, $R^6$, $R^7$ et $R^8$ ont la définition indiquée ci-dessus, avec des dérivés d'acrylonitrile de formule (III)

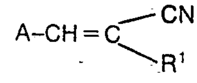

(III)

dans laquelle
$R^1$ a la définition indiquée ci-dessus et
A représente un halogène, le groupe hydroxy ou un groupe alkoxy,

ou au cas où $R^1$ représente l'hydrogène, également avec des 2-halogénacrylonitriles de formule (IIIa)

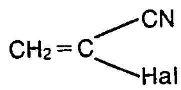

(IIIa)

dans laquelle
Hal représente un halogène,

ou avec des 2,3-dihalogénopropionitriles de formule (IIIb)

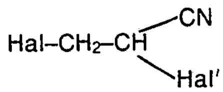

(IIIb)

dans laquelle
Hal et Hal′ représentent des atomes d'halogènes identiques ou différents,
le cas échéant en présence d'un diluant ainsi qu'en la présence éventuelle d'une substance auxiliaire de réaction.

9. Le 5-amino-1-(2,6-dichloro-4-trifluorométhylphényl)-4-nitropyrazole.
10. Le 5-amino-1-(2,6-dichloro-4-tri-fluorométhylsulfonylphényl)-4-nitropyrazole.
11. Le 5-amino-1-(2,3,6-trichloro-4-trifluorométhylphényl)-4-nitropyrazole.
12. Le 5-(1-chloropropionamido)-1-(2,6-dichloro-4-trifluorométhylphényl-4-nitropyrazole.